# EUROPÄISCHE PATENTSCHRIFT

(11) **EP 1 711 627 B1**
(45) Veröffentlichungstag und Bekanntmachung des Hinweises auf die Patenterteilung: **04.08.2010**
(21) Anmeldenummer: 05701192.6
(22) Anmeldetag: 26.01.2005
(51) Int. Cl.: C12Q 1/68

(54) **VERFAHREN ZUR GENO- UND PATHOTYPISIERUNG VON PSEUDOMONAS AERUGINOSA**
METHOD FOR GENO- AND PATHOTYPING PSEUDOMONAS AERUGINOSA
PROCEDE DE GENOTYPAGE ET DE PATHOTYPAGE DE PSEUDOMONAS AERUGINOSA

(30) Priorität: 26.01.2004 DE 102004003860
(43) Veröffentlichungstag der Anmeldung: 18.10.2006
(73) Patentinhaber: CLONDIAG GmbH, 07749 Jena (DE)
(72) Erfinder: WAGNER, Gerd, 07743 Jena (DE); WIEHLMANN, Lutz, 30177 Hannover (DE); TUEMMLER, Burkhard, 30179 Hannover (DE)
(74) Vertreter: Huenges, Martin
(86) Internationale Anmeldenummer: PCT/EP2005/000751
(87) Internationale Veröffentlichungsnummer: WO 2005/071108

(56) Entgegenhaltungen:
- WO-A-03/059516
- DATABASE EMBL [Online] 2. November 2001 (2001-11-02), "Sequence 35 from Patent WO0177172." XP002329868 gefunden im EBI accession no. EM_PAT:AX280412 Database accession no. AX280412
- DATABASE GEO [Online] 11. März 2002 (2002-03-11), "Affymetrix GeneChip Pseudomonas aeruginosa Array" XP002329869 gefunden im NCBI Database accession no. GPL84 -& ANONYMOUS: "GeneChip Pseudomonas aeruginosa Genome Array" AFFYMETRIX DATA SHEET, [Online] XP002331092 Gefunden im Internet: URL:http://www.affymetrix.com/support/tech nical/datasheets/pseudomonas_datasheet.pdf >
- WOLFGANG MATTHEW C ET AL: "Conservation of genome content and virulence determinants among clinical and environmental isolates of Pseudomonas aeruginosa." PROCEEDINGS OF THE NATIONAL ACADEMY OF SCIENCES OF THE UNITED STATES OF AMERICA, Bd. 100, Nr. 14, 8. Juli 2003 (2003-07-08), Seiten 8484-8489, XP002329865 ISSN: 0027-8424
- MILLER D A ET AL: "Analysis of virulence in a multiresistant and transmissible clone of Pseudomonas aeruginosa by differential genomic DNA hybridisation." ABSTRACTS OF THE GENERAL MEETING OF THE AMERICAN SOCIETY FOR MICROBIOLOGY, Bd. 103, 2003, Seiten B-289 URL, XP002329866 & 103RD AMERICAN SOCIETY FOR MICROBIOLOGY GENERAL MEETING; WASHINGTON, DC, USA; MAY 18-22, 2003 ISSN: 1060-2011
- WAGNER VICTORIA E ET AL: "Microarray analysis of Pseudomonas aeruginosa quorum-sensing regulons: Effects of growth phase and environment." JOURNAL OF BACTERIOLOGY, Bd. 185, Nr. 7, April 2003 (2003-04), Seiten 2080-2095, XP008047722 ISSN: 0021-9193
- DATABASE GENBANK [Online] 19. Februar 2003 (2003-02-19), STOVER, C.K. ET AL.: "Complete genome sequence of Pseudomonas aeruginosa PA01, an opportunistic pathogen" XP002329870 gefunden im GENBANK accession no. GI:9945818 Database accession no. AE004440

## Beschreibung

Die Erfindung betrifft ein Verfahren zur Geno- und Pathotypisierung von Bakterien der Spezies *Pseudomonas aeruginosa* mittels Hybridisierungsassays auf einem Biochip bzw. einem Microarray. Weiter betrifft die Erfindung spezifische Oligonukleotid-Sonden, die im Rahmen des Nachweisverfahrens eingesetzt werden können, sowie Biochips mit derartigen Oligonukleotid-Sonden.

*Pseudomonas aeruginosa* ist ein ubiquitärer Umweltkeim, der als opportunistisches Pathogen eine hohe Morbidität und Mortalität bei Patienten verursacht, die ein lokal oder systemisch geschwächtes Immunsystem haben. Zusätzlich nimmt *Pseudomonas aeruginosa* durch die chronische Besiedlung der Atemwege von Mukoviszidose-Patienten einen entscheidenden Einfluss auf den Verlauf der Erkrankung. Aufgrund der umfangreichen metabolischen und adaptiven Fähigkeiten von *Pseudomonas aeruginosa* ist die Behandlung einer Infektion oft sehr aufwändig und eine vollständige Elimination des Bakteriums oft nicht möglich.

Es wurde gezeigt, dass 70% der Infektionen mit *Pseudomonas aeruginosa* auf Intensivstationen von Erregern verursacht wurden, die bereits vorher, zum Teil sogar mehrfach oder mit mehreren Wochen Abstand, bei anderen Patienten nachgewiesen wurden. Derartige nosokomiale Infektionen bedeuten, neben allen unmittelbaren Konsequenzen für die betroffenen Patienten, einen immensen Kostenaufwand für das Gesundheitssystem. Aufgrund dieser hohen Rate an Übertragungen innerhalb des Klinikumfeldes besteht somit ein Bedarf an der Überwachung auftretender Infektionen sowie an der Vermeidung der Ausbreitung und Persistenz von Erregern im Zuge einer Hygienekontrolle.

Für eine erfolgreiche Infektionsvermeidung ist das Erkennen der Infektionsquellen essentiell, wobei zusätzlich zum Erregemachweis oft zu klären ist, ob mehrfach isolierte Stämme der selben Spezies einem gemeinsamen Klon entstammen oder unterschiedlichen Ursprungs sind. Diesbezügliche Untersuchungen werden unter dem Begriff "Erregertypisierung" zusammengefasst.

Eine verlässliche Typisierung von Stämmen von *Pseudomonas aeruginosa* ließ sich bislang nur mittels molekularbiologischer Methoden durchführen, die vergleichsweise kompliziert und teuer sind. So wurden bisher *Pseudomonas aeruginosa*-Isolate mit Hilfe einer Wechselfeldgelelektrophorese typisiert und den verschiedenen Subgruppen zugeordnet. Dafür wurde die genomische DNA des jeweiligen Stammes mit Restriktionsenzymen geschnitten und aufgetrennt. Eine solche Untersuchung setzt, neben dem Zeitaufwand von mehreren Wochen für jede Analyse, ein hohes Maß an Vorkenntnissen voraus und kann nur in wenigen Laboratorien durchgeführt werden.

Eine molekularbiologische Routine-Typisierung ist somit aufgrund der entstehenden Kosten bislang nicht gerechtfertigt. Es besteht somit ein Bedarf an Nachweisverfahren für *Pseudomonas aeruginosa,* die auf kostengünstiger Weise von nicht spezialisierten molekularbiologischen Routinelabors durchgeführt werden können.

Wolfgang, M.C. et al. (2003) Proc. Natl. Acad. Sci. USA, Bd. 100, Nr. 14, Seiten 8484-8489 beschreiben die Verwendung eines "whole genome" DNA-Microarrays zur Bestimmung des Genominhalts verschiedener *Pseudomonas aeruginosa*-Stämme, um festzustellen, ob eine Variation im genomischen Repertoire von *Pseudomonas aeruginosa*-Stämmen mit einer bestimmten Infektion assoziiert sein kann.

Der vorliegenden Erfindung liegt somit die Aufgabe zugrunde, ein Verfahren zum spezifischen Nachweis und zur Geno- und Pathotypisierung von Bakterienstämmen der Spezies *Pseudomonas aeruginosa* bereitzustellen, das mit relativ geringem technischen Aufwand und kostengünstig durchgeführt werden kann. Eine weitere Aufgabe der vorliegenden Erfindung ist es, eine Vorrichtung zum spezifischen Nachweis und zur Geno- und Pathotypisierung von Bakterienstämmen der Spezies *Pseudomonas aeruginosa* zur Verfügung zu stellen, die sich durch eine leichte Handhabbarkeit sowie durch eine Kompatibilität mit üblicherweise in molekularbiologischen Laboratorien verwendeten Geräten wie beispielsweise Tischzentrifugen und Pipetten auszeichnet.

Diese und weitere Aufgaben der vorliegenden Erfindung werden durch die Bereitstellung der in den Patentansprüchen angegebenen Gegenstände gelöst.

Bevorzugte Ausführungsformen sind in den Unteransprüchen definiert.

Die Aufgaben werden erfindungsgemäß dadurch gelöst, dass ein Biochip bzw. Nukleinsäure-Chip mit Oligonukleotid-Sonden für den spezifischen Nachweis von Bakterienstämmen der Spezies *Pseudomonas aeruginosa* bereitgestellt wird.

Eine Ausführungsform der Erfindung betrifft eine Mikroarray-Vorrichtung zur Geno- und Pathotypisierung der Spezies *Pseudomonas aeruginosa,* umfassend ein Trägerelement mit darauf auf vorbestimmten Bereichen immobilisierten Oligonukleotid-Sonden, wobei eine Oligonukleotide-Sonde die folgende Nukleinsäuresequenz (Sequenz in 5'→3'-Richtung) aufweist: GAAGCCCAGCAATTGCGTGTTTC.

Der erfindungsgemäße Nukleinsäure-Chip bietet den wesentlichen Vorteil, dass auf diese Weise *Pseudomonas aeruginosa* auf schnelle und einfache Weise in einem diagnostischen Routinelabor innerhalb eines Tages nachgewiesen werden kann. Insbesondere ermöglicht der erfindungsgemäße Nukleinsäure-Chip die Geno- und Pathotypisierung von *Pseudomonas aeruginosa.* Somit können auftretende Infektionen überwacht werden und bei Verdacht auf eine nosokomiale Ausbreitung dieses Erregers umgehend Vorkehrungen getroffen werden, um die Ausbreitung und Persistenz von *Pseudomonas aeruginosa* zu vermeiden.

Unter einem Nukleinsäure-Chip wird im Rahmen der vorliegenden Erfindung ein Trägerelement mit darauf auf vorbestimmten Bereichen immobilisierten Oligonukleotid-Sonden bezeichnet. Die vorbestimmten Bereiche auf dem Träger werden im Folgenden auch als Array-Elemente bezeichnet.

Die Verwendung eines Nukleinsäure-Chips zum spezifischen Nachweis der *Pseudomonas aeruginosa*-Stämme ermöglicht die Detektion der Wechselwirkungsreaktion zwischen den in der zu untersuchenden Probe vorliegenden Target-Nukleinsäuren und Oligonukleotid-Sonden durch übliche Verfahren wie beispielsweise durch Fluoreszenzdetektion oder radiochemische Methoden. Als besonders vorteilhaft hat sich die Anwendung von Absorptionsmessungen erwiesen, da diese besonders kostengünstig durchzuführen sind. Eine derartige Absorptionsmessung kann durch Verwendung einer reaktiven Färbemethode, die an den Oberflächenbereichen stattfindet, an denen eine Wechselwirkungsreaktion stattgefunden hat, wesentlich verbessert und verbilligt werden. Hier hat sich u.a. die Abscheidung von Silber an mit Goldnanokügelchen markierten Targetmolekülen bewährt (siehe DE 100 33 334.6 und WO 02/02810). Zum Nachweis der Silberabscheidung kann ein Gerät verwendet werden, das eine oder mehrere Leuchtdioden beliebiger geeigneter Emissionswellenlänge als Lichtquelle verwendet und beispielsweise eine CCD-Kamera zur ortsaufgelösten Detektion der Wechselwirkungsreaktion auf den vorbestimmten Bereichen des Chips aufweist.

Zur Beschreibung der vorliegenden Erfindung werden u.a. folgende Definitionen verwendet:
Unter einem Mikroarray bzw. Microarray bzw. Sonden-Array wird im Rahmen der vorliegenden Erfindung eine Anordnung von molekularen Sonden bzw. einer Substanzbibliothek auf einem Träger verstanden, wobei die Position einer jeden Sonde separat bestimmt ist. Vorzugsweise umfasst der Array definierte Stellen bzw. vorbestimmte Bereiche, so genannte Array-Elemente, die besonders bevorzugt in einem bestimmten Muster angeordnet sind, wobei jedes Array-Element üblicherweise nur eine Spezies an Sonden beinhaltet. Die Anordnung der Moleküle bzw. Sonden auf dem Träger kann dabei durch kovalente oder nicht-kovalente Wechselwirkungen erzeugt werden. Eine Position innerhalb der Anordnung, d.h. des Arrays, wird üblicherweise als Spot bezeichnet. Der Sonden-Array bildet somit die Detektionsfläche.

Unter einem Array-Element bzw. einem vorbestimmten Bereich bzw. einem Spot wird im Rahmen der vorliegenden Erfindung ein für die Deposition einer molekularen Sonde bestimmtes Areal bzw. nach der Deposition mit einer oder mehreren definierten molekularen Sonden belegtes Areal auf einer Oberfläche verstanden, die Summe aller belegten Array-Elemente ist das Sonden-Array bzw. Microarray.

Unter einer Sonde bzw. Oligonukleotid-Sonde wird im Rahmen der vorliegenden Erfindung ein Molekül verstanden, das zum Nachweis anderer Moleküle durch ein bestimmtes, charakteristisches Bindungsverhalten bzw. eine bestimmte Reaktivität verwendet wird. Für die auf dem Array angeordneten Sonden kommt jede Art von Nukleinsäuren und/oder deren Analoga in Frage, die sich an feste Oberflächen koppeln lassen und eine spezifische Affinität aufweisen. Die Oligonukleotide können DNA-Moleküle, RNA-Moleküle und/oder deren Analoga wie z.B. artifizielle bzw. modifizierte Nukleotide umfassen. Beispielsweise kann es sich bei den Oligonukleotid-Sonden um Oligonukleotide mit einer Länge von 10 bis 100 Basen, vorzugsweise 15 bis 50 Basen und besonders bevorzugt von 20 bis 30 Basen Länge handeln, die auf der Array-Oberfläche immobilisiert sind.

Typischerweise handelt es sich erfindungsgemäß bei den Oligonukleotid-Sonden um einzelsträngige Nukleinsäuremoleküle oder Moleküle von Nukleinsäureanaloga, bevorzugt einzelsträngige DNA-Moleküle oder RNA-Moleküle, die mindestens über einen Sequenzbereich verfügen, der zu einem Sequenzbereich der Target-Nukleinsäuren komplementär ist. Je nach Nachweisverfahren und Anwendung können die Oligonukleotid-Sonden auf einem festen Trägersubstrat z.B. in Form eines Microarrays immobilisiert sein. Darüber hinaus können sie je nach Nachweisverfahren radioaktiv oder nicht radioaktiv markiert sein, so dass sie über eine im Stand der Technik übliche Nachweisreaktion nachgewiesen werden können.

Unter einem Target bzw. einer Target-Nukleinsäure wird im Rahmen der vorliegenden Erfindung insbesondere eine im Genom von *Pseudomonas aeruginosa* vorliegende Nukleinsäure verstanden, die Hinweise auf die Identität eines in der Probe vorliegenden Stamms der Spezies *Pseudomonas aeruginosa,* von krankheitsassoziierten Genen und/oder auf die Identität des vorliegenden Flagellen-Typs liefert. Die Target-Nukleinsäuren umfassen in der Regel Sequenzen mit einer Länge von 40 bis 10.000 Basen, bevorzugt von 60 bis 2.000 Basen, ebenfalls bevorzugt von 60 bis 1.000 Basen, insbesondere bevorzugt von 60 bis 500 Basen und am meisten bevorzugt von 60 bis 150 Basen. Ihre Sequenz beinhaltet ggf. die Sequenzen von Primern sowie die durch die Primer definierten Sequenzbereiche des Templates. Bei den Target-Nukleinsäuren kann es sich insbesondere um einzel- oder doppelsträngige Nukleinsäuremoleküle handeln, von denen ein oder beide Stränge nach einer erfolgten geeigneten Behandlung, wie sie z.B. im Stand der Technik beschrieben ist, markiert sind, so dass sie in einem der im Stand der Technik üblichen Nachweisverfahren nachgewiesen werden können. Besonders bevorzugt handelt es sich bei Target-Nukleinsäuren um Nukleinsäuren, die in mindestens 30% der Population von *Pseudomonas aeruginosa* eine Basensubstitution verglichen mit der Sequenz des Genoms des Referenzstamms PAO1 (siehe www.pseudomonas.com) von *Pseudomonas aeruginosa* aufweisen; um Nukleinsäuren, die in nicht in allen Stämmen der Spezies *Pseudomonas aeruginosa* vorkommen; um Nukleinsäuren, die in Pathogenitätsinseln im Genom von *Pseudomonas aeruginosa* vorliegen; um Nukleinsäuren, die in krankheitsassoziierten Genen wie *exoS* und exo*U* vorliegen; sowie um Nukleinsäuren, die in für Flagellen von *Pseudomonas aeruginosa* kodierenden Genen enthalten sind.

Als Target-Sequenz wird erfindungsgemäß der Sequenzbereich des Targets bezeichnet, der durch Hybridisierung mit der Sonde nachgewiesen wird. Erfindungsgemäß wird auch davon gesprochen, dass dieser Bereich durch die Sonde adressiert wird.

Unter einer Substanzbibliothek wird im Rahmen der vorliegenden Erfindung eine Vielzahl von unterschiedlichen Sondenmolekülen verstanden, vorzugsweise mindestens 2 bis 1.000.000 unterschiedliche Moleküle, besonders bevorzugt mindestens 10 bis 10.000 unterschiedliche Moleküle und am meisten bevorzugt zwischen 50 und 1.000 unterschiedlichen Molekülen. Bei speziellen Ausgestaltungen kann eine Substanzbibliothek auch nur mindestens 50 oder weniger oder mindestens 30.000 unterschiedliche Moleküle umfassen. Die Substanzbibliothek ist vorzugsweise als Array auf einem Träger in der Reaktionskammer der erfindungsgemäßen Vorrichtung angeordnet. Die Anordnung der Substanzen bzw. Sondenmoleküle auf dem Träger erfolgt bevorzugt derart, dass jeder Substanz bzw. jeder Spezies von Sondenmolekülen ein bestimmter, eindeutig zu identifizierender Ort zugeordnet ist und dass jede Substanz bzw. jede Spezies von Sondenmolekülen getrennt von den anderen immobilisiert ist.

Unter einem Trägerelement bzw. Träger bzw. Substanzbibliothekenträger wird im Rahmen der vorliegenden Erfindung ein Festkörper verstanden, auf dem das Sonden-Array aufgebaut ist. Bei dem Träger, der üblicherweise auch als Substrat oder Matrix bezeichnet wird, kann es sich z.B. um Objektträger oder Wafer oder aber auch keramische Materialien handeln. Die Gesamtheit aus in Array-Anordnung auf der Detektionsfläche abgelegten Molekülen bzw. der in Array-Anordnung auf der Detektionsfläche abgelegten Subtanzbibliothek und dem Träger wird häufig auch als "Nukleinsäure-Chip", "Chip", "Biochip", "Mikroarray", "Microarray", "DNA-Chip", "Sondenarray" etc. bezeichnet.

Herkömmliche Nukleinsäure-Chips bzw. Arrays bzw. Microarrays im Rahmen der vorliegenden Erfindung umfassen etwa 10 bis 5.000, vorzugsweise 20 bis 500 und besonders bevorzugt 50 bis 100 unterschiedliche Spezies von Oligonukleotid-Sonden auf einer, vorzugsweise quadratischen, Fläche von z.B. 1 mm bis 4 mm x 1 mm bis 4 mm, vorzugsweise von 2 mm x 2 mm oder etwa 17,64 mm². In weiteren Ausgestaltungen umfassen Mikroarrays im Rahmen der vorliegenden Erfindung etwa 50 bis etwa 80.000, vorzugsweise etwa 100 bis etwa 65.000, besonders bevorzugt etwa 1.000 bis etwa 10.000 unterschiedliche Spezies von Sondenmolekülen auf einer Fläche von mehreren mm² bis mehreren cm², vorzugsweise etwa 1 mm² bis 10 cm², besonders bevorzugt etwa 2 mm² bis etwa 1 cm² und am meisten bevorzugt etwa 4 mm² bis etwa 6,25 mm². Beispielsweise weist ein herkömmliches Mikroarray von 100 bis 65.000 unterschiedliche Spezies von Sondenmolekülen auf einer Fläche von etwa 2,4 mm x etwa 2,4 mm auf. Weitere beispielhafte Größen der Flächen des Mikroarrays bzw. der Flächen für die Synthese der Biopolymere sind etwa 1 bis 10 mm x etwa 1 bis 10 mm, vorzugsweise etwa 2,4 bis 5 mm x etwa 2,4 bis 5 mm und besonders bevorzugt etwa 3,5 bis 4,5 mm x etwa 3,5 x 4,5 mm.

Eine Markierung bezeichnet im Rahmen der vorliegenden Erfindung eine detektierbare Einheit, beispielsweise ein Fluorophor oder eine Ankergruppe, an die eine detektierbare Einheit oder ein die Umsetzung eines löslichen Edukts bzw. Substrats in ein unlösliches Produkt katalysierender Katalysator oder Kristallisationskeim gekoppelt werden kann.

Unter einem Edukt bzw. Substrat (im Sinne eines enzymatischen Substrats) wird im Rahmen der vorliegenden Erfindung ein im Reaktionsmedium gelöst vorliegendes Molekül bzw. eine Kombination von Molekülen verstanden, das bzw. die mit Hilfe eines Katalysators bzw. eines Kristallisationskeimes und/oder eines umsetzenden Agens lokal abgeschieden wird. Das umsetzende Agens kann beispielsweise ein reduzierenden Agens wie bei der Silberabscheidung oder ein oxidierendes Agens wie bei der Erzeugung eines Farbstoffs durch enzymatische Oxidation sein.

Als Probe bzw. Probenlösung bzw. Analyt wird im Rahmen der vorliegenden Erfindung die zu analysierende Flüssigkeit mit den nachzuweisenden und ggf. zu amplifizierenden Targetmolekülen bezeichnet.

Eine Vervielfältigungsreaktion bzw. eine Amplifikationsreaktion umfasst im Rahmen der vorliegenden Erfindung üblicherweise 10 bis 50 oder mehr Amplifikationszyklen, vorzugsweise etwa 25 bis 45 Zyklen, besonders bevorzugt etwa 40 Zyklen. Eine zyklische Amplifikationsreaktion ist im Rahmen der vorliegenden Erfindung vorzugsweise eine Polymerase-Kettenreaktion (polymerase chain reaction, PCR).

Als Primer wird üblicherweise ein kurzes DNA- oder RNA-Oligonukleotid mit etwa 12 bis 30 Basen bezeichnet, das komplementär zu einem Abschnitt eines größeren DNA- oder RNA-Moleküls ist und über eine freie 3'-OH-Gruppe an seinem 3'-Ende verfügt. Aufgrund dieser freien 3'-OH-Gruppe kann der Primer als Substrat für beliebige DNA- oder RNA-Polymerasen dienen, die in 5'-3'-Richtung Nukleotide an den Primer synthetisieren. Die Sequenz der neu synthetisierten Nukleotide ist dabei durch die Sequenz des mit dem Primer hybridisierten Templates vorgegeben, die jenseits der freien 3'-OH-Gruppe des Primers liegt. Primer üblicher Länge umfassen zwischen 12 bis 50 Nukleotide, bevorzugt zwischen 15 und 30 Nukleotide.

Als Template oder Template-Strang wird üblicherweise ein doppelsträngiges Nukleinsäuremolekül oder ein Nukleinsäurestrang bezeichnet, der als Vorlage zur Synthese von komplementären Nukleinsäuresträngen dient.

Als Hybridisierung wird die Bildung von doppelsträngigen Nukleinsäuremolekülen oder Duplexmolekülen aus komplementären einzelsträngigen Nukleinsäuremolekülen bezeichnet. Dabei findet die Assoziation vorzugsweise immer zu Paaren von A und T bzw. G und C statt. Eine Assoziation kann vorzugsweise auch über nicht klassische Basenpaarungen wie Wobbelbasenpaarungen z.B. zwischen Inosin und G bzw. Inosin und C erfolgen. Im Rahmen einer Hybridisierung können z.B. DNA-DNA-Duplexes, DNA-RNA- oder RNA-RNA-Duplexes gebildet werden. Durch eine Hybridisierung können auch Duplexes mit Nukleinsäureanaloga gebildet werden, wie z.B. DNA-PNA-Duplexes, RNA-PNA-Duplexes, DNA-LNA-Duplexes und RNA-LNA-Duplexes. Hybridisierungsexperimente werden üblicherweise benutzt, um die Sequenzkomplementarität und damit die Identität zwischen zwei verschiedenen Nukleinsäuremolekülen nachzuweisen.

Dabei bedeutet "spezifische Hybridisierung" das unter den hier beschriebenen oder dem Durchschnittsfachmann im Zusammenhang mit *in situ-* und *in-vitro-*Hybridisierungstechniken bekannten stringenten Hybridisierungsbedingungen die Target-Nukleinsäuren stärker an die Sonde binden als die Nicht-Target-Nukleinsäuren und vorzugsweise im wesentlichen nur die Target-Nukleinsäuren, nicht aber Nicht-Target-Nukleinsäuren an die Sonde binden.

Bei einem Aspekt der vorliegenden Erfindung wird somit eine Microarray-Vorrichtung, umfassend ein Trägerelement mit darauf auf vorbestimmten Bereichen immobilisierten Sonden für den spezifischen Nachweis von Bakterienstämmen der Spezies *Pseudomonas aeruginosa,* bereitgestellt. Die Gesamtheit aus in vorbestimmten Bereichen bzw. in Array-Anordnung auf der Detektionsfläche abgelegten Sonden für den spezifischen Nachweis von Bakterienstämmen der Spezies *Pseudomonas aeruginosa* und dem Träger wird im folgenden auch als "Nukleinsäure-Chip", "Chip", "Biochip", "Microarray", "Sondenarray" etc. bezeichnet.

Als Nukleinsäure-Chips können im Rahmen der vorliegenden Erfindung insbesondere Chips eingesetzt werden, wie sie von den Firmen Affymetrix (Santa Clara, Kalifornien, USA) und Clondiag (Jena, Deutschland) vertrieben werden. Beispielsweise werden erfindungsgemäß Nukleinsäurechips eingesetzt, die in Microarray-Vorrichtungen implementiert sind, wie sie in den internationalen Patentanmeldungen WO 01/02094 und WO 03/031063 beschrieben sind. Auf die in diesen Dokumenten enthaltene Offenbarung bezüglich der Anordnung des Chips in einer Vorrichtung wird hiermit ausdrücklich Bezug genommen.

Besonders bevorzugt werden im Rahmen der vorliegenden Erfindung Vorrichtungen mit Nukleinsäure-Chips eingesetzt, wie sie in der internationalen Patentanmeldung WO 03/059516 beschrieben sind. Auf die in diesem Dokument enthaltene Offenbarung bezüglich einer Vorrichtung zur Durchführung von Array-Verfahren wird hiermit ebenfalls ausdrücklich Bezug genommen.

Insbesondere wird somit als Vorrichtung zum Nachweis von Bakterienstämmen der Spezies *Pseudomonas aeruginosa* ein, z.B. in WO 03/059516 beschriebenes, Reaktionsgefäß eingesetzt, welches eine für ein Laborreaktionsgefäß typische Form und/oder typische Größe aufweist, und bei dem auf einer seiner Grundflächen ein Trägerelement mit darauf auf vorbestimmten Bereichen immobilisierten Oligonukleotid-Sonden für den spezifischen Nachweis von Bakterienstämmen der Spezies *Pseudomonas aeruginosa* angeordnet ist.

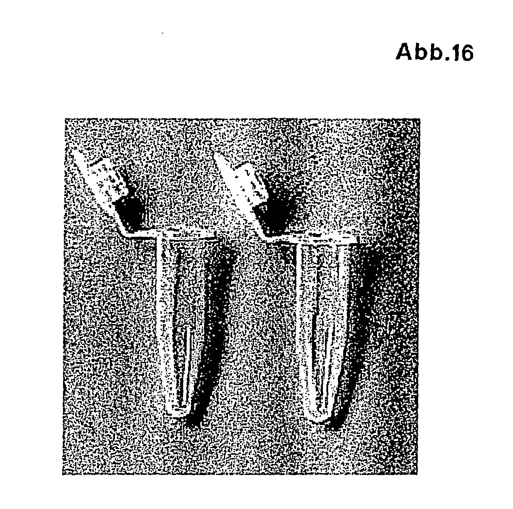

Unter Laborreaktionsgefäßen mit einer typischen Form und Größe werden im Rahmen der vorliegenden Erfindung Reaktionsgefäße verstanden, die als Einweg-Reaktionsgefäße, in der Standardausführung 1,5 ml fassend, in, insbesondere biologischen bzw. molekularbiologischen, Laboratorien üblicherweise verwendet werden. Derartige Laborreaktionsgefäße werden auch als Tubes und, nach dem bedeutendsten Hersteller, insbesondere als Eppendorf-Tubes oder "Eppis" (Hamburg, Deutschland) bezeichnet. So werden Laborreaktionsgefäße mit einer typischen Form und Größe von Eppendorf als Standard-Reaktionsgefäße oder Safe-Lock-Reaktionsgefäße angeboten. Selbstverständlich können im Rahmen der vorliegenden Erfindung auch Reaktionsgefäße von Herstellern wie Greiner (Frickenhausen, Deutschland), Millipore (Eschborn, Deutschland), Heraeus (Hanau, Deutschland) und BIOplastics (Landgraaf, Niederlande) sowie anderen Herstellern eingesetzt werden, die eine Form und Größe aufweisen, wie sie für Laborreaktionsgefäße insbesondere von Eppendorf typisch ist. Beispiele für Laborreaktionsgefäße mit einer typischen Form und Größe sind in Abbildung 16 gezeigt.

Unter Laborreaktionsgefäßen typischer Form und Größe werden im Rahmen der vorliegenden Erfindung insbesondere nicht Rundkolben oder andere Kolben wie Erlenmeyerkolben, Bechergläser oder Messzylinder verstanden.

Ein Reaktionsgefäß im Rahmen der vorliegenden Erfindung unterscheidet sich von den vorstehenden genannten Reaktionsgefäßen dadurch, dass auf einer seiner Grundflächen ein Trägerelement mit darauf auf vorbestimmten Bereichen immobilisierten Sondenmolekülen angeordnet ist. Trotz der Modifizierung eines herkömmlichen Laborreaktionsgefäßes durch Einbau eines derartigen Chips weist das Reaktionsgefäß eine für ein Laborreaktionsgefäß typische Form und/oder Größe auf. Das Reaktionsgefäß weist somit eine rotationssymmetrische Form, insbesondere eine zylindrische bzw. im Wesentlichen zylindrische Form auf. Von den für herkömmliche Laborreaktionsgefäße typischen Formen und damit für das erfindungsgemäße Reaktionsgefäß denkbaren Formen ist ferner eine von der zylindrischen Grundform abweichende konische Form umfasst, wobei die Verjüngung vorzugsweise in Richtung der Affinitätsmatrix auftritt. Typische Formen sind ferner Kombinationen von zylindrischen bzw. im Wesentlichen zylindrischen Bereichen und konischen Bereichen (siehe u.a. Abbildungen 1-4 und 21 in WO 03/059516). Aufgrund der für Laborreaktionsgefäße typischen Form und Größe ist das Reaktionsgefäß mit dem darin eingebrachten Chip insbesondere mit üblichen Tischzentrifugen wie beispielsweise von Herstellern wie Eppendorf oder Heraeus kompatibel, d.h. das Reaktionsgefäß mit Nukleinsäure-Chip ist zur Zentrifugation in üblichen Tischzentrifugen geeignet. Übliche maximale Außendurchmesser für Standard-Laborreaktionsgefäße und damit auch für das Reaktionsgefäß mit Nukleinsäure-Chip liegen im Bereich von 0,8 cm bis 2 cm, vorzugsweise 1,0 cm bis 1,5 cm und besonders bevorzugt 1,1 cm bis 1,3 cm. Weitere bevorzugte Außendurchmesser sind bis 0,9 cm, bis 1,2 cm, bis 1,4 cm, bis 1,6 cm und bis 1,7 cm. Die Höhe des erfindungsgemäßen Reaktionsgefäßes beträgt üblicherweise 1,5 cm bis 5,0 cm, vorzugsweise 2,0 cm bis 4,0 cm, besonders bevorzugt 2,5 cm bis 3,5 cm, und am meisten bevorzugt 2,8 cm bis 3,2 cm. Weitere bevorzugte Höhen sind bis 2,6 cm, bis 2,7 cm, bis 2,9 cm, bis 3,0 cm, bis 3,1 cm, bis 3,3 cm und bis 3,4 cm. In speziellen Ausgestaltungen kann die Höhe auch 1,0 cm oder mehr betragen.

Das Reaktionsgefäß mit Nukleinsäure-Chip ist in üblichen Tischzentrifugen zentrifugierbar und kann somit beispielsweise in herkömmlichen Tischzentrifugen wie einer Standard-Tischzentrifuge mit Standard-Rotor von Eppendorf sowie auch in üblichen Racks und Haltern für Reaktionsgefäße wie beispielsweise einem Tube-Rack von Eppendorf eingesetzt werden. Zum Einbringen der zu untersuchenden Probe sowie anderer zur Durchführung der Nachweisreaktion erforderlicher Reagenzien in das Reaktionsgefäß mit Nukleinsäure-Chip können übliche Pipetten oder Spritzen wie beispielsweise variable und Fixvolumen-Pipetten von Eppendorf verwendet werden.

Das Reaktionsgefäß mit Nukleinsäure-Chip weist eine für ein Laborreaktionsgefäß typische Größe auf. Typische Füllvolumina liegen im Bereich von 100 µl bis 2,5 ml, können aber bei speziellen Ausgestaltungen auch höher oder niedriger sein. Besonders bevorzugt hat das Reaktionsgefäß ein für ein Standard-Eppendorf-Tube übliches Füllvolumen von bis zu 1,5 ml. Weitere bevorzugte Füllvolumina sind bis 0,25 ml, bis 0,4 ml, bis 0,5 ml, bis 0,7 ml, bis 1,0 ml oder bis 2,0 ml.

Bei einer speziellen Ausführungsform der erfindungsgemäßen Vorrichtung wird ein Nukleinsäure-Chip eingesetzt, bei dem ein Glas-Träger mit den darauf immobilisierten Oligonukleotiden direkt in ein 1,5 ml Reaktionsgefäß, wie es in der internationalen Patentanmeldung WO 03/059516 beschrieben ist, eingebunden ist. Derartige Reaktionsgefäße mit Nukleinsäure-Chips werden von der Firma Clondiag beispielsweise als ArrayTube® vertrieben.

Wie bereits vorstehend erwähnt, kann es sich bei der Nukleinsäuresonde im Sinne der vorliegenden Erfindung um eine DNA- oder RNA-Sonde handeln, die in der Regel zwischen 12 und 100 Nukleotide umfassen wird, bevorzugt zwischen 15 und 50 und besonders bevorzugt zwischen 17 und 25 Nukleotide. Komplementarität sollte vorzugsweise bei einer Sonde von 15 bis 25 Nukleotiden über 100% der Sequenz gegeben sein.

Die Auswahl der Nukleinsäuresonden geschieht insbesondere unter dem Gesichtspunkt, ob eine komplementäre Sequenz in dem nachzuweisenden Stamm von *Pseudomonas aeruginosa* vorliegt.

Durch eine wie z.B. im Folgenden beschrieben ausgewählte, definierte Sequenz werden vorzugsweise mindestens 20% oder mindestens 25% und besonders bevorzugt mindestens 30% oder mindestens 35% und am meisten bevorzugt mindestens 45% oder mindestens 50% der Population von Stämmen von *Pseudomonas aeruginosa* erfasst. Derartige ausgewählte bzw. definierte Sondensequenzen liefern zwar nicht ein für einen einzigen Stamm charakteristisches Signal. Durch eine Vielzahl unterschiedlicher Spezies von derartig definierten Sonden auf der Chip-Oberfläche wird jedoch ein Signalmuster bereitgestellt, das bei geeigneter Anzahl, z.B. etwa 50 oder etwa 70, verschiedener Sondensequenzen für jeden Stamm charakteristisch ist.

Sonden, die eine Auswahl von mehr als 70% der Population von Stämmen von *Pseudomonas aeruginosa* erfassen, sind allerdings weniger bevorzugt, da die Diskriminierung einzelner Stämme durch diese Sonden zu gering sein könnte.

Sonden, die eine Auswahl von weniger als 20% der Population von *Pseudomonas aeruginosa* erfassen, sind ebenfalls weniger bevorzugt, da diese zwar eine hohe Selektivität aufweisen, aber nur für wenige Stämmen ein Signal liefern und somit für den überwiegenden Teil von *Pseudomonas aeruginosa*-Stämmen nicht zur Information beitragen.

Insbesondere sind die Oligonukleotid-Sonden des erfindungsgemäßen Nukleinsäure-Chips spezifisch für Nukleinsäuren, die eine Basensubstitution verglichen mit der Sequenz des Referenzstamms von *Pseudomonas aeruginosa* aufweisen. Als Referenzstamm wird die Sequenz des Genoms des Stamms PAO1 herangezogen, die unter http://www.pseudomonas.com zugänglich ist. Vorzugsweise sind die Oligonukleotid-Sonden spezifisch für Nukleinsäuren, die eine Basensubstitution verglichen mit der Sequenz von konservierten Genen des Referenzstamms PAO1 von *Pseudomonas aeruginosa* aufweisen. Ferner ist es bevorzugt, dass die Basensubstitution in mindestens 20%, mindestens 25%, mindestens 30%, mindestens 35%, mindestens 40% und besonders bevorzugt mindestens 50% einer Population von Pseudomonas aeruginosa auftritt. Das heißt, zur Typisierung werden erfindungsgemäß insbesondere Single Nucleotide Polymorphisms (SNP's) aus konservierten *Pseudomonas aeruginosa*-Genen ausgewählt, die z.B. in mindestens 30% und besonders bevorzugt mindestens 50% der Population eine Basensubstitution aufweisen. Auf diese Weise können Stämme von *Pseudomonas aeruginosa* mit einer Nachweisgenauigkeit von mehr als 99,7% bestimmt bzw. identifiziert werden.

Bei einer weiteren Ausführungsform umfasst der erfindungsgemäße Nukleinsäure-Chip, insbesondere zusätzlich zu den vorstehend beschriebenen Sonden, Oligonukleotid-Sonden, die für Nukleinsäuren spezifisch sind, nicht in allen Stämmen der Spezies *Pseudomonas aeruginosa* und vorzugsweise in mindestens 30% oder mindestens 50% der Population vorkommen.

Bei einer weiteren bevorzugten Ausführungsform umfasst der erfindungsgemäße Nukleinsäure-Chip Oligonukleotid-Sonden, die spezifisch für Nukleinsäuren sind, die in Pathogenitätsinseln im Genom von *Pseudomonas aeruginosa* vorliegen. Pathogenitätsinseln stellen distinkte DNA-Bereiche im Genom pathogener Bakterien dar, die sich vom restlichen Genom durch die Präsenz mehrerer Pathogenitätsassoziierter Gene und durch eine Reihe weiterer struktureller Besonderheiten unterscheiden. Insbesondere weisen verschiedene *Pseudomonas aeruginosa*-Stämme eine bemerkenswerte genomische Diversität auf, die im Wesentlichen durch die Insertion oder Deletion von mobilen DNA-Einheiten wie (Pro)phagen, Plasmiden oder anderen Elementen verursacht wird. Derartige Pathogenitätsinseln liefern somit ebenfalls wertvolle Informationen für die Diskriminierung von unterschiedlichen Stämmen von *Pseudomonas aeruginosa.*

Bei einer weiteren Ausführungsform umfasst der erfindungsgemäße Nukleinsäure-Chip, insbesondere zusätzlich zu den für die Diskriminierung unterschiedlicher *Pseudomonas aeruginosa*-Stämme geeigneten Sonden, Oligonukleotid-Sonden, die für Nukleinsäuren spezifisch sind, die in krankheitsassoziierten Genen wie *exoS* und exoUvorliegen. Das Wissen über das Vorliegen bestimmter krankheitsassoziierter Gene lässt Aussagen über die Prognose des erkrankten Patienten zu und erleichtert somit die weitere Behandlung.

Bei einer weiteren Ausführungsform umfasst der erfindungsgemäße Nukleinsäure-Chip Oligonukleotid-Sonden, die für Nukleinsäuren spezifisch sind, die in für Flagellen von *Pseudomonas aeruginosa* kodierenden Genen enthalten sind. Für *Pseudomonas aeruginosa* liegen zwei verschiedene Typen von Flagellen vor. Die Information über den Flagellentyp des detektierten *Pseudomonas aeruginosa*-Stamm kann dem Arzt einen Hinweis für entsprechend einzusetzende Impfstoffe geben.

Bei Verwendung eines Nukleinsäure-Chips, der sämtliche Kategorien der vorstehend beschriebenen Oligonukleotid-Sonden umfasst, d.h. Sonden, die für SNP's spezifisch sind; Sonden, die für Nukleinsäuren spezifisch sind, die nicht in allen Stämmen von *Pseudomonas aeruginosa* vorliegen; Sonden, die für Nukleinsäuren in Pathogenitätsinseln spezifisch sind; Sonden, die für krankheitsassoziierte Gene spezifisch sind; und Sonden, die für Flagellen-kodierende Gene spezifisch sind, erhöht sich die Genauigkeit bei der Bestimmung von *Pseudomonas aeruginosa*-Stämme auf mehr als 99,9%.

Insbesondere haben die Oligonukleotid-Sondenmoleküle die nachstehend angegebenen Längen von Sequenzen (alle Nukleinsäuremoleküle sind in 5'-3'-Richtung notiert). Die erfindungsgemäßen Oligonukleotid-Sondenmoleküle sind zum spezifischen Nachweis von Bakterienstämmen der Spezies *Pseudomonas aeruginosa* und insbesondere zur Geno- und Pathotypisierung der Spezies *Pseudomonas aeruginosa* geeignet und werden dementsprechend insbesondere in dem erfindungsgemäßen Nachweisverfahren eingesetzt. Des Weiteren sind die im Folgenden aufgeführten Oligonukleotid-Sonden aber auch für den Einsatz in beliebigen anderen dem Fachmann bekannten Verfahren zum Nachweis bzw. der Markierung von Bakterienstämmen der Spezies *Pseudomonas aeruginosa* geeignet.

Im Folgenden werden die Oligonukleotide bzw. Nukleinsäure-Sondenmoleküle aufgeführt, die bzw. deren nachstehend beschriebene Abwandlungen für die Geno- und Pathotypisierung von *Pseudomonas aeruginosa* geeignet sind:
GCGGAAAACTTCCTGCACATGATGTT
GCGGAAAACTTCCTCCACATGATGTT
AGCTCAGCAGACTGCTGACGAGG
AGCTCAGCAGACCGCTGACGAG
AAGAGGACGGCCGCCGGGTGACGCC
AAGAGGACGGCCGCCAGGTGACGCCG
GACAAGATGCGCCTCGACGACC
GACAAGATGCGTCTCGACGACCG
AGCCGACCTACGCGCCGGGCAG
CAGCCGACCTATGCGCCGGGCAG
CCGTTCGAACGGCTCATGGAGCA
GCCGTTCGAACGACTCATGGAGCA
TGGAGCAGCAAGTGTTCCCGGC
TGGAGCAGCAACTGTTCCCGGC
GAACAAGACCGGTTCCACCAACGG
AACAAGACCGGCTCCACCAACGG
GCGACCTGGGCCTGGTGATCCT
GCGACCTGGGACTGGTGATCCT
GCCGACCAACTGAACTCCAACTCG
GTCGCTGAACGGCACCTACTTCA
CAGCCTGCGGTCATGTCCTCGG
CGCCAGTTTGAGAACGGAGTCACC
GCGCGATCTTCTCCACTTCATCGG
GCCTCCGCGATTGAACATCGTGAT
GTAGCCGGAGTCGAGCGGAATCAT
GTGAGCATGGAATCGGCAGTCGTT
CGAGGAGTTTCGGACCCGCTTTGA
AATAGGACCGGCAGAACGGGCATT
GCGCCTTCTCCTCTTTGCAGATGT
CAGTATGGTACGGACACGAAGCGC
GCATCATTGCGCGTCACATCTGGT
TCTGAACTGCGGCTATCACCTGGA
AATTGATGGCTTCTCAGGCGCAGG
AGTCATGGGACTGAATACGGCGACT
TTCTCGGTGTCGAGGGATTCTCGG
TGGTAGCTCTCGACGTACTGGCTG
CCCGTTGCTCATAACCCGTTCCTG
AGGGCATTCTCAGGTGGACTCAGG
ACCTGTGTCGCTGGAGGGTATGTT
AGCGTCCCTGACCAACCTCATCAG
CGCCAACAATTCGCCATTACAGCG
TCCAACAGGCAGGAGTACAGGGTG
CGCTGCACATACAGGTCCGTTCTC
AGCCCAGCAATTGCGTGTTTCTCCG
AGCCCAGCAACTGCGTGTTTCTCC
GCTGCTGGCGGCGGTGTGC
TGCTGCTGGCAGCGGTGTGCT
CAGAAAGCTCAGCAGACTGCTGACGAG
GAAAGCTCAGCAGACCGCTGACGAG
ACGGCCGCCGGGTGACGCC
ACGGCCGCCAGGTGACGCCG
GCCGACCTACGCGCCGGGC
AGCCGACCTATGCGCCGGGCA
GTTCGAACGGCTCATGGAGCAGCA
GTTCGAACGACTCATGGAGCAGCAAG
CAGCCCAGTCAGGACGCGCA
AGTGACGTGCGTTTCAGCAGTCCC
GTGTCACGGCCCATGTCTAGCAGC
CGAAGTCTGAGGTGTGGACCCGC
CGCTGGAGGGTATGTTCCGCAAGG
CGTACTCAGCTTCTCCACCCAGCG
CCTGGACCTCTCCAAGGTTCGCCT
GCCATTCCGACGACCAAACAAGGC

Daneben ist auch der Einsatz von Oligonukleotid-Sonden bzw. deren nachstehend beschriebenen Abwandlungen im Rahmen der vorliegenden Erfindung denkbar, die für die folgenden Nukleinsäuren spezifisch sind:
GTCTCCCTGGAGCCTGCGAAAGTGGCTCGGTTGCGTAGCCGAC

ATGTTGTATTTTTCTTGCGGTATGAAGATGGGTGGTTGGGTCGGATATAG
GTACTTCTCTCTATTTTCTTTAATTGCTCTTATCTATGG

GACCTCGACCCCCGAGGGCTTCATGGCGTGTCGCGAACTCGCATGGCAAC
AGGC

TGGTCAGCCGAGTAACCGGCAGTTGTCGCCAGGTCTGGAGAATCCCGCC
ATTAGCTTGATTCGACGGAACTATAGCGACTTTGGTCCAACTCTGGCCCA
G

ATGGGCAAGAGAGTGGTTGTATTGCTATGGCTGCTATTCACATCAATGTC
AGCCCACGCCATCGATAAAAAAGTCAA

CGGCTCGGACATGGCCAATTGGGTCAGCAAGCAACGCGCCGGAGGCATG
CCTGGGTTCGCCAGGGGCGGTGCC

GTTCCTGGAACGAGGGTGATGGCTGGGAATACGTGGAGGCGCCACAGCC
G

ATGTTCGTACATGACAAGCGACTGCAGTACACCGTCAGGGTCGC

Die folgenden Oligonukleotid-Sonden bzw. deren nachstehend beschriebene Abwandlungen sind vor allem zum spezifischen Nachweis von SNPs in konservierten Genen von *Pseudomonas aeruginosa* geeignet:

| | |
|---|---|
| oriC T-C_wt | GAAGCCCAGCAATTGCGTGTTTC |
| oriC T-C_mut_1 | GAAGCCCAGCAACTGCGTGTTTC |
| oriC T-C_wt_1 | AGCCCAGCAATTGCGTGTTTCTCCG |
| oriC T-C_mut_2 | AGCCCAGCAACTGCGTGTTTCTCC |
| oprL T-C_wt_1 | GGTGCTGCAGGGTGTTTCGCCGG |
| oprL T-C_mut_1 | GGTGCTGCAGGGCGTTTCGCCGG |
| fliC a A-T_wt_1 | CAAGATCGCCGCAGCGGTCAAC |
| fliC a A-T_mut_1 | CAAGATCGCCGCTGCGGTCAAC |
| alkB2 G-A_wt_2 | GCTGCTGGCGGCGGTGTGC |
| alkB2 G-A_mut_2 | TGCTGCTGGCAGCGGTGTGCT |
| alkB2 A-G_wt_1 | CCTCGCCCTGTTCCCACCGCTCTGG |
| alkB2 A-G_mut_1 | CTCGCCCTGTTCCCGCCGCTCTGG |
| citS A-G_wt_1 | TCGAGCAACTGGCAGAGAAATCCG |
| citS A-G_mut_1 | CGAGCAACTGGCGGAGAAATCCG |
| citS G-C_wt_1 | GCGGAAAACTTCCTGCACATGATGTT |
| citS G-C_mut_1 | GCGGAAAACTTCCTCCACATGATGTT |
| oprI T-C_wt_1 | AGCTCAGCAGACTGCTGACGAGG |
| oprI T-C_mut_1 | AGCTCAGCAGACCGCTGACGAG |
| oprI T-C_wt_2 | CAGAAAGCTCAGCAGACTGCTGACGAG |
| oprI T-C_mut_2 | GAAAGCTCAGCAGACCGCTGACGAG |
| ampC_1 G-A_wt_2 | ACGGCCGCCGGGTGACGCC |
| ampC_1 G-A_mut_2 | ACGGCCGCCAGGTGACGCCG |
| ampC_2 C-T_wt | GACAAGATGCGCCTCGACGACC |
| ampC_2 C-T_mut_1 | GACAAGATGCGTCTCGACGACCG |
| ampC_3 C-T_wt | AGCCGACCTACGCGCCGGGCAG |
| ampC_3 C-T_mut_1 | CAGCCGACCTATGCGCCGGGCAG |
| ampC_3 C-T_wt_1 | GCCGACCTACGCGCCGGGC |
| ampC_3 C-T_mut_2 | AGCCGACCTATGCGCCGGGCA |
| ampC_4 G-A_wt_2 | GTTCGAACGGCTCATGGAGCAGCA |
| ampC_4 G-A_mut_2 | GTTCGAACGACTCATGGAGCAGCAAG |
| ampC_5 G-C_wt_1 | TGGAGCAGCAAGTGTTCCCGGC |
| ampC_5 G-C_mut_1 | TGGAGCAGCAACTGTTCCCGGC |
| ampC_6 T-C_wt | GAACAAGACCGGTTCCACCAACGG |
| ampC_6 T-C_mut_1 | AACAAGACCGGCTCCACCAACGG |
| ampC_7 C-A_wt | GCGACCTGGGCCTGGTGATCCT |
| ampC_7 C-A_mut_1 | GCGACCTGGGACTGGTGATCCT |
| oprL T-C_wt_2 | GTGCTGCAGGGTGTTTCGCCG |
| oprL T-C_mut_2 | GCTGCAGGGCGTTTCGCCG |
| oprI T-C_wt_3 | GCTCAGCAGACTGCTGACGAGGCTAACG |
| oprI T-C_mut_3 | GCTCAGCAGACCGCTGACGAGGCTAAC |
| ampC_3 C-T_wt_2 | CGACCTACGCGCCGGGCAG |
| ampC_3 C-T_mut_3 | CGACCTATGCGCCGGGCAGC |
| ampC_4 G-A_wt_3 | CGTTCGAACGGCTCATGGAGCAG |
| ampC_4 G-A_mut_3 | CGTTCGAACGACTCATGGAGCAGC |
| ampC_7 C-A_wt_1 | CGACCTGGGCCTGGTGATCCT |
| ampC_7 C-A_mut_2 | GCGACCTGGGACTGGTGATCCTGG |

Die folgenden Oligonukleotid-Sonden bzw. deren nachstehend beschriebene Abwandlungen sind insbesondere zum Nachweis von DNA-Sequenzen geeignet, die nicht in allen *Pseudomonas aeruginosa*-Stämmen vorkommen.

| | |
|---|---|
| C-47-1 | GCGCGATCTTCTCCACTTCATCGG |
| C-45 | CGAGGAGTTTCGGACCCGCTTTGA |
| C-46 | AATAGGACCGGCAGAACGGGCATT |
| C-46_1 | CGAAGTCTGAGGTGTGGACCCGC |
| C-spezifisch-1 | GCATCATTGCGCGTCACATCTGGT |
| pKL-3 | TCTGAACTGCGGCTATCACCTGGA |
| pKL-11 | AGTCATGGGACTGAATACGGCGACT |
| PAGI-1-1 | TTCTCGGTGTCGAGGGATTCTCGG |
| PAGI-1-8 | TGGTAGCTCTCGACGTACTGGCTG |
| SG17M-1 | CCCGTTGCTCATAACCCGTTCCTG |
| SG17M-4 | AGGGCATTCTCAGGTGGACTCAGG |
| C-Inselspez.-4 | GCGCCTTCTCCTCTTTGCAGATGT |
| C-Inselspez.-5 | CAGTATGGTACGGACACGAAGCGC |
| TB-C47-3 | TCCAACAGGCAGGAGTACAGGGTG |
| TB-C47-4 | CGCTGCACATACAGGTCCGTTCTC |
| fliC a A-T_wt_2 | CAAGATCGCCGCAGCGGTCAACGAC |
| fliC a A-T_mut_2 | CAAGATCGCCGCTGCGGTCAACGAC |
| PA2221 | CAGTTGTCGCCAGGTCTGGAGAATCC |
| PA3835 | CACATCAATGTCAGCCCACGCCA |
| PA0728 | CTGGAGCCTGCGAAAGTGGCTC |
| PA2185 | ACGAGGGTGATGGCTGGGAATACG |
| PA0636 | GCCAATTGGGTCAGCAAGCAACG |
| PA0722 | CGTGTCGCGAACTCGCATGGC |
| Pyov-Rez-Type_I | CCTGAATCCGACCATTCGCGAGTC |
| Pyov-Rez-Type_IIa | TCGGACTGTACTCCTACGAAGCAGC |
| Pyov-Rez-Type_IIb | CCAATCCCTATCGCTGGAACCGTACC |
| Pyov-Rez-Type_III | GCTCGGGACTCGCATTTCGTCC |
| Pyov-Rez-Fpv_B | GCGTTATTGCTCGGTCTCTCCTCG |
| C-Inselspez.-1 | GACCGCAAGCAGAAACGGCATGC |
| C-Inselspez.-6 | CCATGGTCGGAACAGGCACGATATGC |
| C-47-1_2 | CCACTCGATCATGTTGAGCATCGGCTCC |
| SG17M-8 | GGTTAGTCCCTTCTGCCCGCATCG |

Die folgenden Oligonukleotid-Sonden bzw. deren nachstehend beschriebene Abwandlungen sind insbesondere zum Nachweis von Pathogenitätsinseln geeignet:

| | |
|---|---|
| 47D7-1_1 | GTGTCACGGCCCATGTCTAGCAGC |
| 47D7-2 | GTGAGCATGGAATCGGCAGTCGTT |
| fla-insel-1 | ACCTGTGTCGCTGGAGGGTATGTT |
| Fla-Insel-2_orfA | CGCTGGAGGGTATGTTCCGCAAGG |
| Fla-Insel-2_orfC | CGTACTCAGCTTCTCCACCCAGCG |
| Fla-Insel-2_orfI | CCTGGACCTCTCCAAGGTTCGCCT |
| Fla-Insel-2_orfJ | GCCATTCCGACGACCAAACAAGGC |
| 47D7-2_2 | AGGCCATGGGCTAGCCGGATGC |
| PAPI-2-XF1753 | CGAAGCGTAGGGTCTTCGTAGCC |
| PAPI-2-acetyltrans | TGCGAGGACCAGAAACCTTGATGG |
| PA0980 | CGGTATGAAGATGGGTGGTTGGGTCG |
| LES | TGCATAGGAGTCATGCCGACAGCA |
| PKLC102-unbekannt | GCCTGCCTACTTGTTCCCAACGC |
| PKLC102-adhesin | GGCTGTATTGCCCGCCATTCTCC |
| PKLC102-stoffw | CGACAGACAGAAAGGGTTCTTGCGC |
| pKL-1 | CACCATGCAAATGCTCGATGGACTGC |
| TB-C47-3_2 | GCAGGCGTCCAAGTTGGAGCTCTCC |
| PAPI-1_pili-chap | GGAACACAACGTGGGGCGTGAC |
| PAPI-1_lum_bin_pro | CCAGTTGGCACCACCATGCTTGC |

Die folgenden Nukleinsäure-Sondemnoleküle bzw. deren nachstehend beschriebene Abwandlungen sind insbesondere zum Nachweis von krankheitsassozüerten Genen wie exoS und exoU geeignet:

| | |
|---|---|
| exoS-1_1 | CAGCCCAGTCAGGACGCGCA |
| exoU | CGCCAGTTTGAGAACGGAGTCACC |
| exoU_1 | AGTGACGTGCGTTTCAGCAGTCCC |

Die folgenden Nukleinsäure-Sondenmoleküle bzw. deren nachstehend beschriebene Abwandlungen sind insbesondere zur Identifizierung des Flagellentyps geeignet:

| | |
|---|---|
| fliC b | GCCGACCAACTGAACTCCAACTCG |
| fliC a | GTCGCTGAACGGCACCTACTTCA |

Gegenstand der Erfindung sind neben den Oligonukleotid-Sonden mit den vorstehend aufgeführten Sequenzen auch Abwandlungen der vorstehend genannten Oligonukleotide, die trotz der Abweichungen in der Sequenz und/oder Länge eine spezifische Hybridisierung mit Ziel-Nukleinsäuren bzw. Target-Nukleinsäuren zeigen und dadurch einen spezifischen Nachweis von Stämmen von *Pseudomonas aeruginosa* und insbesondere die Geno- und Pathotypisierung *Pseudomonas aeruginosa* gewährleisten.

### Unter diese Abwandlungen fallen insbesondere

a) Nukleinsäuremoleküle, die (i) mit einer der obigen Oligonukleotid-Sequenzen in mindestens 80%, bevorzugt in mindestens 90% und besonders bevorzugt in mindestens 92%, 94%, 96% der Basen übereinstimmen, oder die (ii) sich von obigen Oligonukleotid-Sequenzen durch eine oder mehrere Deletionen und/oder Additionen unterscheiden und eine spezifische Hybridisierung mit Target-Nukleinsäuren von Stämmen der Spezies *Pseudomonas aeruginosa* ermöglichen.
b) Nukleinsäuremoleküle, die mit einer Sequenz, die zu einem der unter a) genannten Nukleinsäuremoleküle komplementär ist, unter stringenten Bedingungen (siehe unten) hybridisieren.
c) Nukleinsäuremoleküle, die eine Oligonukleotid-Sequenz nach a) oder b) umfassen und zusätzlich zu den genannten Sequenzen bzw. deren Abwandlungen nach a) oder b) mindestens ein weiteres Nukleotid aufweisen und eine spezifische Hybridisierung mit Target-Nukleinsäuren ermöglichen.

Der Grad der Sequenzidentität eines Nukleinsäure-Sondenmoleküls mit den oben explizit genannten Oligonukleotid-Sondenmolekülen kann mit üblichen Algorithmen bestimmt werden. Geeignet ist hierzu beispielsweise das Programm zur Bestimmung der Sequenzidentität, das unter http://www.ncbi.nlm.nih.govBLAST (auf dieser Seite z.B. der Link "Standard nucleotide-nucleotide BLAST [blastn]") zugänglich ist.

"Hybridisieren" kann im Rahmen dieser Erfindung gleichbedeutend sein mit "kömplementär". Im Rahmen dieser Erfindung sind auch solche Oligonukleotide umfasst, die mit dem (theoretischen) Gegenstrang eines erfindungsgemäßen Oligonukleotids einschließlich der erfindungsgemäßen Abwandlungen hybridisieren.

Der Begriff "stringente Bedingungen" steht allgemein für Bedingungen, unter denen eine Nukleinsäuresequenz präferentiell an ihre Zielsequenz hybridisieren wird, und zu einem deutlich geringeren Ausmaß oder gar nicht an andere Sequenzen. Stringente Bedingungen sind z.T. sequenzabhängig und werden unter verschiedenen Umständen unterschiedlich sein. Längere Sequenzen hybridisieren spezifisch bei höheren Temperaturen. Im Allgemeinen werden stringente Bedingungen so ausgewählt, dass die Temperatur etwa 5°C unter dem thermischen Schmelzpunkt (Tm) für die spezifische Sequenz bei einer definierten Ionenstärke und einem definierten pH liegt. Die Schmelztemperatur ist die Temperatur (unter definierter Ionenstärke, pH und Nukleinsäurekonzentration), bei der 50% der zu der Zielsequenz komplementären Moleküle zu der Zielsequenz im Gleichgewichtzustand hybridisieren.

Es versteht sich, dass der Fachmann die Konzentrationen der Bestandteile des Hybridisierungspuffers derart auswählen kann, dass die gewünschte Stringenz der Hybridisierungsreaktion erzielt wird. Unter Einsatz der stringenten Bedingungen kann der Fachmann feststellen, ob ein bestimmtes Nukleinsäuremoleküle einen spezifischen Nachweis von Target-Nukleinsäuren von *Pseudomonas aeruginosa* ermöglicht und somit im Rahmen der Erfindung zuverlässig eingesetzt werden kann.

Bei einer weiteren bevorzugten Ausführungsform der erfindungsgemäßen Microarray-Vorrichtung sind auf mindestens einem Array-Element sog. Kontrollsonden angeordnet. Derartige Kontrollsonden dienen z.B. zur Kontrolle der erfolgten Target-Markierung, der Amplifikationsreaktion, der Hybridisierungsreaktionen sowie - insbesondere bei Nachweisverfahren durch Präzipitation - der Färbung des Niederschlags.

Derartige Kontrollsonden weisen z.B. eine spezifische Komplementärität entweder zu einem extern zugegebenen Target oder zu einem in allen mit dem Array zu untersuchenden Samples in ausreichender Konzentration vorhandenen Target auf. Unter ausreichender Konzentration wird in diesem Zusammenhang eine Konzentration an Targetmolekülen verstanden, die zu einem signifikanten, d.h. deutlich detektierbaren Signal nach der Wechselwirkung mit den Sonden führt. Vorzugsweise sind die Array-Elemente, auf denen derartige Kontrollsonden angeordnet sind, über die gesamte Fläche des Arrays verteilt, besonders bevorzugt sind sie gleichmäßig verteilt. Eine Verteilung über die gesamte Fläche des Arrays bedeutet im Rahmen der vorliegenden Erfindung, dass sich ausgehend von dem Mittelpunkt der Array-Oberfläche in verschiedenen Abständen und verschiedenen Richtungen Array-Elemente mit derartigen Kontrollsonden befinden. Eine gleichmäßige Verteilung bedeutet vorzugsweise eine Anordnung der Array-Elemente mit derartigen Kontrollsonden als einheitliches Raster, beispielsweise als 10x10-Raster, bei dem jedes zehnte Array-Element ein derartiges Array-Element mit Kontrollsonden ist. Diese Ausführungsform ermöglicht z.B. die Normalisierung von experimentellen Schwankungen, die nach der Herstellung des Arrays u.a. in Abhängigkeit von dem Ort des Array-Elements auf der Array-Oberfläche auftreten können.

Bei einem weiteren Aspekt der vorliegenden Erfindung wird ein Verfahren zum spezifischen Nachweis von Bakterienstämmen der Spezies *Pseudomonas aeruginosa* in einer Probe bereitgestellt wird, welches die folgenden Schritte umfasst:
a) In Kontakt bringen der Probe mit einem wie vorstehend beschriebenen erfindungsgemäßen Nukleinsäure-Chip mit Oligonukleotid-Sonden für den spezifischen Nachweis von Bakterienstämmen der Spezies *Pseudomonas aeruginosa*; und
b) Detektion der Wechselwirkung zwischen den Oligonukleotid-Sonden und in der Probe enthaltenen Target-Nukleinsäuren.

Die zu untersuchenden Target-Nukleinsäuren bzw. die nachzuweisenden und zu typisierenden *Pseudomonas aeruginosa*-Stämme können in jeder Art von Probe, vorzugsweise in einer biologischen Probe vorliegen. Insbesondere wird das erfindungsgemäße Verfahren zur Untersuchung medizinischer Proben, z.B. von Stuhlproben, Blutkulturen, Sputum, Gewebeproben (auch Schnitten), Wundmaterial, Urin, Proben aus dem Respirationstrakt, Implantaten und Kathetheroberflächen eingesetzt werden.

Bei einer bevorzugten Ausführungsform des erfindungsgemäßen Nachweisverfahrens werden die in der Probe enthaltenen Target-Nukleinsäuren vor der

Detektion amplifiziert. Die Amplifikation erfolgt üblicherweise durch herkömmliche im Stand der Technik bekannte PCR-Methoden. Vorzugsweise wird die Amplifikation als Multiplex-PCR ausgeführt (siehe auch WO 97/45559). Bei einer Multiplex-PCR wird mehr als ein Primer pro Template-DNA in der Polymerase-Kettenreaktion verwendet. Das Ziel einer Multiplex-PCR ist die gleichzeitige Amplifikation mehrerer Bereiche der Target-DNA, um auf diese Weise Zeit zu sparen und Kosten zu minimieren.

Vorzugsweise werden bei der Amplifikation durch Multiplex-PCR Primer eingesetzt, die in etwa dieselbe Schmelztemperatur und in etwa dieselben Bindungskinetiken aufweisen. Auf diese Weise wird eine gleichmäßige Amplifikation sämtlicher Target-Nukleinsäuren gewährleistet und so ein exakter Nachweis auch in unterschiedlicher Ausgangskonzentration vorliegender Target-Nukleinsäuren sichergestellt. Unter einer in etwa gleichen Schmelztemperatur bzw. einem ähnlichen Schmelzpunkt wird im Rahmen der vorliegenden Erfindung eine Schmelztemperatur bzw. ein Schmelzpunkt verstanden, die bzw. der vorzugsweise höchstens 5°C und besonders bevorzugt höchstens 3°C von dem Vergleichsschmelzpunkt abweicht.

Bei einer besonders bevorzugten Ausführungsform wird die Amplifikation linear, d.h. nur auf einem DNA-Strang der Target- bzw. Template-Nukleinsäure durchgeführt. Dadurch wird vermieden, dass auch nur geringe Unterschiede bei den Schmelzpunkten und Bindungskinetiken der Primer wie bei der exponentiellen Amplifikation mittels herkömmlicher PCR zu großen Unterschieden in den nach Abschluss der Amplifikation vorliegenden Konzentrationsverhältnissen der Target-Nukleinsäuren führen, was einen Nachweis von nur in geringer Ausgangskonzentration vorliegenden Target-Nukleinsäuren neben in hohen Ausgangskonzentrationen vorliegenden Target-Nukleinsäuren verhindern würde.

Insbesondere haben die im Rahmen der erfindungsgemäßen Verfahren eingesetzten Primer die nachstehend angegebenen Mengen und Sequenzen (alle Primer sind in 5'-3'-Richtung notiert). Die im Folgenden aufgeführten Primer sind jedoch auch für beliebige andere dem Fachmann bekannte Verfahren zur Amplifikation von Nukleinsäuren geeignet.

47-1/23
ACGCGGATGTCCTGGATTTGG

47-1/39
CTGAAGAAGGGGCGCTACGCG

47-2/22
GCGTACCGGGCAAGGTGATAG

47-2/52
CTCGGTGAAACATCGGGAGGG

C45/18
TCATCCAGCAAGCCATTGCGC

C45/60a

GGAGTCGCTTTCCGCCATCG

C45/60b
TGGAGTCGCTTTCCGCCATCG

C46/15
AAGGGCGTTTCACGCTGACGC

C46/22
ATCCGGAAGGGCGTTTCACG

C46/88
TCCACACCTCAGACTTCGGCG

C47-1/43
TATTGACGACCTACCGCGCGC

C47-2/56a
GCAACTGATGTTCGCCCAGC

C47-2/56b
CGCAACTGATGTTCGCCCAGC

C47-2/59
ACACGCAACTGATGTTCGCCC

CIS-4/36
TGTCCCGGCTCAGTTCAACG

CIS-4/50
AACACCTTGGCGTTTGTCCC

CIS-4/51
GCAACACCTTGGCGTTTGTCC

CIS-5/4
TCAAGCTCGTTGTGGACCGC

CIS-5/48
GTTACGACGGCGTGCTGTCGG

CSP-1/39a
ACGCAACGTATTCGGCGACCC

CSP-1/39b
CGCAACGTATTCGGCGACCC

fliAT/28
AGCTGATGGTATCGCCGTCGC

fliAT/72
CTAGTGATCGCACCGGAGCC

oriC/20
AGCCTCGACACCGGTTCTCG

oriC/54
TCGTTCATCCCCAGGCTTCG

oriC/59
ACCATCTCGTTCATCCCCAGG

oprL/53
TTCTGAGCCCAGGACTGCTCG

oprL/65
TCGACGCGACGGTTCTGAGCC

fliCb/36
TGACGTTCTCGCCGGTAGCG

fliCb/65
CAGTAGCGGTACCGGTCTGCG

fliCb/66
CAGTAGCGGTACCGGTCTGC

alkAG/27
TTCCTCGCCGGCATAGTAGGC

alkGA/32
GGGGTCGAGACGTGTACATGG

alkGA/51
CGAGGACGAGGCATCTTCCGG

citAG/4
GCAGGTAGCAGGTTTCCAGG

citAG/46
AACTGTTCCTTCTGCGCGGCG

citGC/8
TGATCGGCTTGGTCTCGCAGG

citGC/11
GCTGATCGGCTTGGTCTCGC

citGC/75
GAGGCGTTCTGCTCGTGGTCG

oprI/12
TTTTTCCAGCATGCGCAGGG

oprI/17
GCTGGCTTTTTCCAGCATGCG

oprI/22
TTGCGGCTGGCTTTTTCCAGC

am7CA/1
TTGGGATAGTTGCGGTTGGC

am7CA/27
CGTAGGCGATCTTCACCCGC

am7CA/29
TGGCGTAGGCGATCTTCACCC

am3CT/21
GGCGAGATAGCCGAACAGGC

am3CT/22
GCGGCGAGATAGCCGAACAGG

am3CT/69
CACTTGCTGCTCCATGAGCC

am2CT/35
GAGGTCGAGCAGGCTGATGC

am2CT/42
TAGGTCGCGAGGTCGAGCAGG

am2CT/92
GTCCTTCTGCACCGAGTCGG

am1GA/49
CGCATCTTGTCCTGGGTCAGG

am1GA/58
TCGTCGAGGCGCATCTTGTCC

am45/1
ACGTCGAGGTGGGTCTGTTCG

am45/96
GTAGCCTTCGGCATCCAGCG

am6TC/60
TCGGCATTGGGATAGTTGCGG

GI11/15
CCTCCTGTCTCATGCCGATGC

GI11/59
GCATTCGCCACGGAAGGAAGG

GI11/71
GAAGGCATCATGGCATTCGCC

GI18/62
GTCATGGGGTTTCCCAGAGACC

fliCa/41
GATCGCGATGTCGACGGTGCC

fliCa/42
CGATCGCGATGTCGACGGTGC

fliCa/46
TGCCGATCGCGATGTCGACG

SG-1/40
GACGAATACCCAGCTGCGTGG

SG-1/43
GCAGACGAATACCCAGCTGCG

SG-4/1
CGCGACGTCGTGACGTCAGC

SG-4/67
ACTTTCGGCTCTTCGGGCTGG

TB46/21
AGGTAGAGACTCGGGGGAACC

TB46/45
TCGTTTTCGGTCATGGCCAGG

TB471/22
TTCCGCGACGAACATCCGTGG

TB471/25
CGCTTCCGCGACGAACATCCG

TB472/36
GGATCGCTTCCGATAGGGCAGC

TB472/84
AGAGGCATGGGTCTGTACCG

TB473/34
TCTGTCAATCCCCTTTGGGG

TB473/41
AGCCCCTTTCTGTCAATCCCC

TB474/36
GGCTTCCTACCGAAGGTCAGG

TB474/41
TGAGGGCTTCCTACCGAAGG

exoS/31
TTCAAGGTCATGGGCAATGCC

exoS/37
AGTCCCTTCAAGGTCATGGGC

exoU/22
GCCGACTGAGCTGTAGCTCGG

exoU/23
GGCCGACTGAGCTGTAGCTCG

exoU/42
ACCAGACTGGTCAATGGTGG

flins/2
CCCGTGTTTCCGTAGACCTTGC

pKL11/49a
AGCAGTTACCCACAGCATGG

pKL11/49b
CAGCAGTTACCCACAGCATGG

pKL3/47
CTACACTCCAACCGCTGGTCC

pKL3/50
GACCTACACTCCAACCGCTGG

pKL3/80
TTCCCTTGCTGCCGAGAAGC

pKL7/14
TAATAGGCGAGCCTGCCGTCC

47D7nwla
TCCACGCCGAGGGACGTGCC

47D7nw1b
GCTCCACGCCGAGGGACGTGCC

C46-nw1a
CGCGGTGCTGGTTGCGCTGC

C46-nw1b
CCAATGCCCAGGGCCAGCGGA

C46-nw1c
CGCTGGCAGTTCCGCTGGCC

ExoSnw1a
CAGGGTCGCCAGCTCGCTCGCC

ExoSnw1b
AGGGTCGCCAGCTCGCTCGC

ExoUnw1a
AGTGATCTGCCGCGGCCCTGCC

ExoUnw1b
GTGATCTGCCGCGGCCCTGC

OrfA-1
GTTCCACAGGCGCTGCGGCGC

OrfA-2
GTTCCACAGGCGCTGCGGCG

OrfA-3
CAAAGCCCCTGGTCGCGCGG

OrfC-1
GCAGCTTTTCCACCGCCGGCGG

Orfl-1
AAACTGCCCCGCCCCCCATCC

OrfI-2
GGAAAAACTGCCCCGCCCCCC

OrfJ-1
ACGCTCGCAGCGCCTCACGCG

OrfJ-2
GGCCTGGCTGCGAACGCTCGC

PA2221/37_Pa-P_064
TTCCTGGGCCAGAGTTGGACC

PA2221/66_Pa-P_065
AGCTTAAGGCCGTGGCACTCG

PA3835/46_Pa-P_066
CCGGAGAATTCGCGTCCACC

PA3835/72_Pa-P_067
TGCTGACGATGAAGCCCCAGC

47-22/3_Pa-P_072
AGGAGGCCGATGACAACACCC

47-22/67_Pa-P_073
TGCCGATTCCATGCTCACGCC

pI2X1753/29_Pa-P_O74
ACGACGTCACCGTCGAGACCG

pI2X1753/69_Pa-P_O75
ACCGCCTTTCTGGTGAGCTGG

PA0728/42_Pa-P_076
AGCCAAGACGGTTGTTCGCGG

PA0728/88_Pa-P_077
TCAATGACGCCGAGTTGGCGC

PA2185-1/42_Pa-P_078
CTCGGACAGGTTCACGCTGG

PA2185-1/70_Pa-P_079
GCCATTCGCTGCAACACCTCC

pI2actrf/39_Pa-P_085
GCGCGCGTTCGAGAAACAGG

pI2actrf/93_Pa-P_086
CGGAGGTTGAAAAGCTGGCCC

PA0636/29_Pa-P_087
ATGCCATCGTTGAAGGCACCGC

PA0636/30_Pa-P_088
TGCCATCGTTGAAGGCACCG

PA0722/4_Pa-P_089
TCTGGCGGAATCAGGTAGGCC

PA0722/55_Pa-P_090
CTTCCGGGGAGAAACCACCG

PA0980/45_Pa-P_093
ACCTCCAGCACCGACACACC

PA0980/53_Pa-P_094
ATCCGATCCACCTCCAGCACC

FpvaI/23_Pa-P_095
CGTTCAGGTCGTAGACCGCGC

FpvaI/84_Pa-P_096
GCGATACCAACTGTCCTGCGGC

FpvaIIa/34_Pa-P_097
TGCCGAAGGTGAATGGCTTGCC

FpvaIIa/65_Pa-P_098
CCTGATGGTCCGATCCCAGC

FpvaIIb/44_Pa-P_099
GCCGAGGGTCAAGAACCACTGG

FpvaIIb/67_Pa-P_100
TCTTGGCCCAGTCATAGCGGC

FpvaIII/16_Pa-P_101
TAACCCCAAGGCCCATTGGAGG

FpvaIII/31_Pa-P_102
GCCACCGCCTTCGAATAACCCC

FpvB/57_Pa-P_103
AATTGCTCGAGGGATGCGGC

FpvB/92_Pa-P_104
GGTCGAAACGGATGCGCAGG

LES/11_Pa-P_105
GCCCCGCGTCATTTTCACGTCG

LES/57_Pa-P_106
AATGCTCTGGGCAACGAGCC

pKLunbek/63_Pa-P_107
CTACCCAGCTTGGGCGTAGC

pKLunbek/141_Pa-P_108
AAGCGATAGCCGTGCTCCTGC

pKLadh/13_Pa-P_109
CCGGCTATATCCGCGGCTACC

pKLadh/59_Pa-P_110
ATTGGCGCTGCTGTTTACGCCC

pKLStiv/30_Pa-P_111
GGTGGCGTCGGGTTTTTCTGC

pKLStw/46_Pa-P_112
AGGTCGTAGCGGAAGGTGGTGG

pKL-1/22_Pa-P_113
ATCTGAACCGAGGGGATCCGC

pKL-1/61_Pa-P_114
CCCGGGAGTCATTGGTCTGG

T47-32/19_Pa-P_117
GCCTGTTGGACCCCTTTGACC

T47-32/26_Pa-P_118
TACTCCTGCCTGTTGGACCCC

pI1pil/15_Pa-P_121
CGCTCAAGCGCTATCCCACC

pI1pil/41_Pa-P_122
CGCCATCGGCCTGTACAACG

pI1lumin/87_Pa-P_123
CGGTAGAGAGCTGGGTTGGC

pI1lumin/209_Pa-P_124
AACCTGGAGCTAGGGCAGAGC

C-Insl/39_Pa-P_125
GGTGCTCGACCCAAGCATCG

C-Ins1/57_Pa-P_126
TCCTTGAGTTCCTTGGCGCGG

C-Ins6/42_Pa-P_131
CAACACGCGACTGGCGATCC

C-Ins6/61_Pa-P_132
TACATCATCCGCAACGGCGGC

C47-12/2_Pa-P_137
TATTGACGACCTACCGCGCGCC

C47-12/94_Pa-P_138
CACCAAGAACCCGCTGCTCG

SG-8/14_Pa-P_141
ATCGTGGCAGGATGTCCACCG

SG-8/86_Pa-P_142
TAGGCGGGCCTTTTGAAGGTGC

Gegenstand der Erfindung sind neben Primern mit den vorstehend aufgeführten Sequenzen auch Abwandlungen der obigen Primer, die trotz der Abweichungen in der Sequenz und/oder Länge eine spezifische Hybridisierung mit den Template-Nukleinsäuren der jeweiligen *Pseudomonas aeruginosa*-Stämme zeigen und sich dadurch für den Einsatz bei der Amplifikation der Target-Nukleinsäuren eignen.

### Hierunter fallen insbesondere

a) Primer, die (i) mit einer der oben explizit genannten Primersequenzen in mindestens 80%, bevorzugt in mindestens 90% und besonders bevorzugt in mindestens 92%, 94%, 96% der Basen übereinstimmen, oder die (ii) sich von obigen Primersequenzen durch eine oder mehrere Deletionen und/oder Additionen unterscheiden und eine spezifische Hybridisierung mit Template- bzw. Target-Nukleinsäuren von *Pseudomonas aeruginosa*-Stämmen ermöglichen.
b) Primermoleküle, die mit einer Sequenz, die zu einer der unter a) genannten Primermoleküle komplementär ist, unter stringenten Bedingungen (siehe oben) hybridisieren.
c) Nukleinsäuremoleküle, die die Sequenz eines Primermoleküls nach a) oder b) umfassen und zusätzlich zu den genannten Sequenzen bzw. deren Abwandlungen nach a) oder b) mindestens ein weiteres Nukleotid aufweisen und eine spezifische Hybridisierung mit Nukleinsäuresequenzen von Target-Organismen ermöglichen.

Bei einer besonders bevorzugten Ausführungsform werden für die Amplifikation pro Target-Nukleinsäure zwei geeignete Primer parallel eingesetzt.

Der Nachweis erfolgt bei dem erfindungsgemäßen Verfahren vorzugsweise dadurch, dass die gebundenen bzw. hybridisierten Target-Nukleinsäuren mit mindestens einer Markierung versehen sind, die in Schritt b) detektiert wird.

Wie bereits vorstehend erwähnt, ist die Markierung, die an die Targets oder Sonden gekoppelt ist, vorzugsweise eine detektierbare Einheit oder eine über eine Ankergruppe an die Targets oder Sonden gekoppelte detektierbare Einheit. Hinsichtlich der Möglichkeiten der Detektion bzw. der Markierung ist das erfindungsgemäße Verfahren äußerst flexibel. So ist das erfindungsgemäße Verfahren mit einer Vielzahl physikalischer, chemischer oder biochemischer Detektionsverfahren kompatibel. Voraussetzung ist lediglich, dass die zu detektierende Einheit bzw. Struktur direkt an eine Sonde oder ein Target, beispielsweise ein Oligonukleotid gekoppelt bzw. über eine mit dem Oligonukleotid koppelbare Ankergruppe verknüpft werden kann.

Die Detektion der Markierung kann auf Fluoreszenz, Magnetismus, Landung, Masse, Affinität, enzymatischer Aktivität, Reaktivität, einer Goldmarkierung und dergleichen beruhen. So kann die Markierung beispielsweise auf der Verwendung von Fluorophor-markierten Strukturen bzw. Bausteinen basieren. In Verbindung mit der Fluoreszenz-Detektion kann die Markierung ein beliebiger an Targets oder Sonden während oder nach deren Synthese koppelbarer Farbstoff sein. Beispiele hierfür sind Cy-Farbstoffe (Amersham Pharmacia Biotech, Uppsala, Schweden), Alexa-Farbstoffe, Texas-Rot, Fluorescein, Rhodamin (Molecular Probes, Eugene, Oregon, USA), Lanthanide wie Samarium, Ytterbium und Europium (EG&G, Wallac, Freiburg, Deutschland).

Neben Fluoreszenz-Markern können im Rahmen der vorliegenden Erfindung als Markierung bzw. als Detektiereinheit, die mit den Targets bzw. Sonden gekoppelt ist, auch Lumineszenz-Marker, Metall-Marker, Enzym-Marker, radioaktive Marker und/oder polymere Marker eingesetzt werden.

Ebenso kann eine Nukleinsäure als Markierung (Tag) genutzt werden, die durch Hybridisierung mit einem markierten Reporter detektiert werden kann (Sandwich-Hybridisierung). Einsatz zum Nachweis des Tags finden diverse molekularbiologische Nachweisreaktionen wie Primer-Extension, Ligation und RCA.

Bei einer alternativen Ausführungsform des erfindungsgemäßen Verfahrens ist die detektierbare Einheit über eine Ankergruppe mit den Targets oder Sonden gekoppelt. Bevorzugt verwendete Ankergruppen sind Biotin, Digoxigenin u. dgl. Die Ankergruppen werden in einer anschließenden Reaktion mit spezifisch bindenden Komponenten, beispielsweise Streptavidin-Konjugaten oder Antikörper-Konjugaten umgesetzt, die selbst detektierbar sind oder eine detektierbare Reaktion auslösen. Bei Einsatz von Ankergruppen kann die Umsetzung der Ankergruppen in detektierbare Einheiten vor, während oder nach Zugabe der Probe umfassend die Targets bzw. ggf. vor, während oder nach der Spaltung der selektiv spaltbaren Bindung in den Sonden erfolgen.

Die Markierung kann erfindungsgemäß auch durch Wechselwirkung eines markierten Moleküls mit den Sonden-Molekülen erfolgen. Beispielsweise kann die Markierung durch Hybridisierung eines wie vorstehend beschrieben markierten Oligonukleotids mit einer Oligonukleotid-Sonde bzw. einem Oligonukleotid-Target erfolgen.

Weitere im Rahmen der vorliegenden Erfindung geeignete Markierungsverfahren und Nachweissysteme sind beispielsweise in Lottspeich und Zorbas, Bioanalytik, Spektrum Akademischer Verlag, Heidelberg, Berlin, 1998, Kapitel 23.3 und 23.4 beschrieben.

Bei einer bevorzugten Ausführungsform des erfindungsgemäßen Verfahrens werden Nachweisverfahren eingesetzt, die im Ergebnis ein Addukt mit einem bestimmten Löslichkeitsprodukt, das eine Präzipitation zur Folge hat, liefern. Zur Markierung werden insbesondere Substrate eingesetzt, die in ein schwer lösliches, üblicherweise gefärbtes Produkt umgesetzt werden können. Beispielsweise können bei dieser Markierungsreaktion Enzyme verwendet werden, die den Umsatz eines Substrats in ein schwer lösliches Produkt katalysieren. Reaktionen, die geeignet sind, um zu einem Niederschlag an Array-Elementen zu führen, sowie Möglichkeiten für die Detektion des Niederschlags sind beispielsweise in der internationalen Patentanmeldung WO 00/72018 und in der internationalen Patentanmeldung WO 02/02810 beschrieben, auf deren Inhalt hiermit ausdrücklich Bezug genommen wird.

Bei einer besonders bevorzugten Ausführungsform des erfindungsgemäßen Verfahrens sind die gebundenen Targets mit einer Markierung versehen, die die Reaktion eines löslichen Substrats zu einem schwer löslichen Niederschlag auf dem Array-Element katalysiert, an dem eine Sonden/Target-Wechselwirkung stattgefunden hat bzw. die als Kristallisationskeim für die Umwandlung eines löslichen Substrats zu einem schwer löslichen Niederschlag auf dem Array-Element wirkt, an dem eine Sonden/Target-Wechselwirkung stattgefunden hat.

Der Einsatz des erfindungsgemäßen Verfahrens erlaubt auf diese Weise die simultane qualitative und quantitative Analyse einer Vielzahl von Sonden/Target-Wechselwirkungen, wobei einzelne Array-Elemente mit einer Größe von ≤ 1000 µm, vorzugsweise von ≤ 100 µm und besonders bevorzugt von ≤ 50 µm realisiert werden können.

In der Immunzytochemie und bei immunologischen Mikrotiterplatten-basierten Tests ist der Einsatz von enzymatischen Markierungen bekannt (siehe E. Lidell und I. Weeks, Antibody Technology, BIOS Scientific Publishers Limited, 1995). So katalysieren beispielsweise Enzyme den Umsatz eines Substrats in ein schwerlösliches, in aller Regel gefärbtes Produkt.

Eine weitere Möglichkeit des Nachweises molekularer Wechselwirkungen auf Arrays besteht im Einsatz von Metallmarkierungen. Hierbei werden beispielsweise kolloidales Gold oder definierte Goldcluster mit den Targets gekoppelt, ggf. über bestimmte Vermittlermoleküle wie Streptavidin. Die durch die Goldmarkierung entstehende Färbung wird vorzugsweise durch nachfolgende Reaktion mit unedleren Metallen wie z.B. Silber verstärkt, wobei die mit den Targets gekoppelte Goldmarkierung als Kristallisationskeim bzw. Katalysator beispielsweise für die Reduktion von Silberionen zu einem Silberniederschlag wirkt. Die mit Goldmarkierungen gekoppelten Targets werden im Folgenden auch als Goldkonjugate bezeichnet.

Bei dieser Ausführungsform des erfmdungsgemäßen Verfahrens kann auch eine relative Quantifizierung der Sonden-/Target-Wechselwirkung erfolgen. Die relative Quantifizierung der Konzentration der gebundenen Targets auf einem Sonden-Array durch Nachweis eines Präzipitats bzw. eines Niederschlags erfolgt über die Konzentration der mit den Targets gekoppelten Markierungen, die die Reaktion eines löslichen Substrats zu einem schwerlöslichen Niederschlag auf dem Array-Element, an dem eine Sonden/Target-Wechselwirkung stattgefunden hat, katalysieren bzw. als Kristallisationskeim für derartige Reaktionen wirken. Beispielsweise beträgt im Fall von mit Nanogold markierten HPLC-gereinigten Oligonukleotidsonden das Verhältnis von gebundenem Target zu Goldpartikel 1:1. In anderen Ausführungsformen der vorliegenden Erfindung kann es ein Vielfaches oder auch einen Bruchteil davon betragen.

Die Detektion bei dieser Ausführungsform des erfindungsgemäßen Nachweisverfahrens erfolgt somit durch Messung der Transmissionsänderung, Reflexion oder Streuung, die durch den Niederschlag hervorgerufen wird, der auf den Array-Elementen, an denen eine Sonden/Target-Wechselwirkung stattgefunden hat, durch die katalytische Wirkung der mit den gebundenen Targets gekoppelten Markierung erzeugt wird.

Die Lichtabsorption wird im Fall der Kopplung von kolloidalem Gold oder definierten Goldcluster mit den Targets bereits durch die Gegenwart dieser metallischen Markierungen hervorgerufen. Zur Verstärkung der Lichtabsorption wird allerdings vorzugsweise katalytisch durch derartige Wechselwirkungshybride, d.h. die mit einer Markierung wie beispielsweise kolloidalem Gold oder definierten Goldclustern versehene Targets, ein nicht transparentes Präzipitat abgeschieden. Als besonders vorteilhaft hat sich im Fall von Goldkonjugaten die Verwendung von Silber als Präzipitat herausgestellt.

Bei einer weiteren bevorzugten Ausführungsform des erfindungsgemäßen Verfahrens wird somit in Schritt c) der zeitliche Verlauf der Niederschlagsbildung an den Array-Elementen in Form von Signalintensitäten detektiert. Auf diese Weise kann eine genaue Bestimmung der relativen quantitativen Menge an gebundenen Targets gewährleistet werden. Eine derartige Vorgehensweise ist ausführlich in der internationalen Patentanmeldung WO 02/02810 beschrieben, auf deren Inhalt hiermit ausdrücklich Bezug genommen wird.

Bei einem weiteren Aspekt der vorliegenden Erfindung werden Kits zur Durchführung der vorstehend beschriebenen Verfahren zur Verfügung gestellt. Die in diesen Kits enthaltenen Hybridisierungsanordnungen bzw. Chip-Vorrichtungen sind z.B. in den internationalen Patentanmeldungen WO 03/059516, WO 01/02094 und WO 03/031063 beschrieben. Auf die in diesen Dokumenten enthaltene Offenbarung bezüglich der Mikroarray-Anordnungen wird hiermit ausdrücklich Bezug genommen.

Außer den dort beschriebenen Hybridisierungsanordnungen, vorzugsweise einem ArrayTube®, umfassen die Kits als wichtigen Bestandteil die erfindungsgemäße Microarray-Vorrichtung bzw. den erfindungsgemäßen Biochip und insbesondere die vorstehend beschriebenen für die nachzuweisenden *Pseudomonas aeruginosa-*Stämme spezifischen Nukleinsäure-Sondenmoleküle, die auf dem Träger angeordnet sind. Gegebenenfalls weiter enthalten sind entsprechende Primer, Hybridisierungspuffer und Konzentrate von entsprechenden Waschlösungen.

Das folgende Beispiel soll die Erfindung erläutern, ohne sie einzuschränken:

### Beispiel

Im Rahmen der vorliegenden Erfindung wurde ein Detektionsverfahren entwickelt, mit dem die Geno- und Pathotypisierung von *Pseudomonas aeruginosa* innerhalb von sechs Stunden, ausgehend von den Bakterien auf einer Agar-Platte, durchgeführt werden kann. Hierzu werden nur grundlegende Labormethoden, wie z.B. PCR und Geräte, die zur Grundausstattung eines molekularbiologischen Labors gehören, benötigt. Ein kritischer Schritt hierbei ist die PCR, bei der über 40 verschiedene Sequenzen im gleichen Reaktionsansatz parallel amplifiziert werden. Um dies zu erreichen, wurden bei einer Ausgestaltung des erfindungsgemäßen Verfahrens 80 DNA-Primer so optimiert, dass sie in etwa gleiche Schmelzpunkte und Bindungskinetiken aufweisen. Weiterhin wurden die Template-Nukleinsäuren nur linear, d.h. auf einem DNA-Strang amplifiziert, um somit auch die Auswirkungen geringer kinetischer Unterschiede zu minimieren. Diese Optimierung ermöglicht die Verwendung einer Multiplex-PCR zur Target-Amplifikation.

Mit dem im Rahmen der vorliegenden Erfindung bereitgestellten DNA-Chip ist es daher möglich, *Pseudomonas aeruginosa* schnell und einfach in einem diagnostischen Routeinelabor innerhalb eines Tages zu untersuchen und so z.B. bei Verdacht auf eine nosokomiale Ausbreitung dieses Erregers schnell reagieren zu können.

Im Folgenden ist ein Versuchsprotokoll angegeben:

### a) Vorbereitung der Bakterien

2 Impfösen der Bakterienkultur (20 µl Bakterien von einer LB-Agarplatte) in 1,5 ml
H₂O aufnehmen
Zentrifugation (3.000 x g, 6 min)
Überstand abnehmen
Pellet 4 x waschen:
Resuspension in 5 mM EDTA
Zentrifugation (14.000 x g, 5 min)
Überstand abnehmen
Pellet in 50 µl dest. H₂O resuspendieren

### b) Polymerase- Kettenreaktion (PCR )

Die zu untersuchenden DNA-Sequenzen der Bakterien werden in einer Polymerase-Kettenreaktion (PCR) vervielfältigt.

| | | | |
|---|---|---|---|
| Polymerase: | Terminator- Polymerase (New England Biolabs) | | |
| dNTPs: | je 2 mM dATP, dGTP, dCTP, | | |
| | 1,5 mM dTTP | | |
| | 0,5 mM Biotin-dUTP (Roche) | | |
| Primer: | Mischung aus je zwei 21 bp Oligonukleotiden für jede zu detektierende Sequenz. Die Primer haben gleiche Schmelzpunkte und Bindungskinetik und binden auf dem gleichen Strang ca. 100 Basen vor der untersuchten DNA-Sequenz. Die verwendete Mischung hat eine Gesamtkonzentration an Oligonukleotiden von 5 µmol/l. Die Sequenzen der verwendeten Primer sind in Abbildung 17 dargestellt. | | |
| | | | |
| | | | |
| | | | |
| | | | |
| | | | |
| Reaktionsansatz: | 10x Reaktionspuffer | | 2,5 µl |
| | dNTP- Mischung | | 2,5 µl |
| | Primer | | 2,5 µl |
| | DMSO | | 1,2 µl |
| | Bakterien-Suspension | | 8,0 µl |
| | Terminator-Polymerase | | 0,5 µl |
| | Wasser | | 7,8 µl |
| | | | = 25 µl |
| Reaktionsablauf: | | | |
| | Start: | 96°C | 300 s |
| | 40 Zyklen | 60°C | 20 s |
| | | 72°C | 40 s |
| | | 96°C | 60 s |
| | Ende | 10°C | |

### c) Hybridisierungsassay

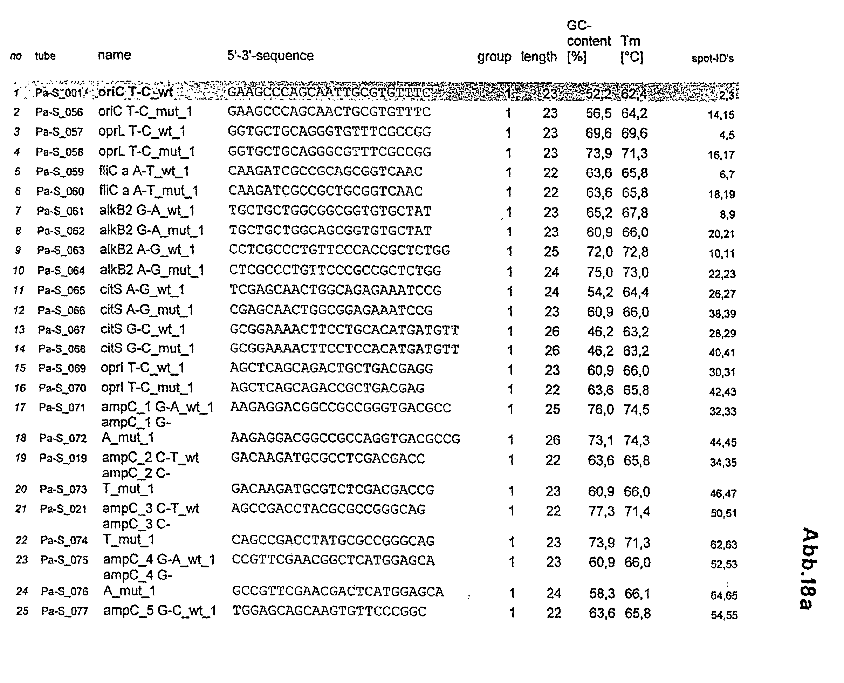

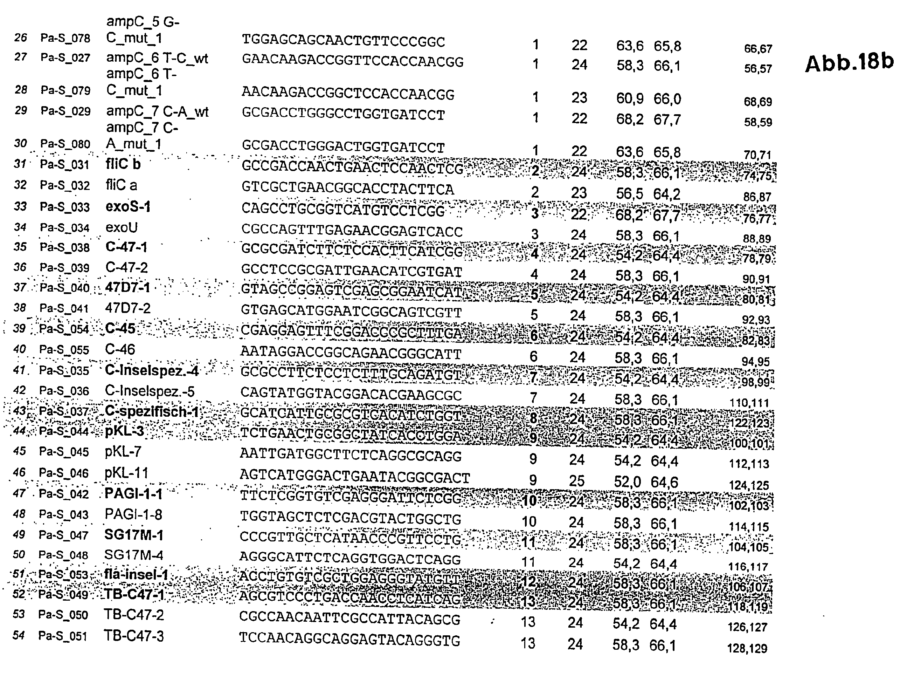

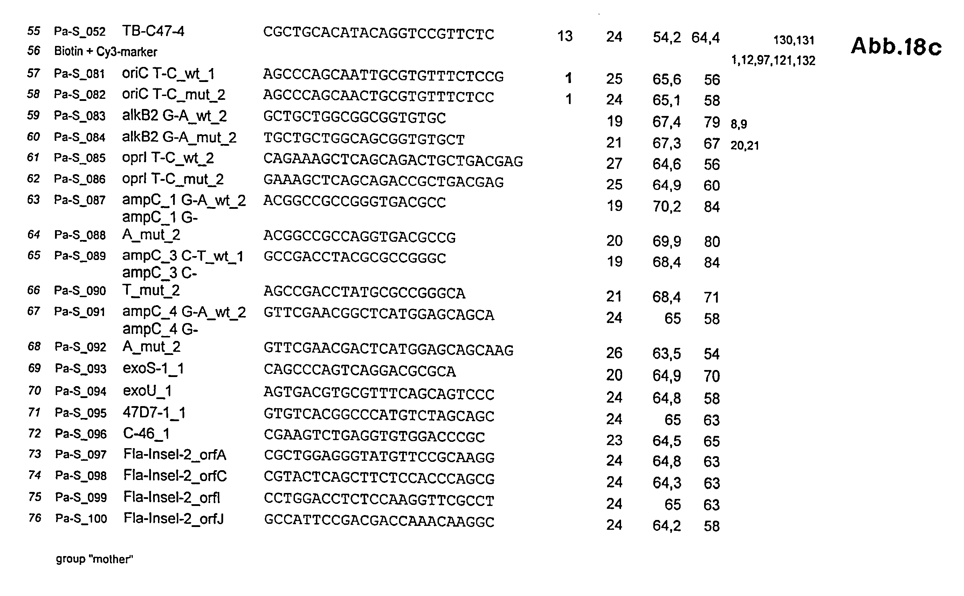

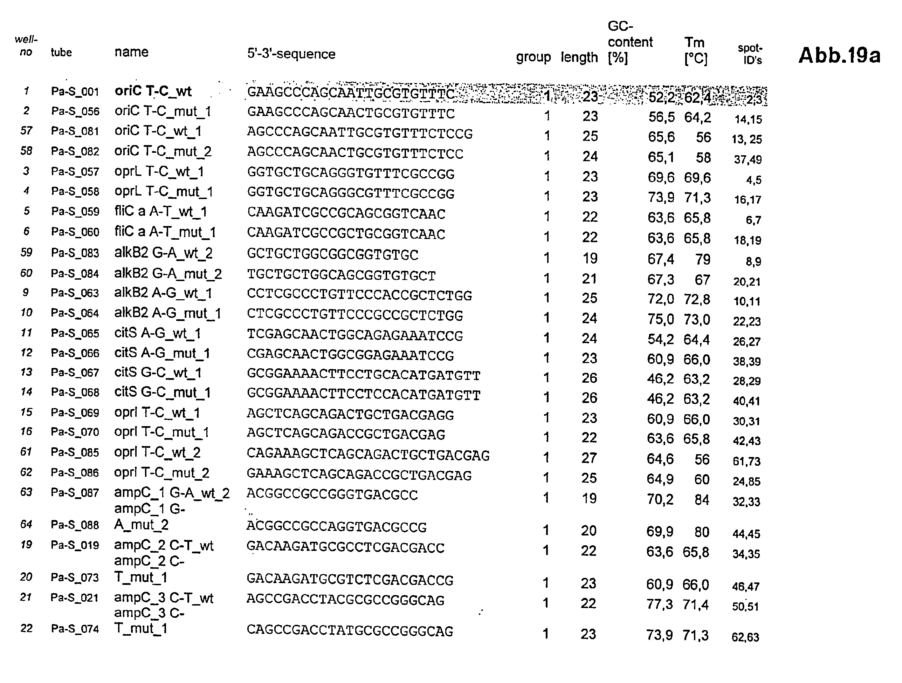

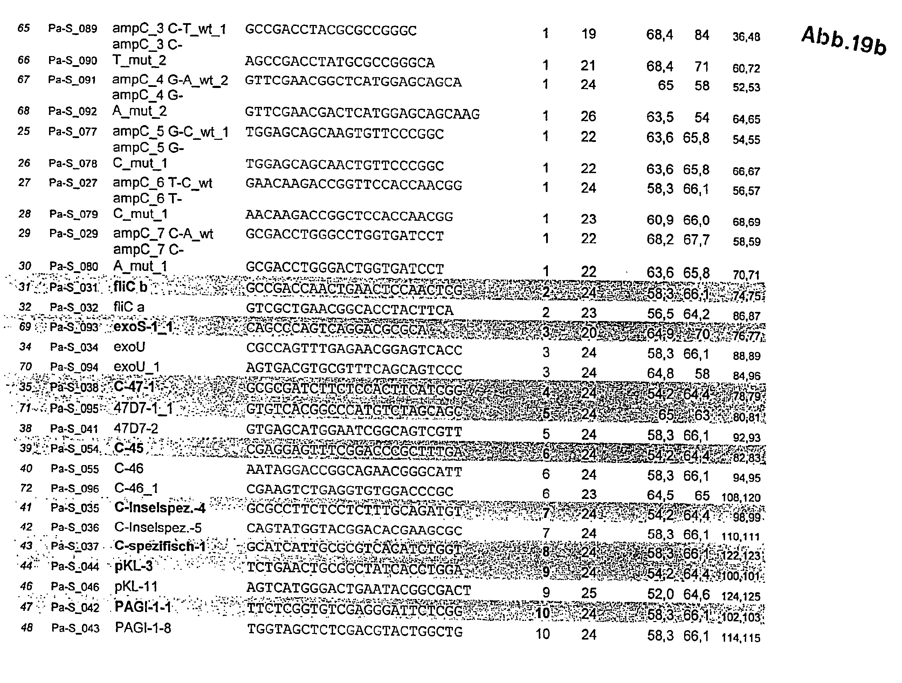

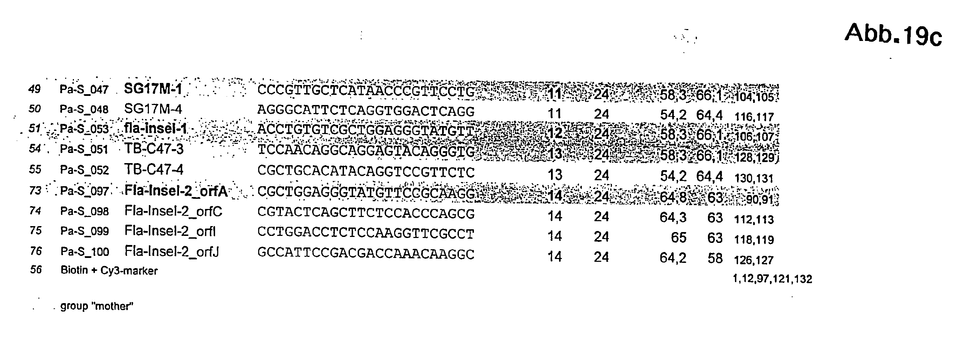

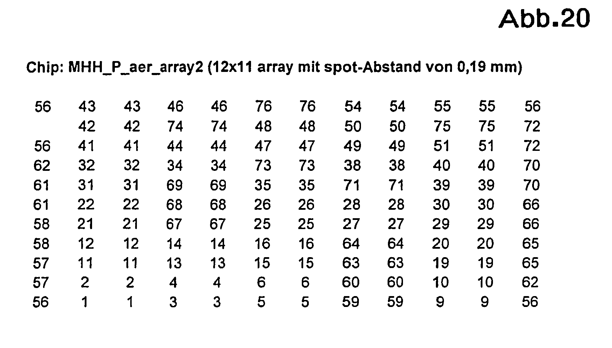

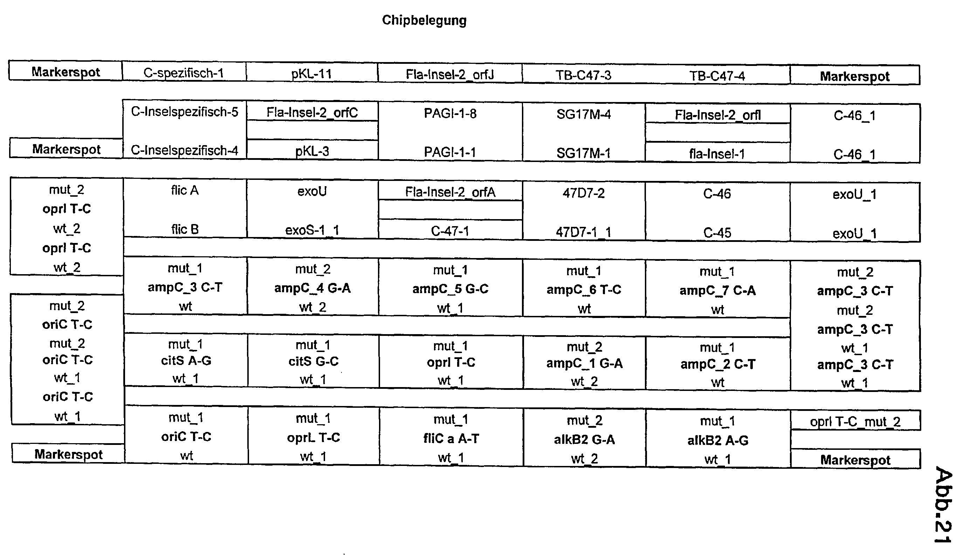

Die eingesetzten Oligonukleotid-Sonden sowie die Anordnung der Oligonukleotid-Sonden auf dem erfindungsgemäßen Nukleinsäurechip sind in den Abbildungen 18 bis 21 gezeigt.

Die Chips werden 2 x 5 Minuten mit 500 µl des Hybridisierungspuffer (6 x SSPE / 0,1%SDS/2% w/v Blocking Reagenz (Roche)) in einem Thermomixer gewaschen (30°C, 550 rpm).

20 µl des PCR-Produkts werden zusammen mit 80 µl Hybridisierungspuffer in einem Heizblock denaturiert (96°C, 5 min) und auf Eis abgekühlt.

Diese Sondenlösung wird auf den ArrayTube®-Chip (Clondiag) gegeben und eine Stunde bei 60 °C und 550 rpm inkubiert (Thermomixer).

Die Sondenlösung wird verworfen und der DNA-Chip gewaschen:
500 µl 2xSSC/0,01%Triton X-100 für 10 min bei 30°C und 550 rpm
500 µl 2xSSC für 10 min bei 20°C und 550rpm
500 µl 0,2xSSC für 10 min bei 20°C und 550rpm

Der ArrayTube®-Chip wird mit 100 µL Meerettich-Streptavidin-Konjugat (1:100 Verdünnung) für 15 min (30°C, 550 rpm) inkubiert und anschließend gewaschen:
500 µl 2 x SSC/0,01%Triton X-100 für 10 min bei 30°C und 550 rpm
500 µl 2 x SSC für 10 min bei 20°C und 550 rpm
500 µl 0,2 x SSC für 10 min bei 20°C und 550 rpm

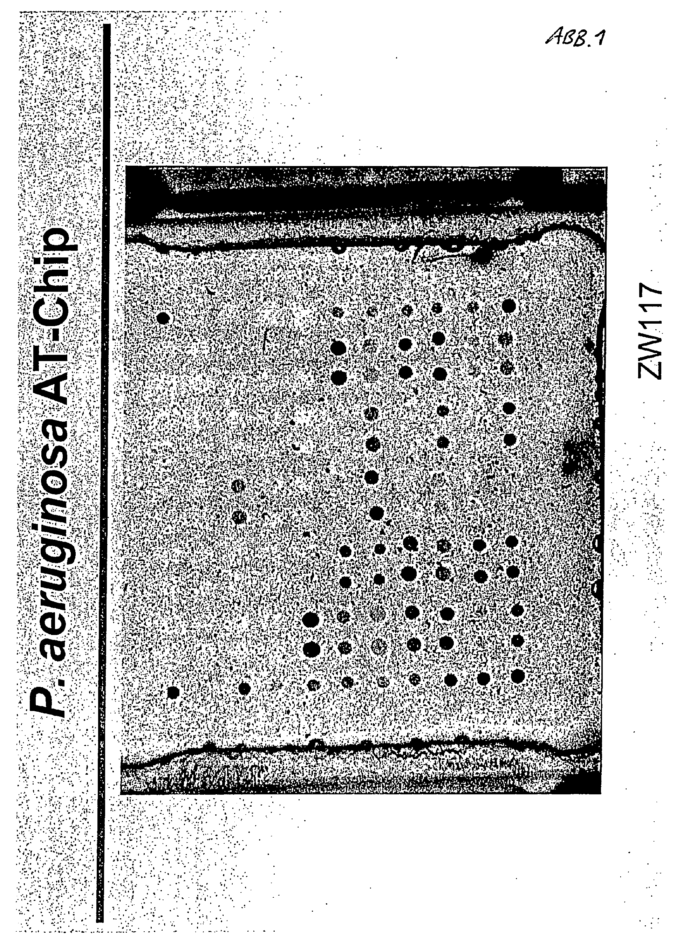

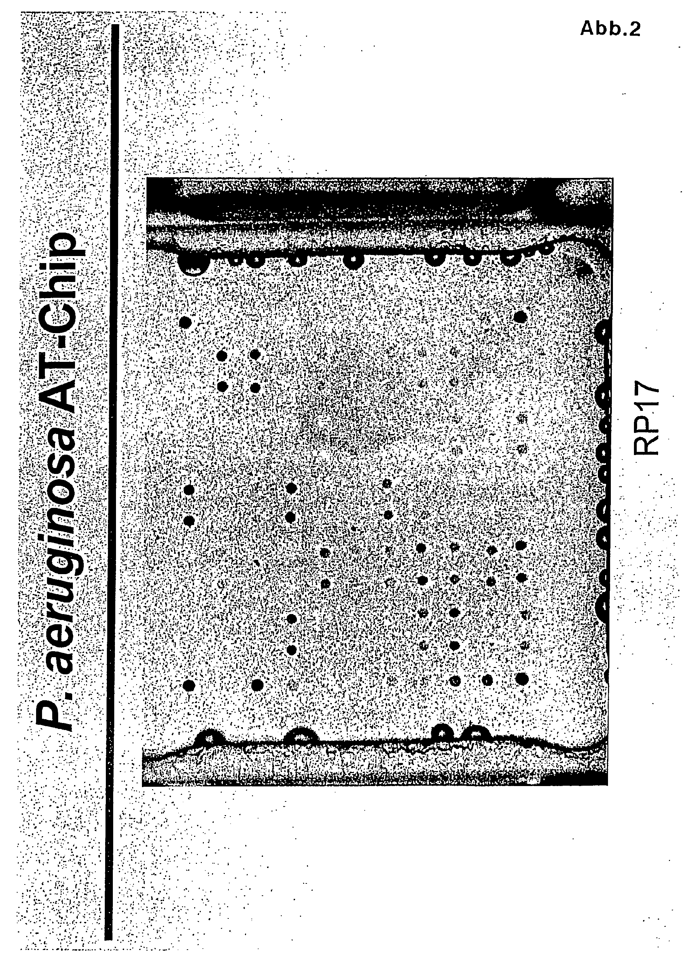

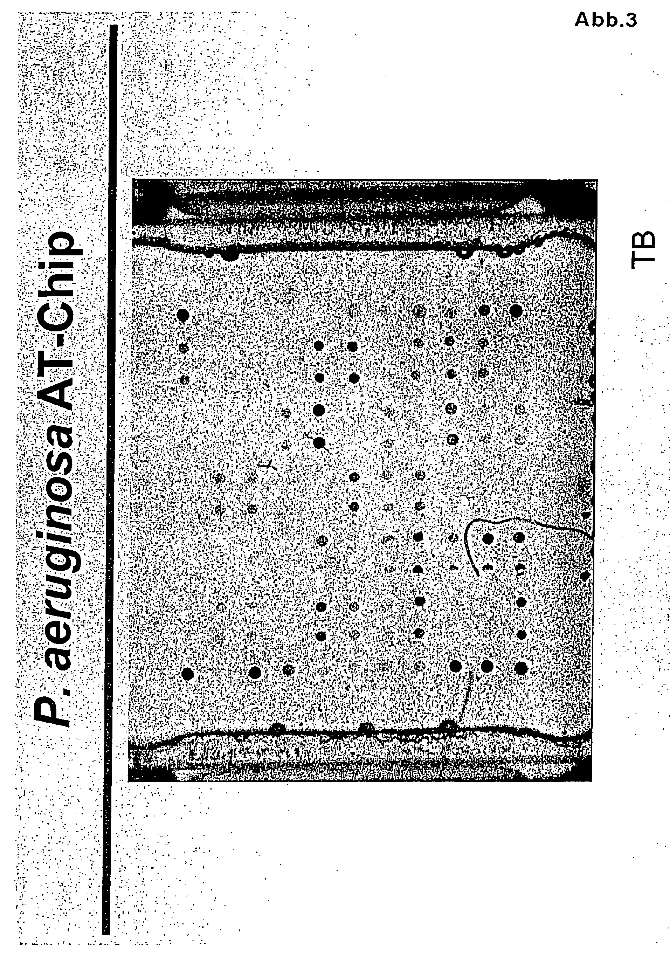

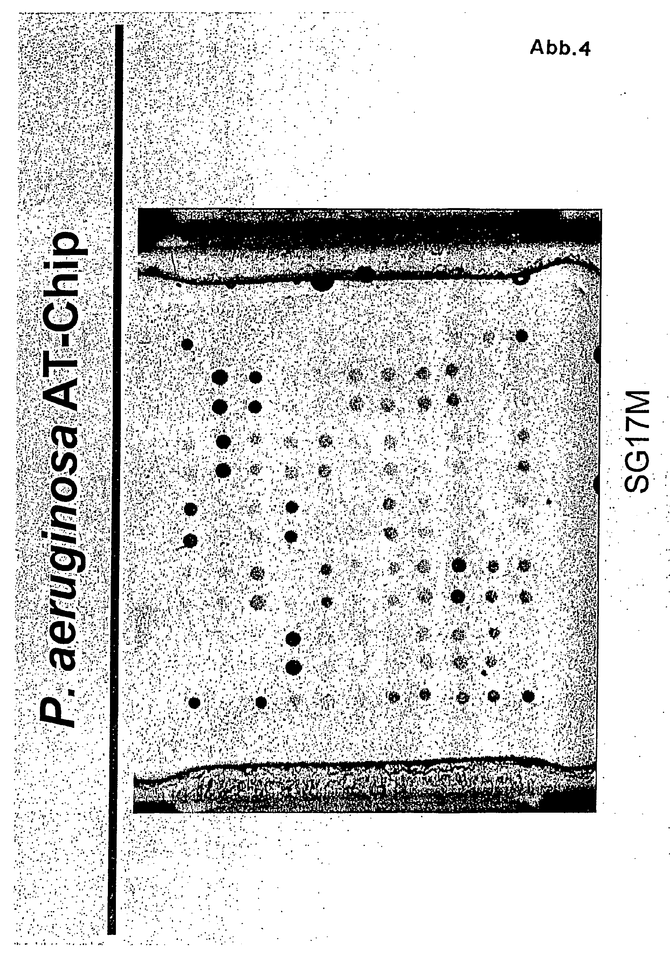

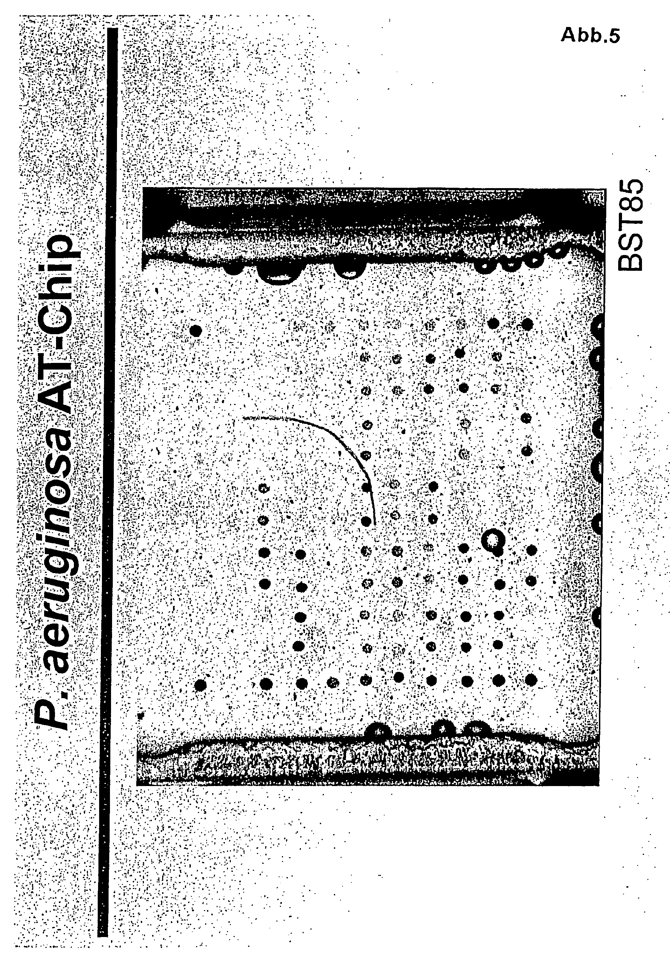

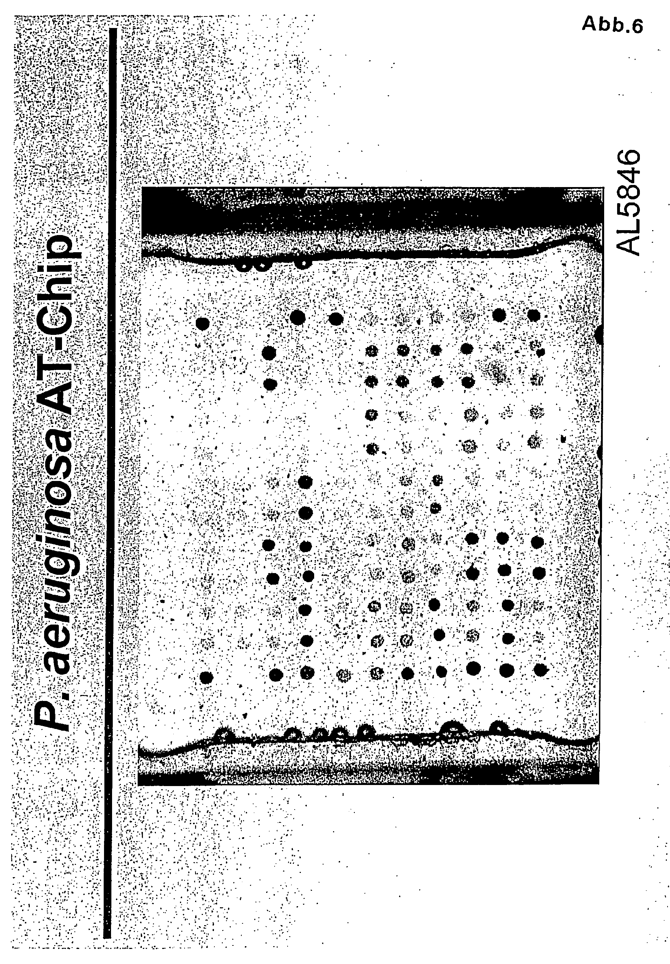

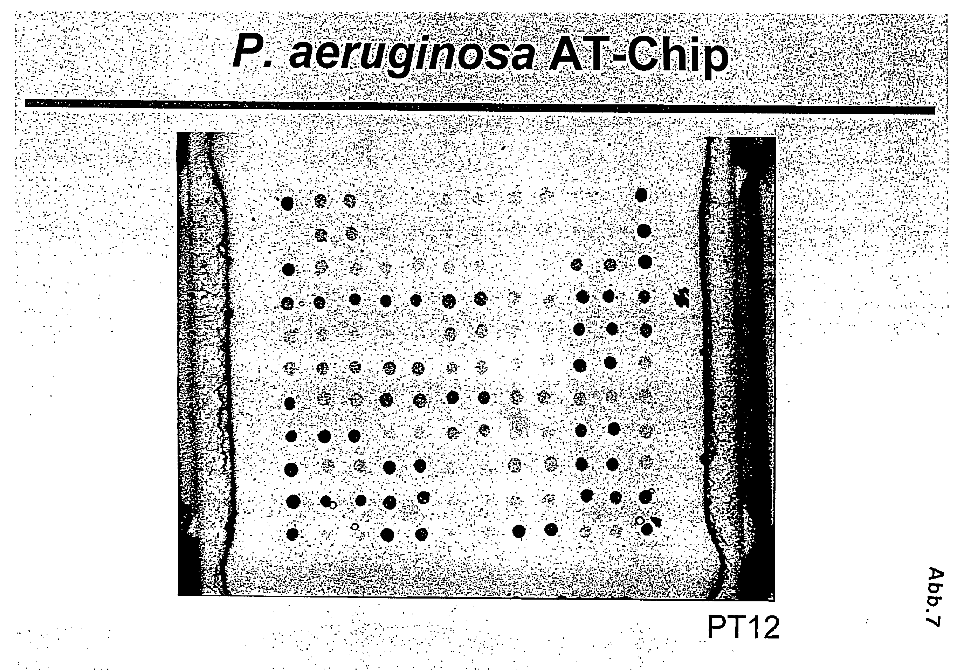

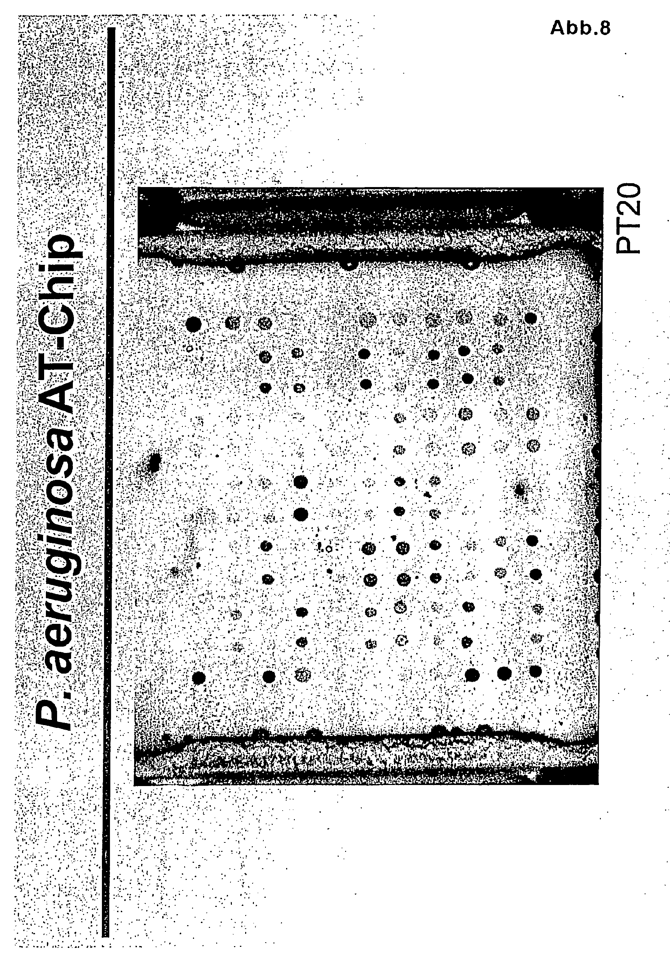

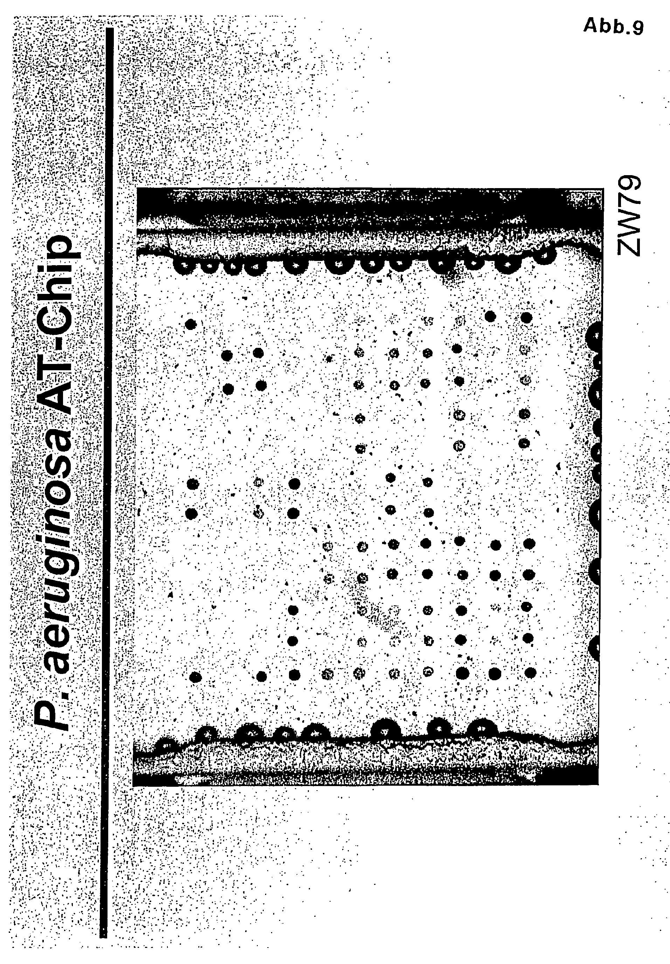

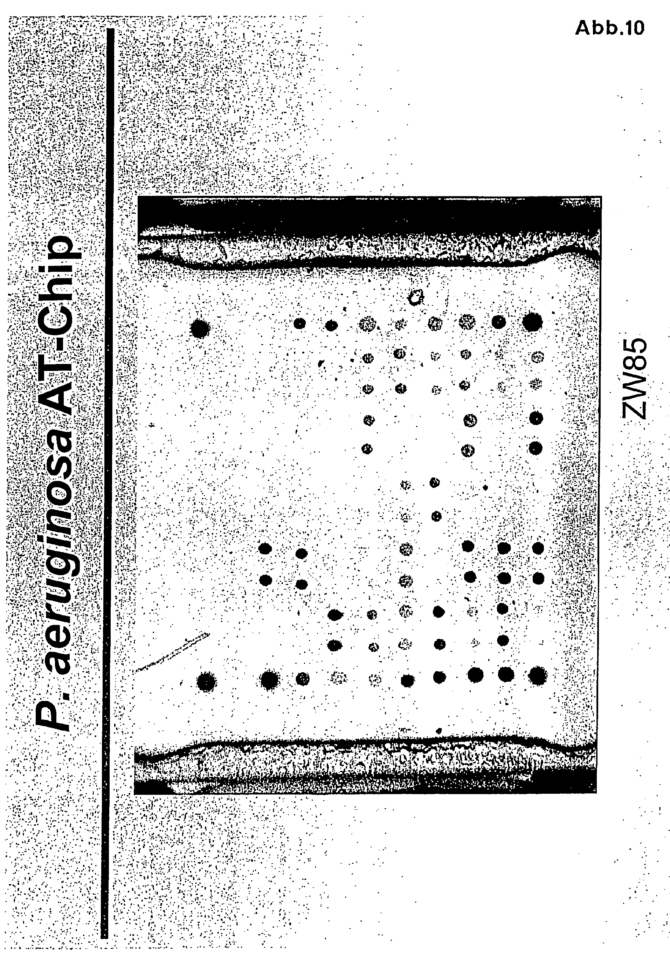

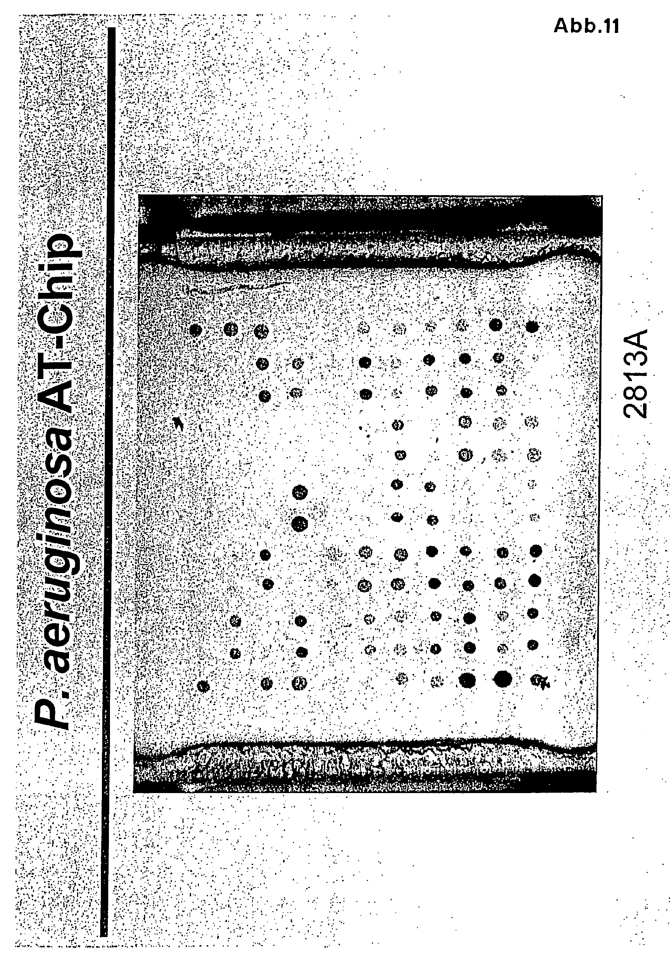

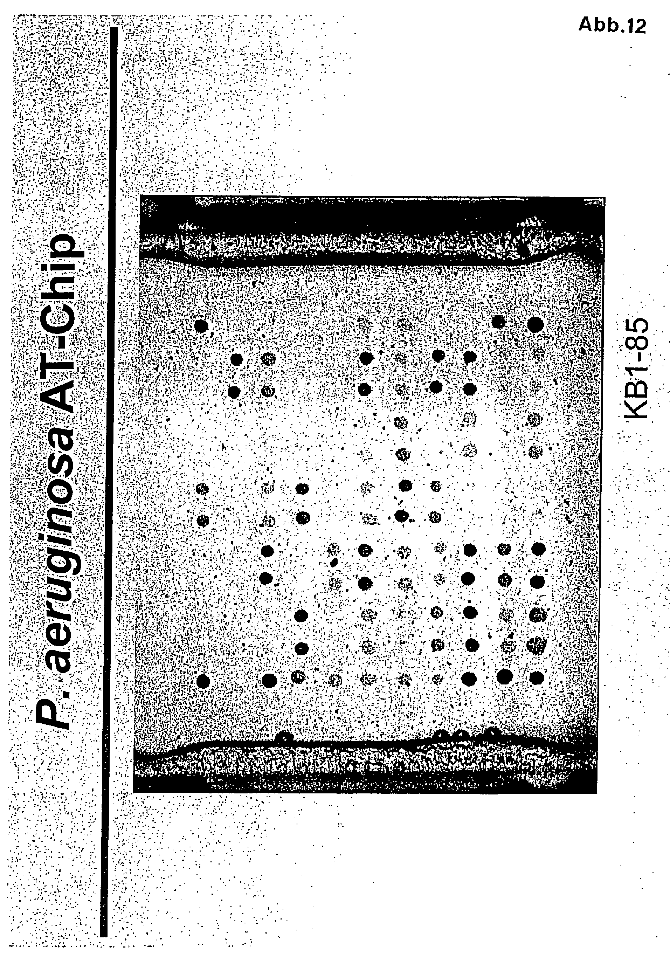

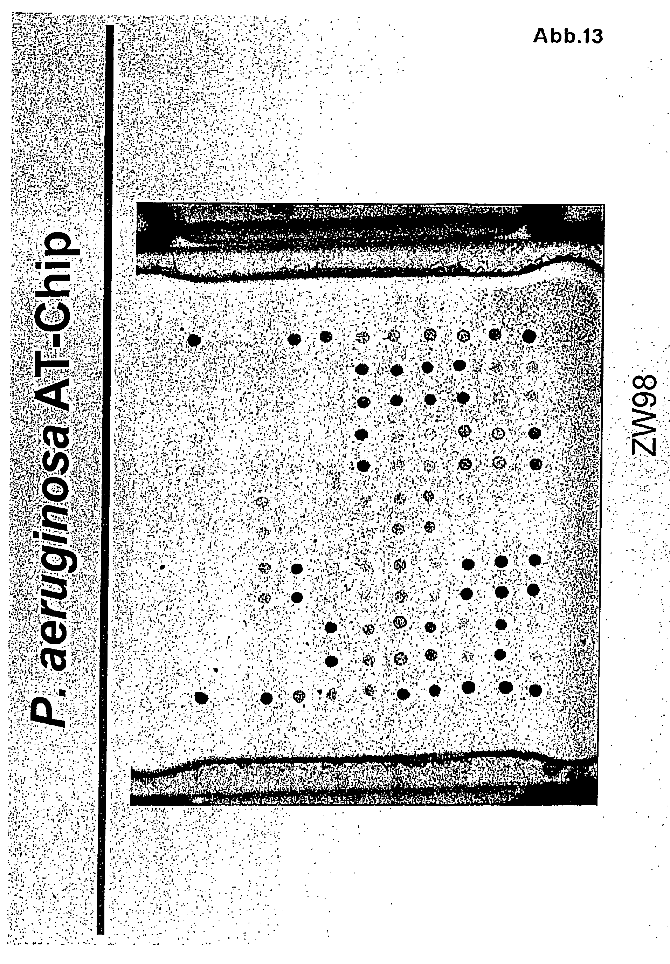

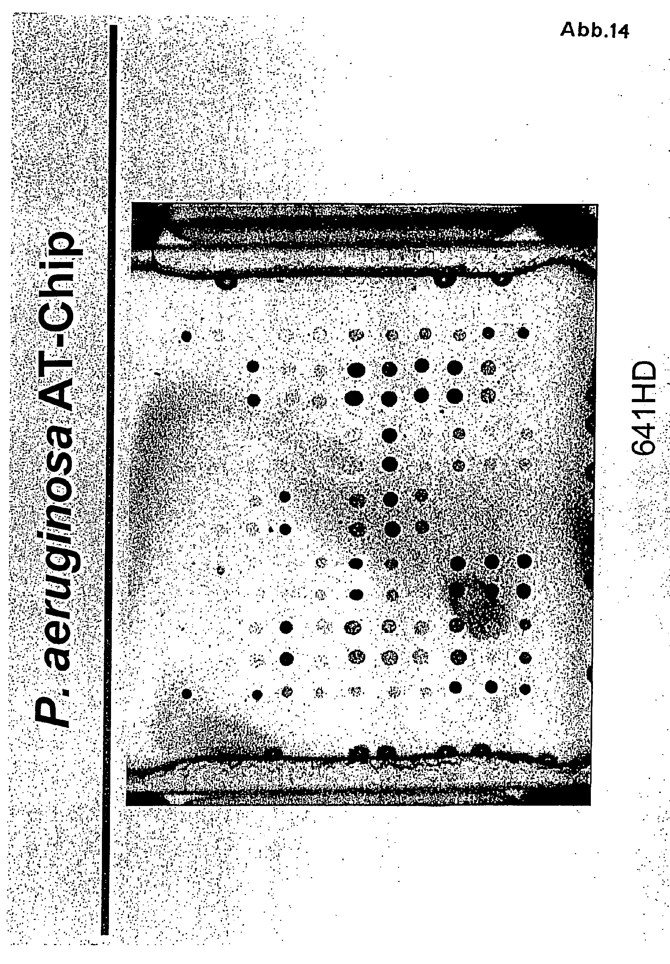

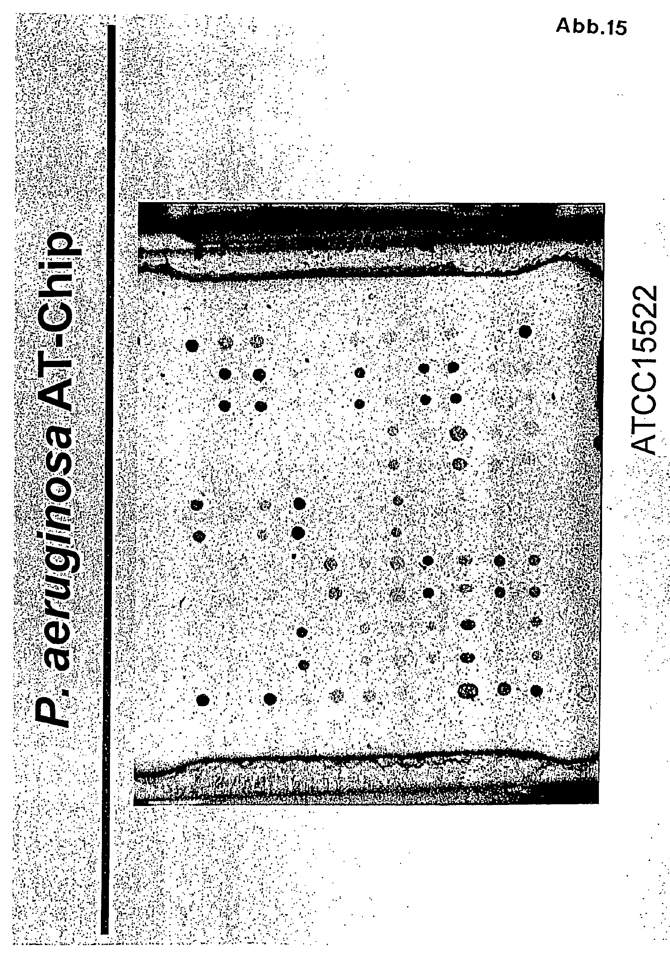

Zur Detektion werden 100 µl eines Tetramethylbenzidin-Derivats (Medac, Wedel, Deutschland) auf den Chip gegeben und das Ergebnis mit Hilfe eines AT-Readers (Clondiag) und des Programms IconoClust (Clondiag) ausgewertet. Die Ergebnisse für diverse Stämme von Pseudomonas aeruginosa sind in den Abbildungen 1 bis 15 dargestellt.

### d) Lösungen

| | |
|---|---|
| 10x SSPE- Puffer | 1,5 M NaCl |
| | 0,1 M Natriumphosphat |
| | 0,01M EDTA |
| | pH 7,4 |
| | |
| 20x SSC- Puffer | 3,0 M NaCl |
| | 0,3 M Natriumcitrat |
| | pH 7,0 |

### Abbildungen

Die Abbildungen 1 bis 15 zeigen hybridisierte DNA-Chips, die mit unterschiedlichen *P. aeruginosa*-Stämmen hybridisiert wurden. Die Aufarbeitung der Stämme erfolgte nach dem oben beschriebenen Protokoll.

Abbildung 16 zeigt ein Laborreaktionsgefäß typischer Form und Größe.

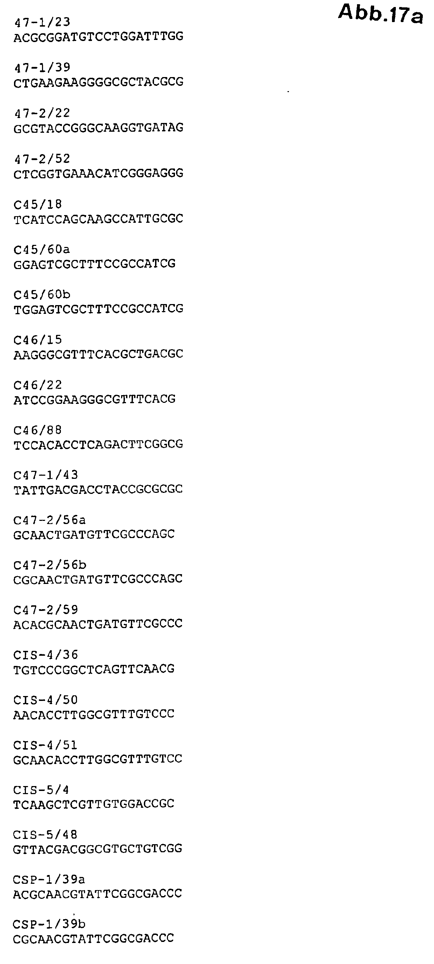

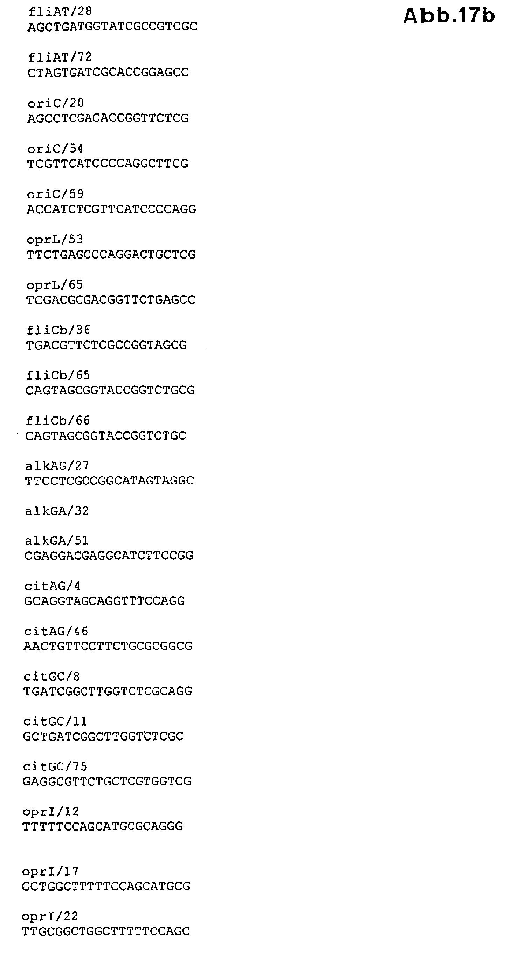

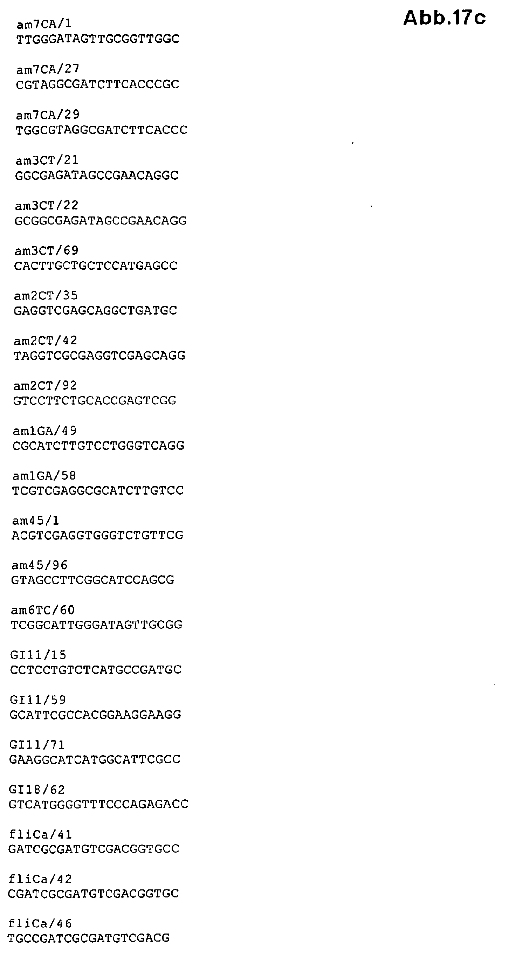

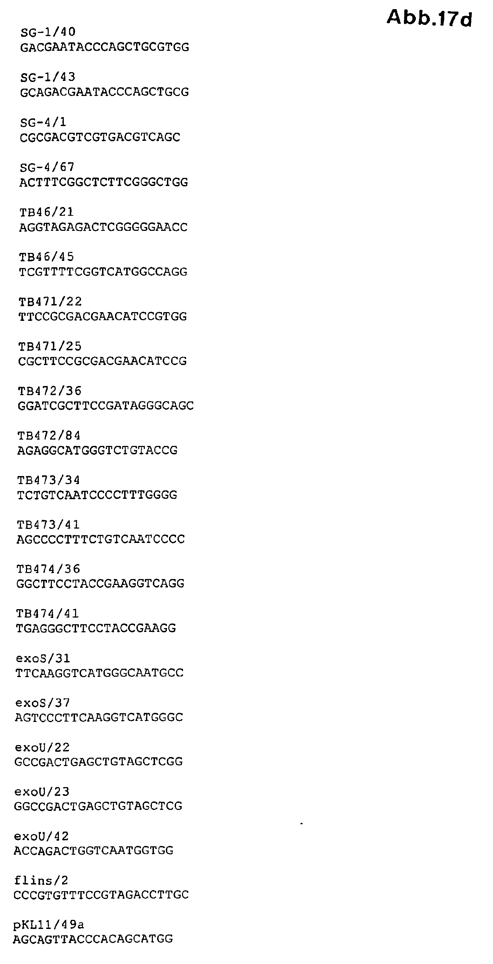

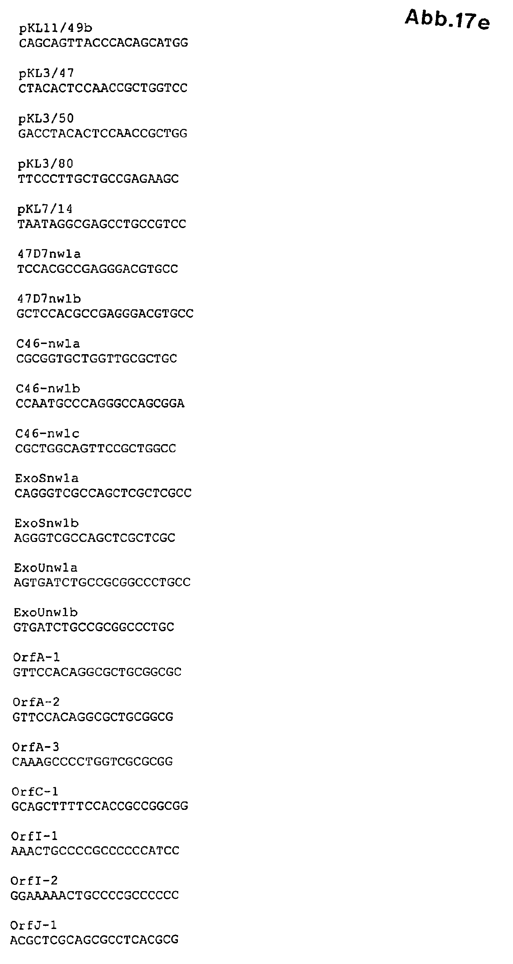

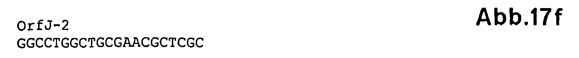

Abbildung 17 zeigt die Nukleotidsequenzen der im Ausführungsbeispiel eingesetzten Primer.

Erfindungsgemäße Oligonukleotid-Sonden sowie die Anordnung der Oligonukleotid-Sonden auf dem erfindungsgemäßen Nukleinsäurechip sind in den Abbildungen 18 bis 21 gezeigt.

## Patentansprüche

1. Microarray-Vorrichtung zur Geno- und Pathotypisierung der Spezies *Pseudomonas aeruginosa,* umfassend ein Trägerelement mit darauf auf vorbestimmten Bereichen immobilisierten Oligonukleotid-Sonden, wobei eine Oligonukleotid-Sonde die folgende Nukleinsäuresequenz (Sequenz in 5' → 3'-Richtung) aufweist:
GAAGCCCAGCAATTGCGTGTTTC.

2. Vorrichtung nach Anspruch 1,
**dadurch gekennzeichnet, dass** das Trägerelement zusätzlich Oligonukleotid-Sonden umfasst, die ausgewählt sind aus der Gruppe bestehend aus (sämtliche Sequenzen in 5' → 3'-Richtung):
GGTGCTGCAGGGTGTTTCGCCGG
GGTGCTGCAGGGCGTTTCGCCGG
CCTCGCCCTGTTCCCACCGCTCTGG
CTCGCCCTGTTCCCGCCGCTCTGG
TCGAGCAACTGGCAGAGAAATCCG
CGAGCAACTGGCGGAGAAATCCG
GAACAAGACCGGTTCCACCAACGG
AACAAGACCGGCTCCACCAACGG
GTCGCTGAACGGCACCTACTTCA
CAGCCCAGTCAGGACGCGCA

3. Vorrichtung nach Anspruch 1 oder 2,
**dadurch gekennzeichnet, dass** die Vorrichtung ein Reaktionsgefäß ist, welches eine für ein Laborreaktionsgefäß (Tube) typische Form und/oder typische Größe aufweist, und bei dem auf einer seiner Grundflächen das Trägerelement angeordnet ist.

4. Vorrichtung nach einem der Ansprüche 1 bis 3,
**dadurch gekennzeichnet, dass** das Trägerelement zusätzlich Oligonukleotid-Sonden umfasst, die ausgewählt sind aus der Gruppe bestehend aus (sämtliche Sequenzen in 5' → 3'-Richtung):
GAAGCCCAGCAACTGCGTGTTTC
AGCCCAGCAATTGCGTGTTTCTCCG
AGCCCAGCAACTGCGTGTTTCTCC
CAAGATCGCCGCAGCGGTCAAC
CAAGATCGCCGCTGCGGTCAAC
GCTGCTGGCGGCGGTGTGC
TGCTGCTGGCAGCGGTGTGCT
GCGGAAAACTTCCTGCACATGATGTT
GCGGAAAACTTCCTCCACATGATGTT
AGCTCAGCAGACTGCTGACGAGG
AGCTCAGCAGACCGCTGACGAG
CAGAAAGCTCAGCAGACTGCTGACGAG
GAAAGCTCAGCAGACCGCTGACGAG
ACGGCCGCCGGGTGACGCC
ACGGCCGCCAGGTGACGCCG
GACAAGATGCGCCTCGACGACC
GACAAGATGCGTCTCGACGACCG
AGCCGACCTACGCGCCGGGCAG
CAGCCGACCTATGCGCCGGGCAG
GCCGACCTACGCGCCGGGC
AGCCGACCTATGCGCCGGGCA
GTTCGAACGGCTCATGGAGCAGCA
GTTCGAACGACTCATGGAGCAGCAAG
TGGAGCAGCAAGTGTTCCCGGC
TGGAGCAGCAACTGTTCCCGGC
GCGACCTGGGCCTGGTGATCCT
GCGACCTGGGACTGGTGATCCT
GTGCTGCAGGGTGTTTCGCCG
GCTGCAGGGCGTTTCGCCG
GCTCAGCAGACTGCTGACGAGGCTAACG
GCTCAGCAGACCGCTGACGAGGCTAAC
CGACCTACGCGCCGGGCAG
CGACCTATGCGCCGGGCAGC
CGTTCGAACGGCTCATGGAGCAG
CGTTCGAACGACTCATGGAGCAGC
CGACCTGGGCCTGGTGATCCT
GCGACCTGGGACTGGTGATCCTGG.

5. Vorrichtung nach einem der Ansprüche 1 bis 4, **dadurch gekennzeichnet, dass** das Trägerelement zusätzlich Oligonukleotid-Sonden umfasst, die ausgewählt sind aus der Gruppe bestehend aus (sämtliche Sequenzen in 5' → 3'-Richtung):
GCGCGATCTTCTCCACTTCATCGG
CGAGGAGTTTCGGACCCGCTTTGA
AATAGGACCGGCAGAACGGGCATT
CGAAGTCTGAGGTGTGGACCCGC
GCATCATTGCGCGTCACATCTGGT
TCTGAACTGCGGCTATCACCTGGA
AGTCATGGGACTGAATACGGCGACT
TTCTCGGTGTCGAGGGATTCTCGG
TGGTAGCTCTCGACGTACTGGCTG
CCCGTTGCTCATAACCCGTTCCTG
AGGGCATTCTCAGGTGGACTCAGG
GCGCCTTCTCCTCTTTGCAGATGT
CAGTATGGTACGGACACGAAGCGC
TCCAACAGGCAGGAGTACAGGGTG
CGCTGCACATACAGGTCCGTTCTC
CAAGATCGCCGCAGCGGTCAACGAC
CAAGATCGCCGCTGCGGTCAACGAC
CAGTTGTCGCCAGGTCTGGAGAATCC
CACATCAATGTCAGCCCACGCCA
CTGGAGCCTGCGAAAGTGGCTC
ACGAGGGTGATGGCTGGGAATACG
GCCAATTGGGTCAGCAAGCAACG
CGTGTCGCGAACTCGCATGGC
CCTGAATCCGACCATTCGCGAGTC
TCGGACTGTACTCCTACGAAGCAGC
CCAATCCCTATCGCTGGAACCGTACC
GCTCGGGACTCGCATTTCGTCC
GCGTTATTGCTCGGTCTCTCCTCG
GACCGCAAGCAGAAACGGCATGC
CCATGGTCGGAACAGGCACGATATGC
CCACTCGATCATGTTGAGCATCGGCTCC
GGTTAGTCCCTTCTGCCCGCATCG.

6. Vorrichtung nach einem der Ansprüche 1 bis 5, **dadurch gekennzeichnet, dass** das Trägerelement zusätzlich Oligonukleotid-Sonden umfasst, die ausgewählt sind aus der Gruppe bestehend aus (sämtliche Sequenzen in 5' → 3'-Richtung):
GTGTCACGGCCCATGTCTAGCAGC
GTGAGCATGGAATCGGCAGTCGTT
ACCTGTGTCGCTGGAGGGTATGTT
CGCTGGAGGGTATGTTCCGCAAGG
CGTACTCAGCTTCTCCACCCAGCG
CCTGGACCTCTCCAAGGTTCGCCT
GCCATTCCGACGACCAAACAAGGC
AGGCCATGGGCTAGCCGGATGC
CGAAGCGTAGGGTCTTCGTAGCC
TGCGAGGACCAGAAACCTTGATGG
CGGTATGAAGATGGGTGGTTGGGTCG
TGCATAGGAGTCATGCCGACAGCA
GCCTGCCTACTTGTTCCCAACGC
GGCTGTATTGCCCGCCATTCTCC
CGACAGACAGAAAGGGTTCTTGCGC
CACCATGCAAATGCTCGATGGACTGC
GCAGGCGTCCAAGTTGGAGCTCTCC
GGAACACAACGTGGGGCGTGAC
CCAGTTGGCACCACCATGCTTGC.

7. Vorrichtung nach einem der Ansprüche 1 bis 6,
**dadurch gekennzeichnet, dass** das Trägerelement zusätzlich Oligonukleotid-Sonden umfasst, die ausgewählt sind aus der Gruppe bestehend aus (sämtliche Sequenzen in 5' → 3'-Richtung):
CAGCCCAGTCAGGACGCGCA
CGCCAGTTTGAGAACGGAGTCACC
AGTGACGTGCGTTTCAGCAGTCCC.

8. Vorrichtung nach einem der Ansprüche 1 bis 7,
**dadurch gekennzeichnet, dass** das Trägerelement zusätzlich Oligonukleotid-Sonden umfasst, die ausgewählt sind aus der Gruppe bestehend aus (sämtliche Sequenzen in 5' → 3'-Richtung):
GCCGACCAACTGAACTCCAACTCG
GTCGCTGAACGGCACCTACTTCA.

9. Vorrichtung nach einem der Ansprüche 1 bis 8,
**dadurch gekennzeichnet, dass** das Trägerelement zusätzlich Oligonukleotid-Sonden umfasst, die ausgewählt sind aus der Gruppe bestehend aus (sämtliche Sequenzen in 5' → 3'-Richtung):
GCTCAGCAGACTGCTGACGAGGCTAACG
GCTCAGCAGACCGCTGACGAGGCTAAC
CGTTCGAACGGCTCATGGAGCAG
CGTTCGAACGACTCATGGAGCAGC
CAGTTGTCGCCAGGTCTGGAGAATCC
CACATCAATGTCAGCCCACGCCA

10. Vorrichtung nach Anspruch 9,
**dadurch gekennzeichnet, dass** das Trägerelement zusätzlich Oligonukleotid-Sonden umfasst, die ausgewählt sind aus der Gruppe bestehend aus:
i) Oligonukleotiden mit den folgenden Nukleinsäuresequenzen (sämtliche Sequenzen in 5' → 3'-Richtung):
GCGGAAAACTTCCTGCACATGATGTT
GCGGAAAACTTCCTCCACATGATGTT
GCCGACCAACTGAACTCCAACTCG
CGCCAGTTTGAGAACGGAGTCACC
TCTGAACTGCGGCTATCACCTGGA
CGCTGGAGGGTATGTTCCGCAAGG
CCTGGACCTCTCCAAGGTTCGCCT
CAAGATCGCCGCAGCGGTCAACGAC
CAAGATCGCCGCTGCGGTCAACGAC
CGACCTACGCGCCGGGCAG
CGACCTATGCGCCGGGCAGC
ACGAGGGTGATGGCTGGGAATACG
CACCATGCAAATGCTCGATGGACTGC
CCACTCGATCATGTTGAGCATCGGCTCC, und
ii) Oligonukleotiden, die mit den Oligonukleotiden unter i) in mindestens 90 % der Basen übereinstimmen und eine spezifische Hybridisierung mit Nukleinsäuresequenzen von Bakterienstämmen der Spezies *Pseudomonas aeruginosa* ermöglichen.

11. Vorrichtung nach Anspruch 10,
**dadurch gekennzeichnet, dass** das Trägerelement zusätzlich Oligonukleotid-Sonden umfasst, die ausgewählt sind aus der Gruppe bestehend aus:
i) Oligonukleotiden mit den folgenden Nukleinsäuresequenzen (sämtliche Sequenzen in 5' → 3'-Richtung):
CAAGATCGCCGCAGCGGTCAAC
CAAGATCGCCGCTGCGGTCAAC
AGCTCAGCAGACTGCTGACGAGG
AGCTCAGCAGACCGCTGACGAG
TGGAGCAGCAAGTGTTCCCGGC
TGGAGCAGCAACTGTTCCCGGC
CGAGGAGTTTCGGACCCGCTTTGA
GCGCCTTCTCCTCTTTGCAGATGT
CAGTATGGTACGGACACGAAGCGC
GCATCATTGCGCGTCACATCTGGT
TTCTCGGTGTCGAGGGATTCTCGG
CCCGTTGCTCATAACCCGTTCCTG
ACCTGTGTCGCTGGAGGGTATGTT
TCCAACAGGCAGGAGTACAGGGTG
ACGGCCGCCGGGTGACGCC
ACGGCCGCCAGGTGACGCCG
GTGTCACGGCCCATGTCTAGCAGC
CGAAGTCTGAGGTGTGGACCCGC
GCCATTCCGACGACCAAACAAGGC
GTGCTGCAGGGTGTTTCGCCG
GCTGCAGGGCGTTTCGCCG
CGACCTGGGCCTGGTGATCCT
GCGACCTGGGACTGGTGATCCTGG
CTGGAGCCTGCGAAAGTGGCTC
GCCAATTGGGTCAGCAAGCAACG
CGTGTCGCGAACTCGCATGGC
AGGCCATGGGCTAGCCGGATGC
CGAAGCGTAGGGTCTTCGTAGCC
TGCGAGGACCAGAAACCTTGATGG
CGGTATGAAGATGGGTGGTTGGGTCG
CCTGAATCCGACCATTCGCGAGTC
TCGGACTGTACTCCTACGAAGCAGC
CCAATCCCTATCGCTGGAACCGTACC
GCTCGGGACTCGCATTTCGTCC
GCGTTATTGCTCGGTCTCTCCTCG
TGCATAGGAGTCATGCCGACAGCA
GCCTGCCTACTTGTTCCCAACGC
GGCTGTATTGCCCGCCATTCTCC
CGACAGACAGAAAGGGTTCTTGCGC
GCAGGCGTCCAAGTTGGAGCTCTCC
GGAACACAACGTGGGGCGTGAC
CCAGTTGGCACCACCATGCTTGC
GACCGCAAGCAGAAACGGCATGC
CCATGGTCGGAACAGGCACGATATGC
GGTTAGTCCCTTCTGCCCGCATCG, und
ii) Oligonukleotiden, die mit den Oligonukleotiden unter i) in mindestens 90 % der Basen übereinstimmen und eine spezifische Hybridisierung mit Nukleinsäuresequenzen von Bakterienstämmen der Spezies *Pseudomonas aeruginosa* ermöglichen.

12. Verfahren zum spezifischen Nachweis von Bakterienstämmen der Spezies *Pseudomonas aeruginosa* in einer Probe, umfassend die folgenden Schritte:
a) Inkontaktbringen der Probe mit einem Nukleinsäure-Chip in einer Microarray-Vorrichtung nach einem der Ansprüche 1 bis 11; und
b) Detektion der Wechselwirkung zwischen den Oligonukleotid-Sonden und in der Probe enthaltenen Target-Nukleinsäuren.

13. Verfahren nach Anspruch 12,
**dadurch gekennzeichnet, dass** die in der Probe enthaltenen Target-Nukleinsäuren vor der Detektion amplifiziert werden.

14. Verfahren nach Anspruch 13,
**dadurch gekennzeichnet, dass** die Amplifikation mittels Multiplex-PCR erfolgt.

15. Verfahren nach Anspruch 14,
**dadurch gekennzeichnet, dass** für die Amplifikation Primer eingesetzt werden, die ähnliche Schmelzpunkte und/oder ähnliche Bindungskinetiken aufweisen.

16. Verfahren nach einem der Ansprüche 13 bis 15,
**dadurch gekennzeichnet, dass** die Amplifikation linear erfolgt.

17. Verfahren nach einem der Ansprüche 13 bis 16,
**dadurch gekennzeichnet, dass** die Primer ausgewählt werden mit einer Nukleinsäuresequenz, ausgewählt aus der Gruppe bestehend aus (sämtliche Sequenzen in 5' → 3'-Richtung):
ACGCGGATGTCCTGGATTTGG
CTGAAGAAGGGGCGCTACGCG
GCGTACCGGGCAAGGTGATAG
CTCGGTGAAACATCGGGAGGG
TCATCCAGCAAGCCATTGCGC
GGAGTCGCTTTCCGCCATCG
TGGAGTCGCTTTCCGCCATCG
AAGGGCGTTTCACGCTGACGC
ATCCGGAAGGGCGTTTCACG
TCCACACCTCAGACTTCGGCG
TATTGACGACCTACCGCGCGC
GCAACTGATGTTCGCCCAGC
CGCAACTGATGTTCGCCCAGC
ACACGCAACTGATGTTCGCCC
TGTCCCGGCTCAGTTCAACG
AACACCTTGGCGTTTGTCCC
GCAACACCTTGGCGTTTGTCC
TCAAGCTCGTTGTGGACCGC
GTTACGACGGCGTGCTGTCGG
ACGCAACGTATTCGGCGACCC
CGCAACGTATTCGGCGACCC
AGCTGATGGTATCGCCGTCGC
CTAGTGATCGCACCGGAGCC
AGCCTCGACACCGGTTCTCG
TCGTTCATCCCCAGGCTTCG
ACCATCTCGTTCATCCCCAGG
TTCTGAGCCCAGGACTGCTCG
TCGACGCGACGGTTCTGAGCC
TGACGTTCTCGCCGGTAGCG
CAGTAGCGGTACCGGTCTGCG
CAGTAGCGGTACCGGTCTGC
TTCCTCGCCGGCATAGTAGGC
CGAGGACGAGGCATCTTCCGG
GCAGGTAGCAGGTTTCCAGG
AACTGTTCCTTCTGCGCGGCG
TGATCGGCTTGGTCTCGCAGG
GCTGATCGGCTTGGTCTCGC
GAGGCGTTCTGCTCGTGGTCG
TTTTTCCAGCATGCGCAGGG
GCTGGCTTTTTCCAGCATGCG
TTGCGGCTGGCTTTTTCCAGC
TTGGGATAGTTGCGGTTGGC
CGTAGGCGATCTTCACCCGC
TGGCGTAGGCGATCTTCACCC
GGCGAGATAGCCGAACAGGC
GCGGCGAGATAGCCGAACAGG
CACTTGCTGCTCCATGAGCC
GAGGTCGAGCAGGCTGATGC
TAGGTCGCGAGGTCGAGCAGG
GTCCTTCTGCACCGAGTCGG
CGCATCTTGTCCTGGGTCAGG
TCGTCGAGGCGCATCTTGTCC
ACGTCGAGGTGGGTCTGTTCG
GTAGCCTTCGGCATCCAGCG
TCGGCATTGGGATAGTTGCGG
CCTCCTGTCTCATGCCGATGC
GCATTCGCCACGGAAGGAAGG
GAAGGCATCATGGCATTCGCC
GTCATGGGGTTTCCCAGAGACC
GATCGCGATGTCGACGGTGCC
CGATCGCGATGTCGACGGTGC
TGCCGATCGCGATGTCGACG
GACGAATACCCAGCTGCGTGG
GCAGACGAATACCCAGCTGCG
CGCGACGTCGTGACGTCAGC
ACTTTCGGCTCTTCGGGCTGG
AGGTAGAGACTCGGGGGAACC
TCGTTTTCGGTCATGGCCAGG
TTCCGCGACGAACATCCGTGG
CGCTTCCGCGACGAACATCCG
GGATCGCTTCCGATAGGGCAGC
AGAGGCATGGGTCTGTACCG
TCTGTCAATCCCCTTTGGGG
AGCCCCTTTCTGTCAATCCCC
GGCTTCCTACCGAAGGTCAGG
TGAGGGCTTCCTACCGAAGG
TTCAAGGTCATGGGCAATGCC
AGTCCCTTCAAGGTCATGGGC
GCCGACTGAGCTGTAGCTCGG
GGCCGACTGAGCTGTAGCTCG
ACCAGACTGGTCAATGGTGG
CCCGTGTTTCCGTAGACCTTGC
AGCAGTTACCCACAGCATGG
CAGCAGTTACCCACAGCATGG
CTACACTCCAACCGCTGGTCC
GACCTACACTCCAACCGCTGG
TTCCCTTGCTGCCGAGAAGC
TAATAGGCGAGCCTGCCGTCC
TCCACGCCGAGGGACGTGCC
GCTCCACGCCGAGGGACGTGCC
CGCGGTGCTGGTTGCGCTGC
CCAATGCCCAGGGCCAGCGGA
CGCTGGCAGTTCCGCTGGCC
CAGGGTCGCCAGCTCGCTCGCC
AGGGTCGCCAGCTCGCTCGC
AGTGATCTGCCGCGGCCCTGCC
GTGATCTGCCGCGGCCCTGC
GTTCCACAGGCGCTGCGGCGC
GTTCCACAGGCGCTGCGGCG
CAAAGCCCCTGGTCGCGCGG
GCAGCTTTTCCACCGCCGGCGG
AAACTGCCCCGCCCCCCATCC
GGAAAAACTGCCCCGCCCCCC
ACGCTCGCAGCGCCTCACGCG
GGCCTGGCTGCGAACGCTCGC
GGGGTCGAGACGTGTACATGG
TTCCTGGGCCAGAGTTGGACC
AGCTTAAGGCCGTGGCACTCG
CCGGAGAATTCGCGTCCACC
TGCTGACGATGAAGCCCCAGC
AGGAGGCCGATGACAACACCC
TGCCGATTCCATGCTCACGCC
ACGACGTCACCGTCGAGACCG
ACCGCCTTTCTGGTGAGCTGG
AGCCAAGACGGTTGTTCGCGG
TCAATGACGCCGAGTTGGCGC
CTCGGACAGGTTCACGCTGG
GCCATTCGCTGCAACACCTCC
GCGCGCGTTCGAGAAACAGG
CGGAGGTTGAAAAGCTGGCCC
ATGCCATCGTTGAAGGCACCGC
TGCCATCGTTGAAGGCACCG
TCTGGCGGAATCAGGTAGGCC
CTTCCGGGGAGAAACCACCG
ACCTCCAGCACCGACACACC
ATCCGATCCACCTCCAGCACC
CGTTCAGGTCGTAGACCGCGC
GCGATACCAACTGTCCTGCGGC
TGCCGAAGGTGAATGGCTTGCC
CCTGATGGTCCGATCCCAGC
GCCGAGGGTCAAGAACCACTGG
TCTTGGCCCAGTCATAGCGGC
TAACCCCAAGGCCCATTGGAGG
GCCACCGCCTTCGAATAACCCC
AATTGCTCGAGGGATGCGGC
GGTCGAAACGGATGCGCAGG
GCCCCGCGTCATTTTCACGTCG
AATGCTCTGGGCAACGAGCC
CTACCCAGCTTGGGCGTAGC
AAGCGATAGCCGTGCTCCTGC
CCGGCTATATCCGCGGCTACC
ATTGGCGCTGCTGTTTACGCCC
GGTGGCGTCGGGTTTTTCTGC
AGGTCGTAGCGGAAGGTGGTGG
ATCTGAACCGAGGGGATCCGC
CCCGGGAGTCATTGGTCTGG
GCCTGTTGGACCCCTTTGACC
TACTCCTGCCTGTTGGACCCC
CGCTCAAGCGCTATCCCACC
CGCCATCGGCCTGTACAACG
CGGTAGAGAGCTGGGTTGGC
AACCTGGAGCTAGGGCAGAGC
GGTGCTCGACCCAAGCATCG
TCCTTGAGTTCCTTGGCGCGG
CAACACGCGACTGGCGATCC
TACATCATCCGCAACGGCGGC
TATTGACGACCTACCGCGCGCC
CACCAAGAACCCGCTGCTCG
ATCGTGGCAGGATGTCCACCG
TAGGCGGGCCTTTTGAAGGTGC

18. Verwendung der Vorrichtung nach einem der Ansprüche 1 bis 11 bzw. des Verfahrens nach einem der Ansprüche 12 bis 17 zur Geno- und Pathotypisierung von *Pseudomonas aeruginosa.*

19. Verwendung der Primer gemäß Anspruch 17 zur Amplifikation von Nukleinsäuren von Bakterienstämmen der Spezies *Pseudomonas aeruginosa.*

20. Oligonukleotid-Sonde mit der folgenden Sequenz (Sequenz in 5'→3'-Richtung): GAAGCCCAGCAATTGCGTGTTTC.

## Claims

1. Microarray device for genotyping and pathotyping the species *Pseudomonas aeruginosa*, comprising a support element with oligonucleotide probes immobilized thereon on predetermined regions, wherein an oligonucleotide probe has the following nucleic acid sequence (sequence in 5'→3' direction): GAAGCCCAGCAATTGCGTGTTTC.

2. Device according to claim 1,
**characterized in that** the support element further comprises oligonucleotide probes which are selected from the group consisting of (all sequences in 5'→3' direction):
GGTGCTGCAGGGTGTTTCGCCGG
GGTGCTGCAGGGCGTTTCGCCGG
CCTCGCCCTGTTCCCACCGCTCTGG
CTCGCCCTGTTCCCGCCGCTCTGG
TCGAGCAACTGGCAGAGAAATCCG
CGAGCAACTGGCGGAGAAATCCG
GAACAAGACCGGTTCCACCAACGG
AACAAGACCGGCTCCACCAACGG
GTCGCTGAACGGCACCTACTTCA
CAGCCCAGTCAGGACGCGCA

3. Device according to claim 1 or 2,
**characterized in that** the device is a reaction vessel having a shape and/or size typical for a laboratory reaction vessel (tube) and where the support element is arranged on one of its surface areas.

4. Device according to any of claims 1 to 3,
**characterized in that** the support element further comprises oligonucleotide probes which are selected from the group consisting of (all sequences in 5'→3' direction):
GAAGCCCAGCAACTGCGTGTTTC
AGCCCAGCAATTGCGTGTTTCTCCG
AGCCCAGCAACTGCGTGTTTCTCC
CAAGATCGCCGCAGCGGTCAAC
CAAGATCGCCGCTGCGGTCAAC
GCTGCTGGCGGCGGTGTGC
TGCTGCTGGCAGCGGTGTGCT
GCGGAAAACTTCCTGCACATGATGTT
GCGGAAAACTTCCTCCACATGATGTT
AGCTCAGCAGACTGCTGACGAGG
AGCTCAGCAGACCGCTGACGAG
CAGAAAGCTCAGCAGACTGCTGACGAG
GAAAGCTCAGCAGACCGCTGACGAG
ACGGCCGCCGGGTGACGCC
ACGGCCGCCAGGTGACGCCG
GACAAGATGCGCCTCGACGACC
GACAAGATGCGTCTCGACGACCG
AGCCGACCTACGCGCCGGGCAG
CAGCCGACCTATGCGCCGGGCAG
GCCGACCTACGCGCCGGGC
AGCCGACCTATGCGCCGGGCA
GTTCGAACGGCTCATGGAGCAGCA
GTTCGAACGACTCATGGAGCAGCAAG
TGGAGCAGCAAGTGTTCCCGGC
TGGAGCAGCAACTGTTCCCGGC
GCGACCTGGGCCTGGTGATCCT
GCGACCTGGGACTGGTGATCCT
GTGCTGCAGGGTGTTTCGCCG
GCTGCAGGGCGTTTCGCCG
GCTCAGCAGACTGCTGACGAGGCTAACG
GCTCAGCAGACCGCTGACGAGGCTAAC
CGACCTACGCGCCGGGCAG
CGACCTATGCGCCGGGCAGC
CGTTCGAACGGCTCATGGAGCAG
CGTTCGAACGACTCATGGAGCAGC
CGACCTGGGCCTGGTGATCCT
GCGACCTGGGACTGGTGATCCTGG.

5. Device according to any of claims 1 to 4,
**characterized in that** the support element further comprises oligonucleotide probes which are selected from the group consisting of (all sequences in 5'→3' direction):
GCGCGATCTTCTCCACTTCATCGG
CGAGGAGTTTCGGACCCGCTTTGA
AATAGGACCGGCAGAACGGGCATT
CGAAGTCTGAGGTGTGGACCCGC
GCATCATTGCGCGTCACATCTGGT
TCTGAACTGCGGCTATCACCTGGA
AGTCATGGGACTGAATACGGCGACT
TTCTCGGTGTCGAGGGATTCTCGG
TGGTAGCTCTCGACGTACTGGCTG
CCCGTTGCTCATAACCCGTTCCTG
AGGGCATTCTCAGGTGGACTCAGG
GCGCCTTCTCCTCTTTGCAGATGT
CAGTATGGTACGGACACGAAGCGC
TCCAACAGGCAGGAGTACAGGGTG
CGCTGCACATACAGGTCCGTTCTC
CAAGATCGCCGCAGCGGTCAACGAC
CAAGATCGCCGCTGCGGTCAACGAC
CAGTTGTCGCCAGGTCTGGAGAATCC
CACATCAATGTCAGCCCACGCCA
CTGGAGCCTGCGAAAGTGGCTC
ACGAGGGTGATGGCTGGGAATACG
GCCAATTGGGTCAGCAAGCAACG
CGTGTCGCGAACTCGCATGGC
CCTGAATCCGACCATTCGCGAGTC
TCGGACTGTACTCCTACGAAGCAGC
CCAATCCCTATCGCTGGAACCGTACC
GCTCGGGACTCGCATTTCGTCC
GCGTTATTGCTCGGTCTCTCCTCG
GACCGCAAGCAGAAACGGCATGC
CCATGGTCGGAACAGGCACGATATGC
CCACTCGATCATGTTGAGCATCGGCTCC
GGTTAGTCCCTTCTGCCCGCATCG.

6. Device according to any of claims 1 to 5,
**characterized in that** the support element further comprises oligonucleotide probes which are selected from the group consisting of (all sequences in 5'→3' direction):
GTGTCACGGCCCATGTCTAGCAGC
GTGAGCATGGAATCGGCAGTCGTT
ACCTGTGTCGCTGGAGGGTATGTT
CGCTGGAGGGTATGTTCCGCAAGG
CGTACTCAGCTTCTCCACCCAGCG
CCTGGACCTCTCCAAGGTTCGCCT
GCCATTCCGACGACCAAACAAGGC
AGGCCATGGGCTAGCCGGATGC
CGAAGCGTAGGGTCTTCGTAGCC
TGCGAGGACCAGAAACCTTGATGG
CGGTATGAAGATGGGTGGTTGGGTCG
TGCATAGGAGTCATGCCGACAGCA
GCCTGCCTACTTGTTCCCAACGC
GGCTGTATTGCCCGCCATTCTCC
CGACAGACAGAAAGGGTTCTTGCGC
CACCATGCAAATGCTCGATGGACTGC
GCAGGCGTCCAAGTTGGAGCTCTCC
GGAACACAACGTGGGGCGTGAC
CCAGTTGGCACCACCATGCTTGC.

7. Device according to any of claims 1 to 6,
**characterized in that** the support element further comprises oligonucleotide probes which are selected from the group consisting of (all sequences in 5'→3' direction):
CAGCCCAGTCAGGACGCGCA
CGCCAGTTTGAGAACGGAGTCACC
AGTGACGTGCGTTTCAGCAGTCCC.

8. Device according to any of claims 1 to 7,
**characterized in that** the support element further comprises oligonucleotide probes which are selected from the group consisting of (all sequences in 5'→3' direction):
GCCGACCAACTGAACTCCAACTCG
GTCGCTGAACGGCACCTACTTCA.

9. Device according to any of claims 1 to 8,
**characterized in that** the support element further comprises oligonucleotide probes which are selected from the group consisting of (all sequences in 5'→3' direction):
GCTCAGCAGACTGCTGACGAGGCTAACG
GCTCAGCAGACCGCTGACGAGGCTAAC
CGTTCGAACGGCTCATGGAGCAG
CGTTCGAACGACTCATGGAGCAGC
CAGTTGTCGCCAGGTCTGGAGAATCC
CACATCAATGTCAGCCCACGCCA

10. Device according to claim 9,
**characterized in that** the support element further comprises oligonucleotide probes which are selected from the group consisting of:
i) oligonucleotides having the following nucleotide sequences (all sequences in 5'→3' direction):
GCGGAAAACTTCCTGCACATGATGTT
GCGGAAAACTTCCTCCACATGATGTT
GCCGACCAACTGAACTCCAACTCG
CGCCAGTTTGAGAACGGAGTCACC
TCTGAACTGCGGCTATCACCTGGA
CGCTGGAGGGTATGTTCCGCAAGG
CCTGGACCTCTCCAAGGTTCGCCT
CAAGATCGCCGCAGCGGTCAACGAC
CAAGATCGCCGCTGCGGTCAACGAC
CGACCTACGCGCCGGGCAG
CGACCTATGCGCCGGGCAGC
ACGAGGGTGATGGCTGGGAATACG
CACCATGCAAATGCTCGATGGACTGC
CCACTCGATCATGTTGAGCATCGGCTCC, and
ii) oligonucleotides which match the oligonucleotides under i) in at least 90% of the bases and allow a specific hybridization with nucleic acid sequences of bacterial strains of the species *Pseudomonas aeruginosa.*

11. Device according to claim 10, **characterized in that** the support element further comprises oligonucleotide probes which are selected from the group consisting of:
i) oligonucleotides having the following nucleotide sequences (all sequences in 5'→3' direction):
CAAGATCGCCGCAGCGGTCAAC
CAAGATCGCCGCTGCGGTCAAC
AGCTCAGCAGACTGCTGACGAGG
AGCTCAGCAGACCGCTGACGAG
TGGAGCAGCAAGTGTTCCCGGC
TGGAGCAGCAACTGTTCCCGGC
CGAGGAGTTTCGGACCCGCTTTGA
GCGCCTTCTCCTCTTTGCAGATGT
CAGTATGGTACGGACACGAAGCGC
GCATCATTGCGCGTCACATCTGGT
TTCTCGGTGTCGAGGGATTCTCGG
CCCGTTGCTCATAACCCGTTCCTG
ACCTGTGTCGCTGGAGGGTATGTT
TCCAACAGGCAGGAGTACAGGGTG
ACGGCCGCCGGGTGACGCC
ACGGCCGCCAGGTGACGCCG
GTGTCACGGCCCATGTCTAGCAGC
CGAAGTCTGAGGTGTGGACCCGC
GCCATTCCGACGACCAAACAAGGC
GTGCTGCAGGGTGTTTCGCCG
GCTGCAGGGCGTTTCGCCG
CGACCTGGGCCTGGTGATCCT
GCGACCTGGGACTGGTGATCCTGG
CTGGAGCCTGCGAAAGTGGCTC
GCCAATTGGGTCAGCAAGCAACG
CGTGTCGCGAACTCGCATGGC
AGGCCATGGGCTAGCCGGATGC
CGAAGCGTAGGGTCTTCGTAGCC
TGCGAGGACCAGAAACCTTGATGG
CGGTATGAAGATGGGTGGTTGGGTCG
CCTGAATCCGACCATTCGCGAGTC
TCGGACTGTACTCCTACGAAGCAGC
CCAATCCCTATCGCTGGAACCGTACC
GCTCGGGACTCGCATTTCGTCC
GCGTTATTGCTCGGTCTCTCCTCG
TGCATAGGAGTCATGCCGACAGCA
GCCTGCCTACTTGTTCCCAACGC
GGCTGTATTGCCCGCCATTCTCC
CGACAGACAGAAAGGGTTCTTGCGC
GCAGGCGTCCAAGTTGGAGCTCTCC
GGAACACAACGTGGGGCGTGAC
CCAGTTGGCACCACCATGCTTGC
GACCGCAAGCAGAAACGGCATGC
CCATGGTCGGAACAGGCACGATATGC
GGTTAGTCCCTTCTGCCCGCATCG, and
ii) oligonucleotides which are identical with the oligonucleotides under i) in at least 90% of the bases and allow a specific hybridization with nucleic acid sequences of bacterial strains of the species *Pseudomonas aeruginosa.*

12. Method for specific detection of bacterial strains of the species *Pseudomonas aeruginosa* in a sample, comprising the following steps:
a) contacting the sample with the nucleic acid chip in a microarray device according to any of claims 1 to 11; and
b) detecting the interaction between the oligonucleotide probes and target nucleic acids present in the sample.

13. Method according to claim 12,
**characterized in that** the target nucleic acids contained in the sample are amplified before detection.

14. Method according to claim 13,
**characterized in that** the amplification is carried out by means of multiplex-PCR.

15. Method according to claim 14,
**characterized in that** primers having similar melting points and/or similar binding kinetics are used for the amplification.

16. Method according to any of claims 13 to 15,
**characterized in that** the amplification is carried out linearly.

17. Method according to any of claims 13 to 16,
**characterized in that** the primers are selected having a nucleic acid sequence selected from the group consisting of (all sequences in 5'→3' direction):
ACGCGGATGTCCTGGATTTGG
CTGAAGAAGGGGCGCTACGCG
GCGTACCGGGCAAGGTGATAG
CTCGGTGAAACATCGGGAGGG
TCATCCAGCAAGCCATTGCGC
GGAGTCGCTTTCCGCCATCG
TGGAGTCGCTTTCCGCCATCG
AAGGGCGTTTCACGCTGACGC
ATCCGGAAGGGCGTTTCACG
TCCACACCTCAGACTTCGGCG
TATTGACGACCTACCGCGCGC
GCAACTGATGTTCGCCCAGC
CGCAACTGATGTTCGCCCAGC
ACACGCAACTGATGTTCGCCC
TGTCCCGGCTCAGTTCAACG
AACACCTTGGCGTTTGTCCC
GCAACACCTTGGCGTTTGTCC
TCAAGCTCGTTGTGGACCGC
GTTACGACGGCGTGCTGTCGG
ACGCAACGTATTCGGCGACCC
CGCAACGTATTCGGCGACCC
AGCTGATGGTATCGCCGTCGC
CTAGTGATCGCACCGGAGCC
AGCCTCGACACCGGTTCTCG
TCGTTCATCCCCAGGCTTCG
ACCATCTCGTTCATCCCCAGG
TTCTGAGCCCAGGACTGCTCG
TCGACGCGACGGTTCTGAGCC
TGACGTTCTCGCCGGTAGCG
CAGTAGCGGTACCGGTCTGCG
CAGTAGCGGTACCGGTCTGC
TTCCTCGCCGGCATAGTAGGC
CGAGGACGAGGCATCTTCCGG
GCAGGTAGCAGGTTTCCAGG
AACTGTTCCTTCTGCGCGGCG
TGATCGGCTTGGTCTCGCAGG
GCTGATCGGCTTGGTCTCGC
GAGGCGTTCTGCTCGTGGTCG
TTTTTCCAGCATGCGCAGGG
GCTGGCTTTTTCCAGCATGCG
TTGCGGCTGGCTTTTTCCAGC
TTGGGATAGTTGCGGTTGGC
CGTAGGCGATCTTCACCCGC
TGGCGTAGGCGATCTTCACCC
GGCGAGATAGCCGAACAGGC
GCGGCGAGATAGCCGAACAGG
CACTTGCTGCTCCATGAGCC
GAGGTCGAGCAGGCTGATGC
TAGGTCGCGAGGTCGAGCAGG
GTCCTTCTGCACCGAGTCGG
CGCATCTTGTCCTGGGTCAGG
TCGTCGAGGCGCATCTTGTCC
ACGTCGAGGTGGGTCTGTTCG
GTAGCCTTCGGCATCCAGCG
TCGGCATTGGGATAGTTGCGG
CCTCCTGTCTCATGCCGATGC
GCATTCGCCACGGAAGGAAGG
GAAGGCATCATGGCATTCGCC
GTCATGGGGTTTCCCAGAGACC
GATCGCGATGTCGACGGTGCC
CGATCGCGATGTCGACGGTGC
TGCCGATCGCGATGTCGACG
GACGAATACCCAGCTGCGTGG
GCAGACGAATACCCAGCTGCG
CGCGACGTCGTGACGTCAGC
ACTTTCGGCTCTTCGGGCTGG
AGGTAGAGACTCGGGGGAACC
TCGTTTTCGGTCATGGCCAGG
TTCCGCGACGAACATCCGTGG
CGCTTCCGCGACGAACATCCG
GGATCGCTTCCGATAGGGCAGC
AGAGGCATGGGTCTGTACCG
TCTGTCAATCCCCTTTGGGG
AGCCCCTTTCTGTCAATCCCC
GGCTTCCTACCGAAGGTCAGG
TGAGGGCTTCCTACCGAAGG
TTCAAGGTCATGGGCAATGCC
AGTCCCTTCAAGGTCATGGGC
GCCGACTGAGCTGTAGCTCGG
GGCCGACTGAGCTGTAGCTCG
ACCAGACTGGTCAATGGTGG
CCCGTGTTTCCGTAGACCTTGC
AGCAGTTACCCACAGCATGG
CAGCAGTTACCCACAGCATGG
CTACACTCCAACCGCTGGTCC
GACCTACACTCCAACCGCTGG
TTCCCTTGCTGCCGAGAAGC
TAATAGGCGAGCCTGCCGTCC
TCCACGCCGAGGGACGTGCC
GCTCCACGCCGAGGGACGTGCC
CGCGGTGCTGGTTGCGCTGC
CCAATGCCCAGGGCCAGCGGA
CGCTGGCAGTTCCGCTGGCC
CAGGGTCGCCAGCTCGCTCGCC
AGGGTCGCCAGCTCGCTCGC
AGTGATCTGCCGCGGCCCTGCC
GTGATCTGCCGCGGCCCTGC
GTTCCACAGGCGCTGCGGCGC
GTTCCACAGGCGCTGCGGCG
CAAAGCCCCTGGTCGCGCGG
GCAGCTTTTCCACCGCCGGCGG
AAACTGCCCCGCCCCCCATCC
GGAAAAACTGCCCCGCCCCCC
ACGCTCGCAGCGCCTCACGCG
GGCCTGGCTGCGAACGCTCGC
GGGGTCGAGACGTGTACATGG
TTCCTGGGCCAGAGTTGGACC
AGCTTAAGGCCGTGGCACTCG
CCGGAGAATTCGCGTCCACC
TGCTGACGATGAAGCCCCAGC
AGGAGGCCGATGACAACACCC
TGCCGATTCCATGCTCACGCC
ACGACGTCACCGTCGAGACCG
ACCGCCTTTCTGGTGAGCTGG
AGCCAAGACGGTTGTTCGCGG
TCAATGACGCCGAGTTGGCGC
CTCGGACAGGTTCACGCTGG
GCCATTCGCTGCAACACCTCC
GCGCGCGTTCGAGAAACAGG
CGGAGGTTGAAAAGCTGGCCC
ATGCCATCGTTGAAGGCACCGC
TGCCATCGTTGAAGGCACCG
TCTGGCGGAATCAGGTAGGCC
CTTCCGGGGAGAAACCACCG
ACCTCCAGCACCGACACACC
ATCCGATCCACCTCCAGCACC
CGTTCAGGTCGTAGACCGCGC
GCGATACCAACTGTCCTGCGGC
TGCCGAAGGTGAATGGCTTGCC
CCTGATGGTCCGATCCCAGC
GCCGAGGGTCAAGAACCACTGG
TCTTGGCCCAGTCATAGCGGC
TAACCCCAAGGCCCATTGGAGG
GCCACCGCCTTCGAATAACCCC
AATTGCTCGAGGGATGCGGC
GGTCGAAACGGATGCGCAGG
GCCCCGCGTCATTTTCACGTCG
AATGCTCTGGGCAACGAGCC
CTACCCAGCTTGGGCGTAGC
AAGCGATAGCCGTGCTCCTGC
CCGGCTATATCCGCGGCTACC
ATTGGCGCTGCTGTTTACGCCC
GGTGGCGTCGGGTTTTTCTGC
AGGTCGTAGCGGAAGGTGGTGG
ATCTGAACCGAGGGGATCCGC
CCCGGGAGTCATTGGTCTGG
GCCTGTTGGACCCCTTTGACC
TACTCCTGCCTGTTGGACCCC
CGCTCAAGCGCTATCCCACC
CGCCATCGGCCTGTACAACG
CGGTAGAGAGCTGGGTTGGC
AACCTGGAGCTAGGGCAGAGC
GGTGCTCGACCCAAGCATCG
TCCTTGAGTTCCTTGGCGCGG
CAACACGCGACTGGCGATCC
TACATCATCCGCAACGGCGGC
TATTGACGACCTACCGCGCGCC
CACCAAGAACCCGCTGCTCG
ATCGTGGCAGGATGTCCACCG
TAGGCGGGCCTTTTGAAGGTGC

18. Use of the device according to any of claims 1 to 11 and/or of the method according to any of claims 12 to 17 for genotyping and pathotyping of *Pseudomonas aeruginosa*.

19. Use of the primers according to claim 17 for amplification of nucleic acids of bacterial strains of the species *Pseudomonas aeruginosa*.

20. Oligonucleotide probe having the following sequence (sequence in 5'→3' direction): GAAGCCCAGCAATTGCGTGTTTC.

## Revendications

1. Dispositif micro-array pour la geno-typification et patho-typification de l'espèce *Pseudomonas aeruginosa* comprenant un élément de support ayant là-dessus, sur des zones prédéterminées, des sondes oligonucléotides immobilisées, dans lequel une sonde oligonucléotide a la séquence d'acides nucléiques suivante (séquence en direction 5' → 3') :
GAAGCCCAGCAATTGCGTGTTTC.

2. Dispositif selon la revendication 1,
**caractérisé par le fait que** l'élément de support comprend en outre des sondes oligonucléotides qui sont sélectionnées parmi le group consistant en (toutes les séquences en direction 5' → 3') :
GGTGCTGCAGGGTGTTTCGCCGG
GGTGCTGCAGGGCGTTTCGCCGG
CCTCGCCCTGTTCCCACCGCTCTGG
CTCGCCCTGTTCCCGCCGCTCTGG
TCGAGCAACTGGCAGAGAAATCCG
CGAGCAACTGGCGGAGAAATCCG
GAACAAGACCGGTTCCACCAACGG
AACAAGACCGGCTCCACCAACGG
GTCGCTGAACGGCACCTACTTCA
CAGCCCAGTCAGGACGCGCA.

3. Dispositif selon la revendication 1 ou 2,
**caractérisé par le fait que** le dispositif est un récipient de réaction ayant une forme et/ou une dimension typique pour un récipient de réaction laboratoire (tube) et dans lequel l'élément de support est arrangé sur une des bases de celui-ci.

4. Dispositif selon la revendication 1 à 3,
**caractérisé par le fait que** l'élément de support comprend en outre des sondes oligonucléotides qui sont sélectionnées parmi le group consistant en (toutes les séquences en direction 5' → 3') :
GAAGCCCAGCAACTGCGTGTTTC
AGCCCAGCAATTGCGTGTTTCTCCG
AGCCCAGCAACTGCGTGTTTCTCC
CAAGATCGCCGCAGCGGTCAAC
CAAGATCGCCGCTGCGGTCAAC
GCTGCTGGCGGCGGTGTGC
TGCTGCTGGCAGCGGTGTGCT
GCGGAAAACTTCCTGCACATGATGTT
GCGGAAAACTTCCTCCACATGATGTT
AGCTCAGCAGACTGCTGACGAGG
AGCTCAGCAGACCGCTGACGAG
CAGAAAGCTCAGCAGACTGCTGACGAG
GAAAGCTCAGCAGACCGCTGACGAG
ACGGCCGCCGGGTGACGCC
ACGGCCGCCAGGTGACGCCG
GACAAGATGCGCCTCGACGACC
GACAAGATGCGTCTCGACGACCG
AGCCGACCTACGCGCCGGGCAG
CAGCCGACCTATGCGCCGGGCAG
GCCGACCTACGCGCCGGGC
AGCCGACCTATGCGCCGGGCA
GTTCGAACGGCTCATGGAGCAGCA
GTTCGAACGACTCATGGAGCAGCAAG
TGGAGCAGCAAGTGTTCCCGGC
TGGAGCAGCAACTGTTCCCGGC
GCGACCTGGGCCTGGTGATCCT
GCGACCTGGGACTGGTGATCCT
GTGCTGCAGGGTGTTTCGCCG
GCTGCAGGGCGTTTCGCCG
GCTCAGCAGACTGCTGACGAGGCTAACG
GCTCAGCAGACCGCTGACGAGGCTAAC
CGACCTACGCGCCGGGCAG
CGACCTATGCGCCGGGCAGC
CGTTCGAACGGCTCATGGAGCAG
CGTTCGAACGACTCATGGAGCAGC
CGACCTGGGCCTGGTGATCCT
GCGACCTGGGACTGGTGATCCTGG.

5. Dispositif selon la revendication 1 à 4,
**caractérisé par le fait que** l'élément de support comprend en outre des sondes oligonucléotides qui sont sélectionnées parmi le group consistant en (toutes les séquences en direction 5' → 3') :
GCGCGATCTTCTCCACTTCATCGG
CGAGGAGTTTCGGACCCGCTTTGA
AATAGGACCGGCAGAACGGGCATT
CGAAGTCTGAGGTGTGGACCCGC
GCATCATTGCGCGTCACATCTGGT
TCTGAACTGCGGCTATCACCTGGA
AGTCATGGGACTGAATACGGCGACT
TTCTCGGTGTCGAGGGATTCTCGG
TGGTAGCTCTCGACGTACTGGCTG
CCCGTTGCTCATAACCCGTTCCTG
AGGGCATTCTCAGGTGGACTCAGG
GCGCCTTCTCCTCTTTGCAGATGT
CAGTATGGTACGGACACGAAGCGC
TCCAACAGGCAGGAGTACAGGGTG
CGCTGCACATACAGGTCCGTTCTC
CAAGATCGCCGCAGCGGTCAACGAC
CAAGATCGCCGCTGCGGTCAACGAC
CAGTTGTCGCCAGGTCTGGAGAATCC
CACATCAATGTCAGCCCACGCCA
CTGGAGCCTGCGAAAGTGGCTC
ACGAGGGTGATGGCTGGGAATACG
GCCAATTGGGTCAGCAAGCAACG
CGTGTCGCGAACTCGCATGGC
CCTGAATCCGACCATTCGCGAGTC
TCGGACTGTACTCCTACGAAGCAGC
CCAATCCCTATCGCTGGAACCGTACC
GCTCGGGACTCGCATTTCGTCC
GCGTTATTGCTCGGTCTCTCCTCG
GACCGCAAGCAGAAACGGCATGC
CCATGGTCGGAACAGGCACGATATGC
CCACTCGATCATGTTGAGCATCGGCTCC
GGTTAGTCCCTTCTGCCCGCATCG.

6. Dispositif selon la revendication 1 à 5,
**caractérisé par le fait que** l'élément de support comprend en outre des sondes oligonucléotides qui sont sélectionnées parmi le group consistant en (toutes les séquences en direction 5' → 3') :
GTGTCACGGCCCATGTCTAGCAGC
GTGAGCATGGAATCGGCAGTCGTT
ACCTGTGTCGCTGGAGGGTATGTT
CGCTGGAGGGTATGTTCCGCAAGG
CGTACTCAGCTTCTCCACCCAGCG
CCTGGACCTCTCCAAGGTTCGCCT
GCCATTCCGACGACCAAACAAGGC
AGGCCATGGGCTAGCCGGATGC
CGAAGCGTAGGGTCTTCGTAGCC
TGCGAGGACCAGAAACCTTGATGG
CGGTATGAAGATGGGTGGTTGGGTCG
TGCATAGGAGTCATGCCGACAGCA
GCCTGCCTACTTGTTCCCAACGC
GGCTGTATTGCCCGCCATTCTCC
CGACAGACAGAAAGGGTTCTTGCGC
CACCATGCAAATGCTCGATGGACTGC
GCAGGCGTCCAAGTTGGAGCTCTCC
GGAACACAACGTGGGGCGTGAC
CCAGTTGGCACCACCATGCTTGC.

7. Dispositif selon la revendication 1 à 6,
**caractérisé par le fait que** l'élément de support comprend en outre des sondes oligonucléotides qui sont sélectionnées parmi le group consistant en (toutes les séquences en direction 5' → 3') :
CAGCCCAGTCAGGACGCGCA
CGCCAGTTTGAGAACGGAGTCACC
AGTGACGTGCGTTTCAGCAGTCCC.

8. Dispositif selon la revendication 1 à 7,
**caractérisé par le fait que** l'élément de support comprend en outre des sondes oligonucléotides qui sont sélectionnées parmi le group consistant en (toutes les séquences en direction 5' → 3') :
GCCGACCAACTGAACTCCAACTCG
GTCGCTGAACGGCACCTACTTCA.

9. Dispositif selon la revendication 1 à 8,
**caractérisé par le fait que** l'élément de support comprend en outre des sondes oligonucléotides qui sont sélectionnées parmi le group consistant en (toutes les séquences en direction 5' → 3') :
GCTCAGCAGACTGCTGACGAGGCTAACG
GCTCAGCAGACCGCTGACGAGGCTAAC
CGTTCGAACGGCTCATGGAGCAG
CGTTCGAACGACTCATGGAGCAGC
CAGTTGTCGCCAGGTCTGGAGAATCC
CACATCAATGTCAGCCCACGCCA.

10. Dispositif selon la revendication 9,
**caractérisé par le fait que** l'élément de support comprend en outre des sondes oligonucléotides qui sont sélectionnées parmi le group consistant en :
i) les oligonucléotides ayant les séquences d'acides nucléiques suivantes (toutes les séquences en direction 5' → 3') :
GCGGAAAACTTCCTGCACATGATGTT
GCGGAAAACTTCCTCCACATGATGTT
GCCGACCAACTGAACTCCAACTCG
CGCCAGTTTGAGAACGGAGTCACC
TCTGAACTGCGGCTATCACCTGGA
CGCTGGAGGGTATGTTCCGCAAGG
CCTGGACCTCTCCAAGGTTCGCCT
CAAGATCGCCGCAGCGGTCAACGAC
CAAGATCGCCGCTGCGGTCAACGAC
CGACCTACGCGCCGGGCAG
CGACCTATGCGCCGGGCAGC
ACGAGGGTGATGGCTGGGAATACG
CACCATGCAAATGCTCGATGGACTGC
CCACTCGATCATGTTGAGCATCGGCTCC, et
ii) les oligonucléotides ayant au moins 90 % d'homologie des bases avec les oligonucléotides de i) et qui permettent une hybridation spécifique sur des séquences d'acides nucléiques de souches bactériennes de l'espèce *Pseudomonas aeruginosa.*

11. Dispositif selon la revendication 10,
**caractérisé par le fait que** l'élément de support comprend en outre des sondes oligonucléotides qui sont sélectionnées parmi le group consistant en
i) les oligonucléotides ayant les séquences d'acides nucléiques suivantes (toutes les séquences en direction 5' → 3') :
CAAGATCGCCGCAGCGGTCAAC
CAAGATCGCCGCTGCGGTCAAC
AGCTCAGCAGACTGCTGACGAGG
AG-CTCAGCAGACCGCTGACGAG
TGGAGCAGCAAGTGTTCCCGGC
TGGAGCAGCAACTGTTCCCGGC
CGAGGAGTTTCGGACCCGCTTTGA
GCGCCTTCTCCTCTTTGCAGATGT
CAGTATGGTACGGACACGAAGCGC
GCATCATTGCGCGTCACATCTGGT
TTCTCGGTGTCGAGGGATTCTCGG
CCCGTTGCTCATAACCCGTTCCTG
ACCTGTGTCGCTGGAGGGTATGTT
TCCAACAGGCAGGAGTACAGGGTG
ACGGCCGCCGGGTGACGCC
ACGGCCGCCAGGTGACGCCG
GTGTCACGGCCCATGTCTAGCAGC
CGAAGTCTGAGGTGTGGACCCGC
GCCATTCCGACGACCAAACAAGGC
GTGCTGCAGGGTGTTTCGCCG
GCTGCAGGGCGTTTCGCCG
CGACCTGGGCCTGGTGATCCT
GCGACCTGGGACTGGTGATCCTGG
CTGGAGCCTGCGAAAGTGGCTC
GCCAATTGGGTCAGCAAGCAACG
CGTGTCGCGAACTCGCATGGC
AGGCCATGGGCTAGCCGGATGC
CGAAGCGTAGGGTCTTCGTAGCC
TGCGAGGACCAGAAACCTTGATGG
CGGTATGAAGATGGGTGGTTGGGTCG
CCTGAATCCGACCATTCGCGAGTC
TCGGACTGTACTCCTACGAAGCAGC
CCAATCCCTATCGCTGGAACCGTACC
GCTCGGGACTCGCATTTCGTCC
GCGTTATTGCTCGGTCTCTCCTCG
TGCATAGGAGTCATGCCGACAGCA
GCCTGCCTACTTGTTCCCAACGC
GGCTGTATTGCCCGCCATTCTCC
CGACAGACAGAAAGGGTTCTTGCGC
GCAGGCGTCCAAGTTGGAGCTCTCC
GGAACACAACGTGGGGCGTGAC
CCAGTTGGCACCACCATGCTTGC
GACCGCAAGCAGAAACGGCATGC
CCATGGTCGGAACAGGCACGATATGC
GGTTAGTCCCTTCTGCCCGCATCG, et
ii) les oligonucléotides ayant au moins 90 % d'homologie des bases avec les oligonucléotides de i) et qui permettent une hybridation spécifique sur des séquences d'acides nucléiques de souches bactériennes de l'espèce *Pseudomonas aeruginosa.*

12. Procédé pour la détection spécifique de souches bactériennes de l'espèce *Pseudomonas aeruginosa* dans une épreuve, comprenant les étapes suivantes :
a) la mise en contact de l'épreuve avec un puce d'acide nucléique dans un dispositif micro-array selon l'une des revendications 1 à 11 ; et
b) la détection de l'interaction entre les sondes oligonucléotides et d'acides nucléiques cibles contenus dans l'épreuve.

13. Procédé selon la revendication 12,
**caractérisé par le fait que** les acides nucléiques cibles contenus dans l'épreuve vont être amplifiés avant la détection.

14. Procédé selon la revendication 13,
**caractérisé par le fait que** l'amplification est réalisée au moyen de PCR multiplex.

15. Procédé selon la revendication 14,
**caractérisé par le fait que** pour l'amplification des amorces ayant des points de fusion similaires et/ou des cinétiques de liaison similaires sont utilisés.

16. Procédé selon l'une des revendications 13 à 15,
**caractérisé par le fait que** l'amplification est réalisée d'une manière linéaire.

17. Procédé selon l'une des revendications 13 à 16,
**caractérisé par le fait que** les amorces sont sélectionnes avec une séquence d'acides nucléiques, sélectionnée parmi le groupe consistant en (toutes les séquences en direction 5' → 3') :
ACGCGGATGTCCTGGATTTGG
CTGAAGAAGGGGCGCTACGCG
GCGTACCGGGCAAGGTGATAG
CTCGGTGAAACATCGGGAGGG
TCATCCAGCAAGCCATTGCGC
GGAGTCGCTTTCCGCCATCG
TGGAGTCGCTTTCCGCCATCG
AAGGGCGTTTCACGCTGACGC
ATCCGGAAGGGCGTTTCACG
TCCACACCTCAGACTTCGGCG
TATTGACGACCTACCGCGCGC
GCAACTGATGTTCGCCCAGC
CGCAACTGATGTTCGCCCAGC
ACACGCAACTGATGTTCGCCC
TGTCCCGGCTCAGTTCAACG
AACACCTTGGCGTTTGTCCC
GCAACACCTTGGCGTTTGTCC
TCAAGCTCGTTGTGGACCGC
GTTACGACGGCGTGCTGTCGG
ACGCAACGTATTCGGCGACCC
CGCAACGTATTCGGCGACCC
AGCTGATGGTATCGCCGTCGC
CTAGTGATCGCACCGGAGCC
AGCCTCGACACCGGTTCTCG
TCGTTCATCCCCAGGCTTCG
ACCATCTCGTTCATCCCCAGG
TTCTGAGCCCAGGACTGCTCG
TCGACGCGACGGTTCTGAGCC
TGACGTTCTCGCCGGTAGCG
CAGTAGCGGTACCGGTCTGCG
CAGTAGCGGTACCGGTCTGC
TTCCTCGCCGGCATAGTAGGC
CGAGGACGAGGCATCTTCCGG
GCAGGTAGCAGGTTTCCAGG
AACTGTTCCTTCTGCGCGGCG
TGATCGGCTTGGTCTCGCAGG
GCTGATCGGCTTGGTCTCGC
GAGGCGTTCTGCTCGTGGTCG
TTTTTCCAGCATGCGCAGGG
GCTGGCTTTTTCCAGCATGCG
TTGCGGCTGGCTTTTTCCAGC
TTGGGATAGTTGCGGTTGGC
CGTAGGCGATCTTCACCCGC
TGGCGTAGGCGATCTTCACCC
GGCGAGATAGCCGAACAGGC
GCGGCGAGATAGCCGAACAGG
CACTTGCTGCTCCATGAGCC
GAGGTCGAGCAGGCTGATGC
TAGGTCGCGAGGTCGAGCAGG
GTCCTTCTGCACCGAGTCGG
CGCATCTTGTCCTGGGTCAGG
TCGTCGAGGCGCATCTTGTCC
ACGTCGAGGTGGGTCTGTTCG
GTAGCCTTCGGCATCCAGCG
TCGGCATTGGGATAGTTGCGG
CCTCCTGTCTCATGCCGATGC
GCATTCGCCACGGAAGGAAGG
GAAGGCATCATGGCATTCGCC
GTCATGGGGTTTCCCAGAGACC
GATCGCGATGTCGACGGTGCC
CGATCGCGATGTCGACGGTGC
TGCCGATCGCGATGTCGACG
GACGAATACCCAGCTGCGTGG
GCAGACGAATACCCAGCTGCG
CGCGACGTCGTGACGTCAGC
ACTTTCGGCTCTTCGGGCTGG
AGGTAGAGACTCGGGGGAACC
TCGTTTTCGGTCATGGCCAGG
TTCCGCGACGAACATCCGTGG
CGCTTCCGCGACGAACATCCG
GGATCGCTTCCGATAGGGCAGC
AGAGGCATGGGTCTGTACCG
TCTGTCAATCCCCTTTGGGG
AGCCCCTTTCTGTCAATCCCC
GGCTTCCTACCGAAGGTCAGG
TGAGGGCTTCCTACCGAAGG
TTCAAGGTCATGGGCAATGCC
AGTCCCTTCAAGGTCATGGGC
GCCGACTGAGCTGTAGCTCGG
GGCCGACTGAGCTGTAGCTCG
ACCAGACTGGTCAATGGTGG
CCCGTGTTTCCGTAGACCTTGC
AGCAGTTACCCACAGCATGG
CAGCAGTTACCCACAGCATGG
CTACACTCCAACCGCTGGTCC
GACCTACACTCCAACCGCTGG
TTCCCTTGCTGCCGAGAAGC
TAATAGGCGAGCCTGCCGTCC
TCCACGCCGAGGGACGTGCC
GCTCCACGCCGAGGGACGTGCC
CGCGGTGCTGGTTGCGCTGC
CCAATGCCCAGGGCCAGCGGA
CGCTGGCAGTTCCGCTGGCC
CAGGGTCGCCAGCTCGCTCGCC
AGGGTCGCCAGCTCGCTCGC
AGTGATCTGCCGCGGCCCTGCC
GTGATCTGCCGCGGCCCTGC
GTTCCACAGGCGCTGCGGCGC
GTTCCACAGGCGCTGCGGCG
CAAAGCCCCTGGTCGCGCGG
GCAGCTTTTCCACCGCCGGCGG
AAACTGCCCCGCCCCCCATCC
GGAAAAACTGCCCCGCCCCCC
ACGCTCGCAGCGCCTCACGCG
GGCCTGGCTGCGAACGCTCGC
GGGGTCGAGACGTGTACATGG
TTCCTGGGCCAGAGTTGGACC
AGCTTAAGGCCGTGGCACTCG
CCGGAGAATTCGCGTCCACC
TGCTGACGATGAAGCCCCAGC
AGGAGGCCGATGACAACACCC
TGCCGATTCCATGCTCACGCC
ACGACGTCACCGTCGAGACCG
ACCGCCTTTCTGGTGAGCTGG
AGCCAAGACGGTTGTTCGCGG
TCAATGACGCCGAGTTGGCGC
CTCGGACAGGTTCACGCTGG
GCCATTCGCTGCAACACCTCC
GCGCGCGTTCGAGAAACAGG
CGGAGGTTGAAAAGCTGGCCC
ATGCCATCGTTGAAGGCACCGC
TGCCATCGTTGAAGGCACCG
TCTGGCGGAATCAGGTAGGCC
CTTCCGGGGAGAAACCACCG
ACCTCCAGCACCGACACACC
ATCCGATCCACCTCCAGCACC
CGTTCAGGTCGTAGACCGCGC
GCGATACCAACTGTCCTGCGGC
TGCCGAAGGTGAATGGCTTGCC
CCTGATGGTCCGATCCCAGC
GCCGAGGGTCAAGAACCACTGG
TCTTGGCCCAGTCATAGCGGC
TAACCCCAAGGCCCATTGGAGG
GCCACCGCCTTCGAATAACCCC
AATTGCTCGAGGGATGCGGC
GGTCGAAACGGATGCGCAGG
GCCCCGCGTCATTTTCACGTCG
AATGCTCTGGGCAACGAGCC
CTACCCAGCTTGGGCGTAGC
AAGCGATAGCCGTGCTCCTGC
CCGGCTATATCCGCGGCTACC
ATTGGCGCTGCTGTTTACGCCC
GGTGGCGTCGGGTTTTTCTGC
AGGTCGTAGCGGAAGGTGGTGG
ATCTGAACCGAGGGGATCCGC
CCCGGGAGTCATTGGTCTGG
GCCTGTTGGACCCCTTTGACC
TACTCCTGCCTGTTGGACCCC
CGCTCAAGCGCTATCCCACC
CGCCATCGGCCTGTACAACG
CGGTAGAGAGCTGGGTTGGC
AACCTGGAGCTAGGGCAGAGC
GGTGCTCGACCCAAGCATCG
TCCTTGAGTTCCTTGGCGCGG
CAACACGCGACTGGCGATCC
TACATCATCCGCAACGGCGGC
TATTGACGACCTACCGCGCGCC
CACCAAGAACCCGCTGCTCG
ATCGTGGCAGGATGTCCACCG
TAGGCGGGCCTTTTGAAGGTGC.

18. Utilisation du dispositif selon l'une des revendications 1 à 11 et du procédé selon l'une des revendications 12 à 17, respectivement, pour la geno-typification et patho-typification du *Pseudomonas aeruginosa.*

19. Utilisation des amorces selon la revendication 17 pour l'amplification d'acides nucléiques de souches bactériennes de l'espèce *Pseudomonas aeruginosa.*

20. Sonde oligonucléotide ayant la séquence suivante (séquence en direction 5' → 3') : GAAGCCCAGCAATTGCGTGTTTC.
